(19) Europäisches Patentamt
European Patent Office
Office européen des brevets

(11) EP 4 644 359 A1

(12) # EUROPEAN PATENT APPLICATION

(43) Date of publication:
**05.11.2025 Bulletin 2025/45**

(21) Application number: **24173086.0**

(22) Date of filing: **29.04.2024**

(51) International Patent Classification (IPC):
***C07C 69/52*** *(2006.01)* ***C08G 63/91*** *(2006.01)*
***C08L 67/06*** *(2006.01)* ***C09D 7/80*** *(2018.01)*
***C09D 167/00*** *(2006.01)* ***C09D 167/07*** *(2006.01)*

(52) Cooperative Patent Classification (CPC):
**C08G 63/52; C07C 69/593; C07C 69/60; C08G 63/918; C09D 167/06**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Hexion Research Belgium SA**
**1348 Ottignies Louvain-la-Neuve (BE)**

(72) Inventors:
- **Steinbrecher, Christophe**
  **4218 Couthuin (BE)**
- **Heymans, Denis**
  **1340 Ottignies Louvain-la-Neuve (BE)**
- **Vanaken, David**
  **3040 Neerijse (BE)**

(74) Representative: **Sonnenhauser, Thomas Martin**
**Wuesthoff & Wuesthoff**
**Patentanwälte und Rechtsanwalt PartG mbB**
**Schweigerstraße 2**
**81541 München (DE)**

(54) **UV-CURABLE RESIN FORMULATION BASED ON ITACONATES AND (BRANCHED) GLYCIDYL ESTERS**

(57) The invention refers to a resin formulation comprising a) an oligomer comprising 3 to 20, structural units independently selected from formula (1), formula (2) and formula (35):

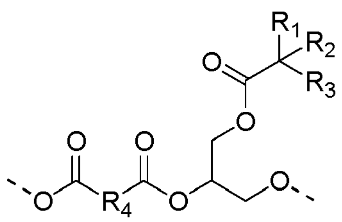
(1)

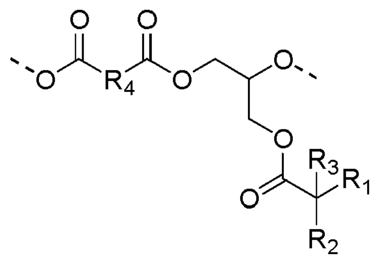
(2)

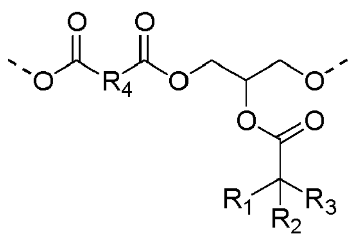
(35)

, wherein the structural units of the oligomer can be the same or can be different, wherein the dashed lines represent the linking in the oligomer, wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12, wherein $R_3$ is methyl, wherein $R_4$ is a divalent organic group comprising one or more UV curable carbon-carbon double bonds; b) a reactive diluent comprising exactly one structural unit according to formulae (1), (2) or (35); c) a photoinitiator. The invention further refers to a use of the resin formulation; to the preparation of the resin formulation; to a coated article; to an oligomer and a resin formulation comprising the oligomer;

EP 4 644 359 A1

and to a reactive diluent and a resin formulation comprising the reactive diluent.

## Description

The present invention particularly relates to diluents and oligomers/resins based on both itaconates and (branched) glycidyl esters, and in addition, to their combination in a UV-curable resin formulation/composition.

## Technical background

The majority of the oligomers and monomers used in UV-curing process typically have acrylic/acrylate moieties (e.g. Trimethylolpropane triacrylate (TMPTA), 1,6-Hexanediol diacrylate (HDDA), or the ACE™ hydroxyl acrylate monomer (the adduct of acrylic acid and the glycidyl ester of neodecanoic acid)) to allow the radical curing mechanism to occur in a suitable manner.

For example, WO 2008/045478 A1 discloses a radiation curable inkjettable adhesive composition combining the advantages of radical and cationic cure. The composition includes an acrylate ester of carboxylate acid ester, copolymerized with a di-functional component, which is preferably an aliphatic epoxy, utilizing both a photocation polymerization initiator and a free-radical photoinitiator. The adhesive composition is advantageously utilized in drop-on-demand printheads, resulting in a polymeric film having dark cure and excellent adhesion to multiple substrates. Here, the acrylate ester of carboxylate acid ester represents an acrylate monomer based on a branched acrylate and a functional hydroxyl group such as the ACE™ hydroxyl acrylate monomer (the adduct of acrylic acid and the glycidyl ester of neodecanoic acid).

Furthermore, CA 2232875 A1 discloses a paint containing the following components: (A) 5-60 wt.% at least one polyester-modified polyacrylate resin, consisting of (a1) 20-60 wt.% at least one polyester and (a2) 40-80 wt.% at least one polyacrylate resin containing hydroxyl groups and produced at least in part in the presence of component (a1); (B) 1-35 wt.% at least one siloxane-modified polyacrylate resin containing hydroxyl groups; and (C) 5-50 wt.% an isocyanate crosslinking agent. The invention also pertains to a process for producing a protective and/or decorative coating with coating agents of the type described and their use as a covering and repair paint.

However, systems based on acrylic/acrylate moieties are leading to environmental issues and skin hazards. Thus, the current sustainable trend shows an interest of using bio-based alternatives to develop UV-curable systems free of acrylic moieties. For example, itaconate derivatives such as itaconic acid and itaconic anhydride, which are biomass-based raw materials with a UV-polymerizable double bond, are of interest. Literature describes inter alia the use of itaconic acid as a suitable solution to replace acrylic-based moieties for the photo-curing mechanism, and further, it is also described to use epoxy moieties to polymerize hydroxyesters usable as reactive diluents for UV-curable systems. It is therefore, e.g., possible to produce resins by UV-curing using itaconate (di)hydroxyesters and an itaconate polyester oligomer.

DE 1768622 A discloses a process for the preparation of 2-hydroxyalkyl esters of unsaturated dicarboxylic acids by catalysed reaction of the acids with a vicinal epoxide, characterized in that a tertiary phosphine is used as the catalyst. Here, DE 1768622 A particularly refers to epoxides such as ethylene oxide, propylene oxide, butylene oxide or styrene oxide, i.e. low molecular weight aliphatic or aromatic epoxides. However, it is not disclosed or taught to synthesise 2-hydroxyalkyl esters explicitly based on branched or sterically more demanding epoxides or epoxides comprising functional groups such as ester or ether groups.

WO 2013/113938 A1 relates to polymers and polymeric materials obtained and/or obtainable from certain 2-methylidenebutanedioate diester monomers (also referred to as higher itaconate diesters), to a process for making such polymers and their use in compositions for, e.g., coatings, inks and/or adhesives, wherein the itaconate based monomers are substituted with optionally a substituted hydrocarbo moiety having from 4 to 10 carbon atoms. However, it is not disclosed or taught to use copolymer (compositions) that are explicitly based on itaconate monomers substituted with branched or sterically more demanding groups or functional groups such as ester or ether groups.

P. Li et al. (Itaconic Acid as a Green Alternative to Acrylic Acid for Producing a Soybean Oil-Based Thermoset: Synthesis and Properties. ACS Sustainable Chem. Eng., 2017, 5, 1228-1236) discloses the synthesis monomethyl itaconated epoxidized soybean oil via the reaction of itaconic acid with methanol to produce monomethyl itaconate followed by melt ring-opening esterification of mono-methyl itaconate with epoxidized soybean oil. The comparison with an acrylated epoxidized soybean is shown, inter alia with regard to the gel content % values. The monomethyl itaconated epoxidized soybean oil has a soybean-like architecture with long chains. A disadvantage is that these compounds can exhibit yellowness and impurities being typical for natural oils.

DE 3803141 A1 relates to oxidatively drying, low-solvent coating materials which are stable on storage and are based on polymer binder/reactive diluent systems, and an discloses the use of a diesteritaconate (dibutylitaconate) as reactive diluent.

WO 2022/058473 A1 discloses a process for producing free-radically curable compositions for free-radical polymerization, wherein particular reactive diluents are used. These reactive diluents are especially suitable for free-radically curable compositions for free-radical polymerization of these compositions, and it is possible to provide essentially bio-based compositions. In a further aspect WO 2022/058473 A1 is directed to such free-radically curable

compositions, especially UV compositions, obtainable by the process of the invention, and to the use of itaconate derivatives as reactive diluents and to novel itaconate derivatives as such. More specifically, WO 2022/058473 A1 refers to reactive diluents, i.e. α-hydroxyesters and di-α-hydroxyesters, based on itaconic anhydride/acid and substituted with alkyl, alkenyl, cycloalkyl, aryl, alkoxyaryl, alkylaryl, or heteroaryl groups, wherein these groups may be substituted. However, WO 2022/058473 A1 does not particularly refers to branched or sterically more demanding epoxides as well as epoxides comprising functional groups such as ester or ether groups.

S. Pérocheau Arnaud et al. (Itaconic Acid-Based Reactive Diluents for Renewable and Acrylate-Free UV-Curing Additive Manufacturing Materials. ACS Sustainable Chem. Eng., 2021, 9, 17142-17151) discloses inter alia the synthesis of reactive diluents, i.e. α-hydroxyesters and di-α-hydroxyesters, based on itaconic anhydride or itaconic acid. The α-hydroxyesters were synthesized via the reaction of 1 equivalent of itaconic anhydride (synthesized from itaconic acid) with 1 equivalent of an alcohol and subsequent reaction of the formed product with 1 equivalent of an epoxide. The di-α-hydroxyesters were synthesized via the reaction of 1 equivalent of itaconic acid with 2 equivalents of an epoxide. As alcohol methanol, 1-butanol, cyclohexanol, phenoxyethanol, and benzylalcohol were used. The epoxides used were cyclohexene oxide, 1,2-epoxybutane, styrene oxide, and (+)-limonene oxide, i.e. aliphatic, aromatic and cyclic epoxides. S. Pérocheau Arnaud et al. further discloses the combination of these reactive diluents with polypropylene itaconate in a resin formulation. However, S. Pérocheau Arnaud et al. does not disclose diluents and/or oligomers/polymers based on itaconic anhydride/acid and branched or sterically more demanding epoxides as well as epoxides comprising functional groups such as ester or ether groups.

L. Papadopoulos et al. (Bio-based additive manufacturing materials: An in-depth structure-property relationship study of UV-curing polyesters from itaconic acid. European Polymer Journal, 2023, 186, 111872) discloses the synthesis of UV-curable polyesters based on itaconic acid in combination with different dicarboxylic acids (succinic acid, sebacic acid, phthalic anhydride, isophthalic acid, and 2,5-furandicarboxylic acid) as well as diols (1,3-propanediol and 1,12-dodecanediol). In respective resin formulations, these resins were combined with different reactive diluents (acryloyl morpholine and isobornyl acrylate). However, L. Papadopoulos et al. does not disclose diluents based on itaconic anhydride/acid and/or their combination with the these itaconate based oligomers/polymers. Furthermore, Papadopoulos et al. does not disclose the synthesis of itaconate based diluents or oligomers/polymers via a combination with epoxides, let alone branched or sterically more demanding epoxides as well as epoxides comprising functional groups such as ester or ether groups.

Further examples of itaconate-based resin systems may be found at:

- S. Ma et al., Bio-based epoxy resin from itaconic acid and its thermosets cured with anhydride and comonomers. Green Chem., 2013, 15, 245;
- S. Wang et al., Making organic coatings greener: Renewable resource, solvent-free synthesis, UV curing and repairability. European Polymer Journal, 2020, 123, 109439; and
- J.-T. Miao et al., Three-Dimensional Printing Fully Biobased Heat-Resistant Photoactive Acrylates from Aliphatic Biomass. ACS Sustainable Chem. Eng., 2020, 8, 9415-9424.

## Summary of invention

To the knowledge of the inventors there is no mentioning in the prior art of a combination of branched or sterically more demanding epoxides as well as epoxides comprising functional groups such as glycidyl esters with itaconates to synthesize respective diluents and/or oligomers/polymers as well as their combination in a composition in order to bring different performances in the final UV-cured application that can be interesting for the market and various industrial applications. The usage of glycidyl esters to develop those sustainable (poly)esters can greatly ease both the esterification process and the radical polymerization process (solvent-free, catalyst-free, no water byproduct) while imparting interesting mechanical properties to the final UV-cured coatings due to a potential bulkiness caused by the substitution pattern and the presence of specific hydroxyl moieties.

The present invention is therefore directed to a resin formulation comprising

a) an oligomer comprising 3 to 20, structural units independently selected from formula (1), formula (2) and formula (35):

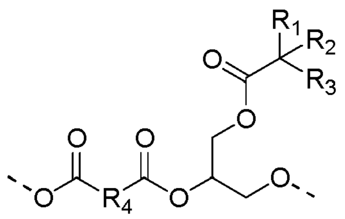
(1)
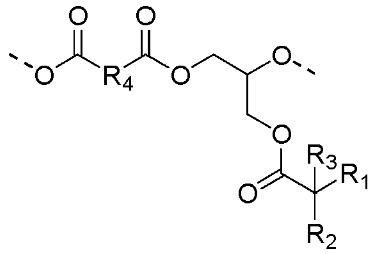
(2)
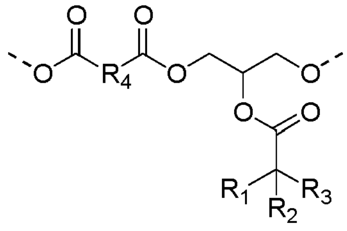
(35)

, wherein the structural units of the oligomer can be the same or can be different,
wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12,
wherein $R_3$ is methyl,
wherein $R_4$ is a divalent organic group comprising one or more UV curable carbon-carbon double bonds;

b) a reactive diluent comprising exactly one structural unit according to formulae (1), (2) or (35);
c) a photoinitiator.

The present invention is further directed to a use of a resin formulation as defined herein in UV-curing processes.
The present invention is further directed to a preparation of a resin formulation by combining at least (a) and (b) as defined herein.
The present invention is further directed to a coated article comprising a coating obtained by applying a resin formulation onto an article and drying said resin formulation, wherein said resin formulation is as defined herein.
The present invention is further directed to an oligomer comprising 3 to 20 structural units independently selected from formula (1), formula (2) and formula (35):

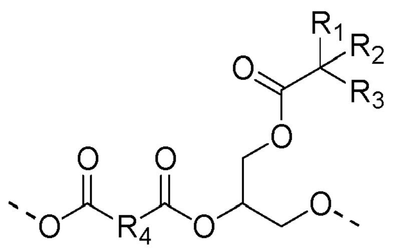
(1)
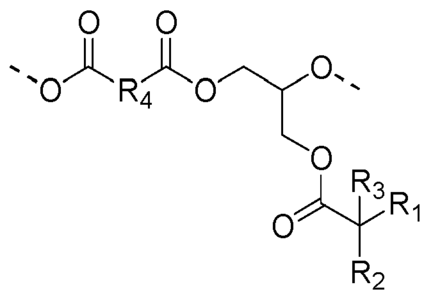
(2)
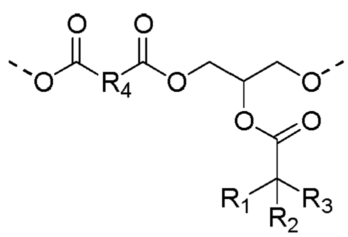
(35)

, wherein the structural units of the oligomer can be the same or can be different,
, wherein the dashed lines represent the linking in the oligomer,
wherein $R_1$, $R_2$, $R_3$ are as defined herein at any occurrence;
wherein $R_4$ is a divalent organic group comprising one or more carbon-carbon double bonds.

The present invention is further directed to resin formulation comprising the oligomer as defined herein.

The present invention is further directed to a reactive diluent selected from formula (23), formula (24) and formula (43):

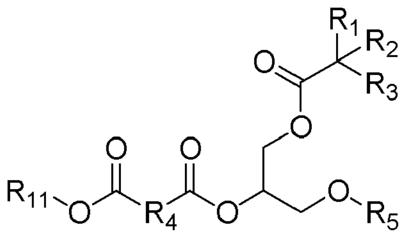
(23)

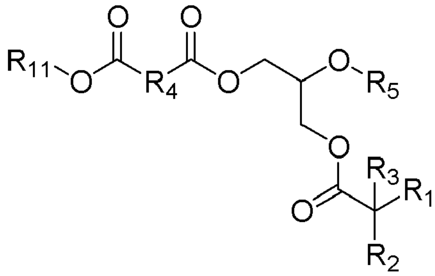
(24)

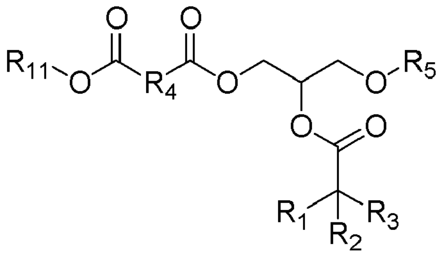
(43)

, $R_1$, $R_2$, $R_3$ are as defined herein at any occurrence,
wherein $R_4$ is a divalent organic group comprising one or more UV curable carbon-carbon double bonds,
wherein $R_5$ is H or represented by formula (8):

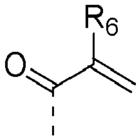
(8)

, wherein $R_6$ is H or methyl,
wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, or
$R_{11}$ is independently selected from formulae (25) to (30) and formulae (44) to (46):

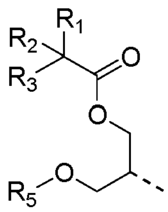
(25)

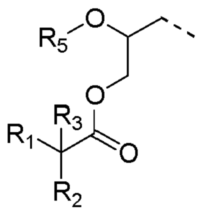
(26)

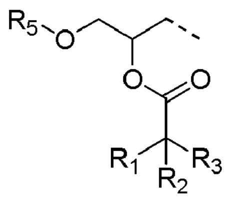
(44)

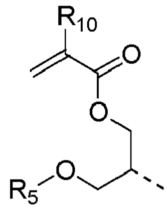
(27)

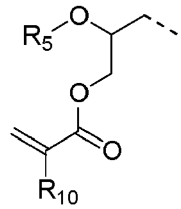
(28)

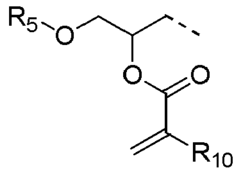
(45)

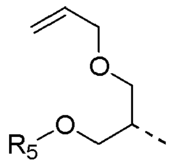
(29)

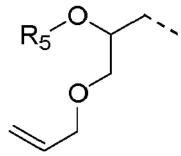
(30)

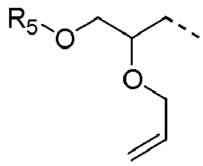
(46)

, wherein $R_{10}$ is H or methyl.

The present invention is further directed to a resin formulation comprising the reactive diluent as defined herein.

**Detailed description of the invention**

The present invention refers to a resin formulation comprising, preferably consisting essentially of, more preferably consisting of,

a) an oligomer comprising, preferably consisting essentially of, more preferably consisting of, 3 to 20, preferably 3 to 10, more preferably 3 to 5, most preferably 3, structural units independently selected from formula (1), formula (2) and formula (35):

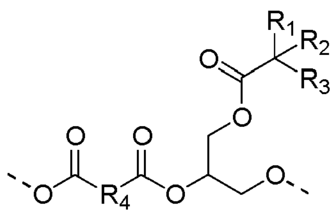
(1)

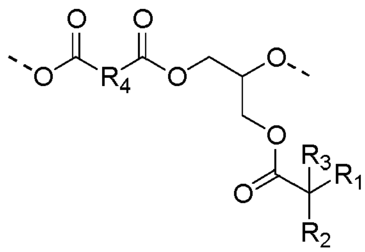
(2)

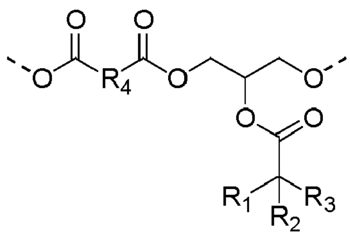
(35)

, wherein the structural units of the oligomer can be the same or can be different,
wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably in the range from 3 to 12, further preferred in the range from 4 to 12, particularly preferred in the range from 6 to 10, most preferably $R_1 + R_2 = 7$,
wherein $R_3$ is methyl,
wherein $R_4$ is a divalent organic group comprising one or more, preferably one, (UV curable) carbon-carbon double bond(s);

b) a reactive diluent (comprising one or more, preferably one, (UV curable) carbon-carbon double bond(s)), wherein the reactive diluent comprises exactly one structural unit according to formulae (1), (2) or (35);
c) (optionally) a photoinitiator;

wherein the invention additionally or alternatively refers to a resin formulation comprising, preferably consisting essentially of, more preferably consisting of,

a) an oligomer comprising, preferably consisting essentially of, more preferably consisting of, 3-20, preferably 3 to 10, more preferably 3 to 5, most preferably 3, structural units each comprising exactly one group according to formula (1'):

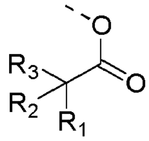 (1')

, wherein the dashed line represents the bond to the backbone of the oligomer,
wherein $R_1$, $R_2$ are independently selected from the group consisting of hydrogen and linear or branched unsubstituted $C_{1-11}$ alkyl groups,
wherein the total number of carbon atoms of $R_1$ and $R_2$ is in the range from 1 to 12, preferably from 3 to 12, further preferred from 4 to 12, particularly preferred from 6 to 10, most preferably is $R_1 + R_2 = 7$,
wherein $R_3$ is methyl, and
wherein each unit can additionally contain exactly one (UV curable) carbon-carbon double bond, wherein at least one unit comprises exactly one (UV curable) carbon-carbon double bond;

b) a reactive diluent comprising
at least one, preferably one, (UV curable) carbon-carbon double bond, and one or two groups according to formula (1'), and wherein the reactive diluent does not comprise repetition units;
c) (optionally) a photoinitiator.

If the oligomer consists (essentially) of 3-20 structural units comprising only structural units of formulae (1), (2) and (35), the oligomer will additionally comprise two end groups which are connected to said formula (1)/(2)/(35), such as the end groups such as hydrogen atoms or $C_{1-6}$ substituted or unsubstituted alkyl groups.
The reactive diluent is preferably neither an oligomer nor a polymer. In one embodiment the molecular weight of the reactive diluent is more than 300 g/mol and below 1500 g/mol, or more than 300 g/mol and below 1000 g/mol, or not more than 800, or not more than 700, or not more than 500 g/mol. Preferably, the reactive diluent does not comprise more than 5, or more than 4, or more than 3, or more than 2 (UV curable) carbon-carbon double-bonds. In a preferred embodiment of the present invention the reactive diluent comprises, preferably contains, one (UV curable) carbon-carbon double-bond.
In one embodiment the molecular weight of the oligomer is more than 1000 g/mol and below 5000 g/mol, or the molecular weight of the oligomer is more than 1000 g/mol and below 3000 g/mol.
If the oligomer consists (essentially) of 3-20 structural units comprising exactly one substituent of formula (1') or only structural units of formulae (1), (2) and (35) (each comprising one substituent of formula (1'), the oligomer contains as many structural units as substituents of formula (1').
In one embodiment all except one structural unit comprise a (UV curable) carbon-carbon double-bond.
In one embodiment of the present invention the oligomer comprises, preferably consists essentially of, more preferably consists of, repetition units or (co-)monomers based on itaconic acid/anhydride and groups one or two groups according to formula (1').

In one embodiment of the present invention the oligomer comprises/has OH groups.

Preferably, the oligomer represents the (uncured) resin of the resin formulation.

The difference between formulae (1), (2) and (35) is that the one oxygen atom which is adjacent to the linking of the structural unit in the oligomer (represented by dashed line) and which is closer to the ester group carrying $R_1$, $R_2$ and $R_3$ (than the other one oxygen atom which is also adjacent to the linking of the structural unit in the oligomer) is bonded to a primary carbon atom in formulae (1) and (35) (i.e. has a -$CH_2$O--- group), whereas said oxygen atom is bonded to a secondary carbon atom in formula (2) (i.e. has a -CHRO--- group). For better understanding, this is shown in the following schemes:

secondary carbon atom (i.e. -CHRO--- group)

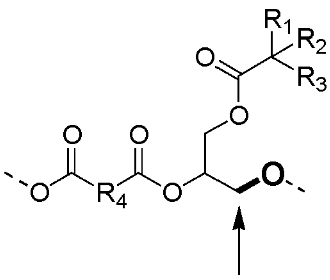

(1)

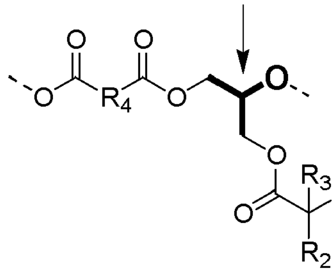

(2)

primary carbon atom (i.e. -$CH_2$O--- group)

primary carbon atom (i.e. -$CH_2$O--- group)

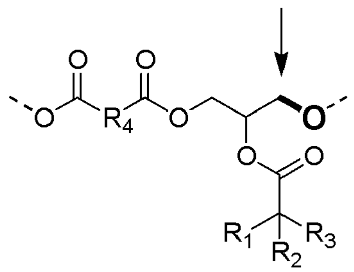

(35)

This difference also applies analogously to the other structures in this document and the claims (i.e. at least to formulae (6), (7), (36), (9), (10), (37), (11), (12), (38), (15), (16), (39), (17), (18), (40), (33), (34), (47), (21), (22), (42), (19), (20), (41) and (23) to (30), (43) to (46).

The oligomer can comprise or consist (essentially) of each of formulae (1), (2) and (35) in one oligomer molecule, whereas the diluent comprises either formula (1) or formula (2) or formula (35).

The resin formulation can comprise oligomers comprising, preferably consisting essentially of, more preferably consisting of, only structural units of formula (1) and/or oligomers comprising, preferably consisting essentially of, more preferably consisting of, only structural units of formula (2) and/or oligomers comprising, preferably consisting essentially of, more preferably consisting of, only structural units of formula (35). The resin formulation can also comprise oligomers comprising, preferably consisting essentially of, more preferably consisting of, combinations of formulae (1), (2), (35), i.e. the resin formulation can comprise (co-)oligomers comprising, preferably consisting essentially of, more preferably consisting of, structural units of formula (1) and/or structural units of formula (2) and/or structural units of formula (35).

The resin formulation can comprise diluents comprising only structural units of formula (1) and/or diluents comprising only structural units of formula (2) and/or diluents comprising only structural units of formula (35), i.e. the resin formulation can comprise a mixture of reactive diluents (each comprising exactly one structural unit according to formulae (1), (2) or (35)), such as a resin formulation comprising a reactive diluent comprising exactly one structural unit according to formula (1), and/or a reactive diluent comprising exactly one structural unit according to formula (2), and/or a reactive diluent comprising exactly one structural unit according to formula (35).

Whether structural elements of formula (1), (2) or (35) is present has its origin in the synthesis of the oligomers or diluents. Whereas formulae (1) and (2) are formed via direct opening of an epoxy ring in the synthesis of a glycidyl ester with a carboxylic acid group, formula (35) is formed by ortho-ester mediation. Any of formulae (1), (2) or (35) may be present in the oligomer or diluent by appropriate synthesis. Which of the formulas is preferred depends inter alia on the reaction conditions and/or the substitution pattern of the starting materials. For example, ortho-ester mediation is preferred for the

reaction of acrylic acid with Cardura™ E10P under specific conditions, whereas direct opening yielding a secondary carbon atom as in formula (2) can occur under different conditions. This is exemplarily shown in the following scheme. A similar result is expected for the reaction of itaconic acid/anhydride with Cardura™ E10P.

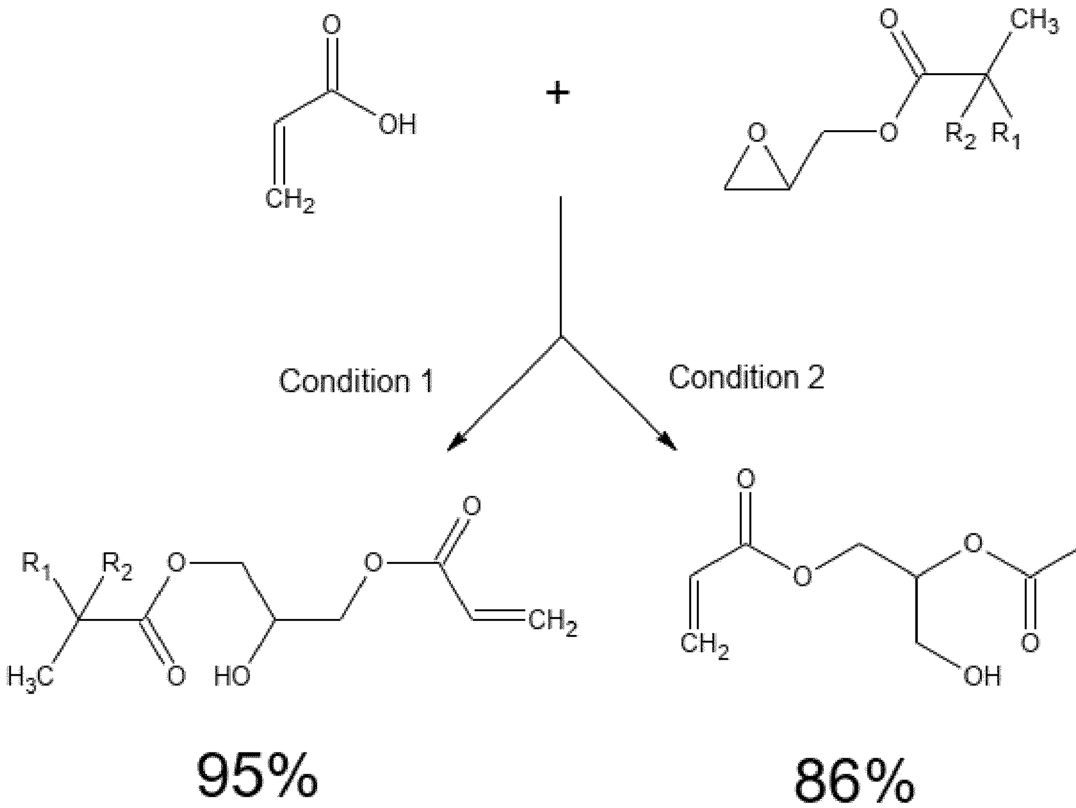


The following scheme exemplarily shows the synthesis of a linear oligomer carrying only structural elements of formula (1) using itaconic acid, itaconic anhydride and Cardura™ E10P (the glycidyl ester of Versatic™ Acid 10) as educts:

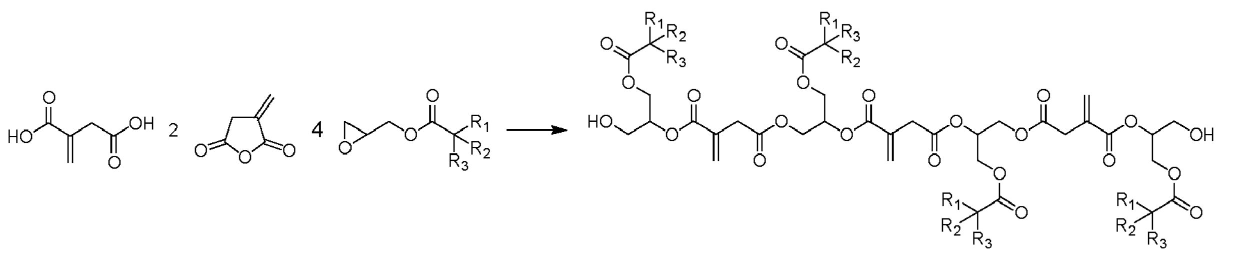

The following scheme exemplarily shows the synthesis of a linear oligomer carrying only structural elements of formula (2) using itaconic acid, itaconic anhydride and Cardura™ E10P (the glycidyl ester of Versatic™ Acid 10) as educts:

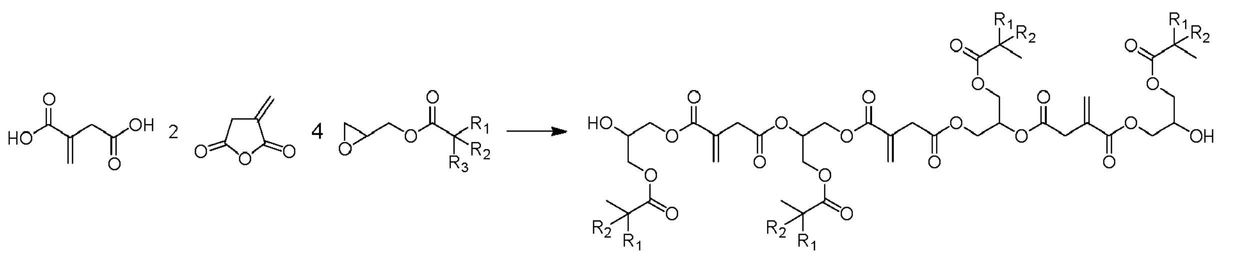

The following scheme shows the mechanism for the ortho-ester mediation:

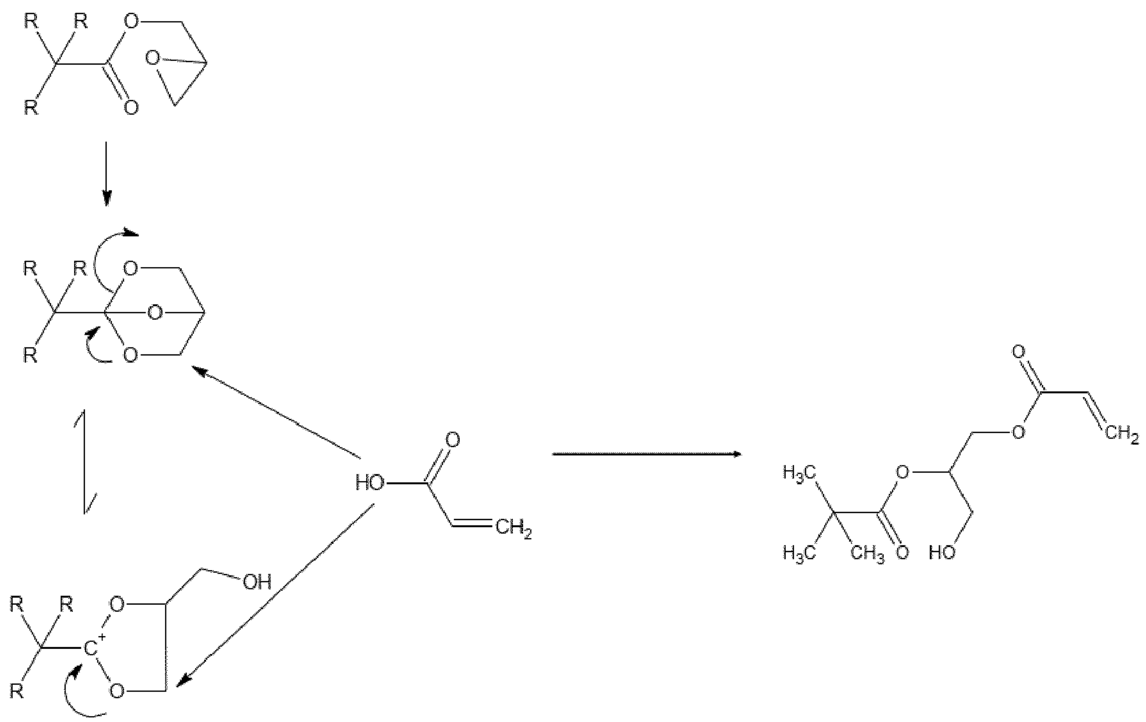

In one embodiment of the present invention the oligomer comprises, preferably consists essentially of, more preferably consists of, only structural units of formula (1) and the oligomer does not comprise structural units of formulae (2) or (35). In one embodiment of the present invention the oligomer comprises, preferably consists essentially of, more preferably consists of, only structural units of formula (2) and the oligomer does not comprise structural units of formulae (1) or (35). In one embodiment of the present invention the oligomer comprises, preferably consists essentially of, more preferably consists of, only structural units of formula (35) and the oligomer does not comprise structural units of formulae (1) or (2).

In one embodiment of the present invention the oligomer comprises, preferably consists essentially of, more preferably consists of, each of structural units of formulae (1), (2) and (35). In one embodiment of the present invention the resin formulation comprises, oligomers comprising, preferably consisting essentially of, more preferably consisting of, only structural units of formula (1) and/or oligomers comprising, preferably consisting essentially of, more preferably consisting of, only structural units of formula (2) and/or oligomers comprising, preferably consisting essentially of, more preferably consisting of, only structural units of formula (35) and/or oligomers comprising, preferably consisting essentially of, more preferably consisting of, structural units of formulae (1), (2) and (35) and/or combinations thereof.

The following scheme exemplarily shows the synthesis of a reactive diluent carrying only structural elements of formula (1) using cyclohexanol, itaconic anhydride and Cardura™ E10P (the glycidyl ester of Versatic™ Acid 10) as educts:

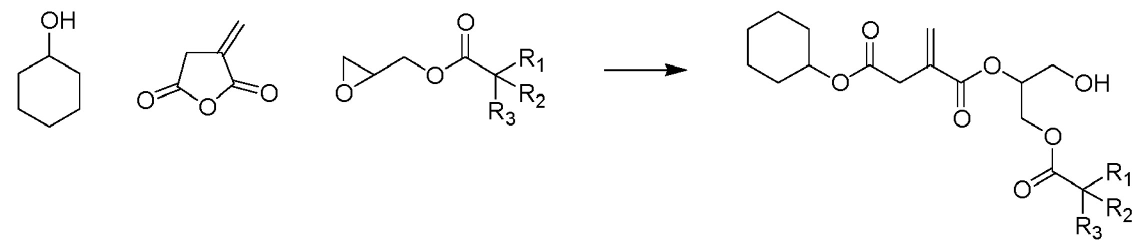

The following scheme exemplarily shows the synthesis of a reactive diluent carrying only structural elements of formula (2) using cyclohexanol, itaconic anhydride and Cardura™ E10P (the glycidyl ester of Versatic™ Acid 10) as educts:

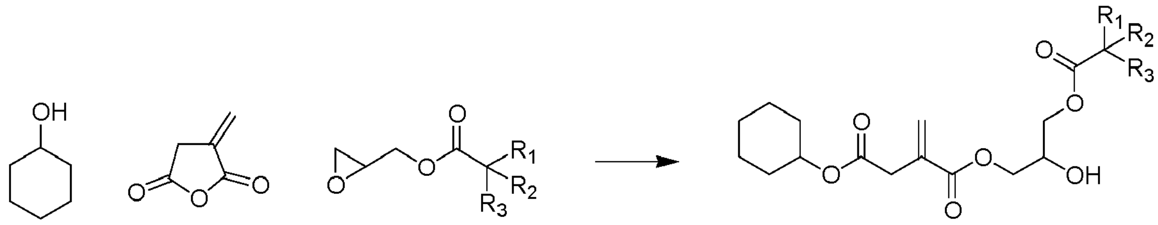

The following scheme exemplarily shows the synthesis of a reactive diluent carrying only structural elements of formula (35) using cyclohexanol, itaconic anhydride and Cardura™ E10P (the glycidyl ester of Versatic™ Acid 10) as educts:

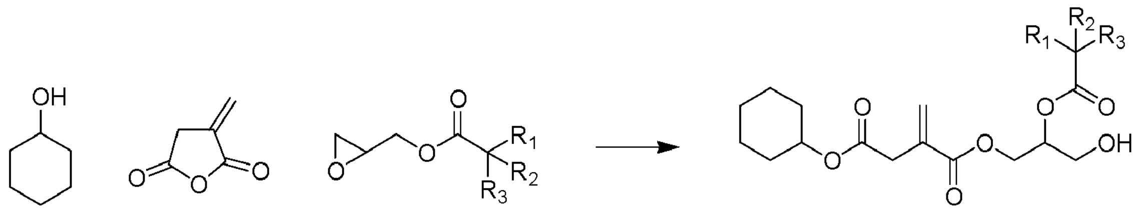

In one embodiment of the present invention the resin formulation comprises reactive diluents comprising only structural units of formula (1) and/or reactive diluents comprising only structural units of formula (2) and/or reactive diluents comprising only structural units of formula (35). In one embodiment of the present invention the resin formulation comprises reactive diluents comprising only structural units of formula (1). In one embodiment of the present invention the resin formulation comprises reactive diluents comprising only structural units of formula (2). In one embodiment of the present invention the resin formulation comprises reactive diluents comprising only structural units of formula (35). In one embodiment of the present invention the resin formulation comprises a mixture of a reactive diluent comprising a structural unit of formula (1) and a reactive diluent comprising a structural unit of formula (2) and a reactive diluent comprising a structural unit of formula (35).

In one embodiment of the present invention the oligomer comprises, preferably consisting essentially of, more preferably consisting of, 3 to 20, structural units represented by formula (1):

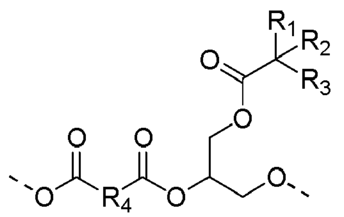

(1)

, wherein the dashed lines represent the linking in the oligomer,

wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12,

wherein $R_3$ is methyl,

wherein $R_4$ is a divalent organic group comprising one or more, preferably one, UV curable carbon-carbon double bonds;

and

the reactive diluent comprises exactly one structural unit according to formula (1).

In one embodiment of the present invention the oligomer comprises preferably consisting essentially of, more preferably consisting of, 3 to 20, structural units represented by formula (2):

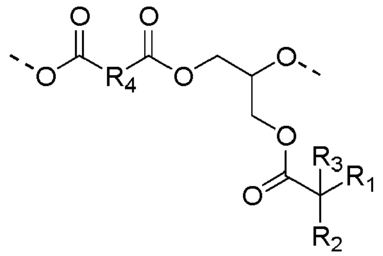

(2)

, wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12,
wherein $R_3$ is methyl,
wherein $R_4$ is a divalent organic group comprising one or more, preferably one, UV curable carbon-carbon double bonds;
and
the reactive diluent comprises exactly one structural unit according to formula (2).

In one embodiment of the present invention the oligomer comprises, preferably consisting essentially of, more preferably consisting of, 3 to 20, structural units represented by formula (35):

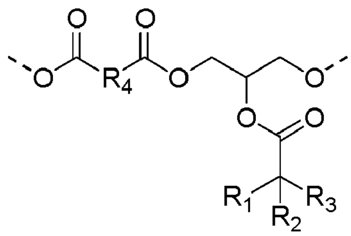

(35)

, wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12,
wherein $R_3$ is methyl,
wherein $R_4$ is a divalent organic group comprising one or more, preferably one, UV curable carbon-carbon double bonds;
and
the reactive diluent comprises exactly one structural unit according to formula (35).

In one embodiment of the present invention the total number of carbon atoms of $R_4$ is below 10, preferably below 7, more preferably below 5.

In one embodiment of the present invention $R_4$ can be selected from the group of formulae (3), (4) and (5):

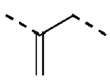 (3) 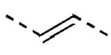 (4) 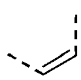 (5)

wherein the $R_4$-formulae of the structural units of the oligomer can be the same or can be different.

In one embodiment of the present invention $R_4$ is represented by formula (3):

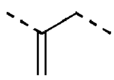 (3)

.

In one embodiment of the present invention the resin formulation comprises at least 1 wt% of the oligomer in (a) based on the total weight of the resin formulation and at least 1 wt% of the reactive diluent in (b) based on the total weight of the resin formulation;

preferably the resin formulation is liquid.

The resin formulation is preferably liquid.

In one embodiment of the present invention the resin formulation has less than 5 wt% of organic solvents, preferably contains no organic solvents.

The structural units of the oligomer independently selected from formulae (1), (2) and (35) incorporate bulky and hydrophobic groups via the ester group carrying $R_1$, $R_2$ and $R_3$ and one or more UV curable carbon-carbon double bonds via $R_4$ which can be addressed in the (UV) curing process. The functionalities inter alia impart desirable properties to the (cured) resin formulation such as excellent compatibility with polar solvents, excellent acid and alkali resistance (through the sterically protected ester group), superior outdoor durability, improved gloss, improved pigment utilization, low solution viscosity, high solids resins, improved polar solvent resistance, excellent solubility in aliphatic solvents, excellent adhesion to apolar substrates and a reduced/controlled shrinkage.

The structural units of the reactive diluent independently selected from formulae (1), (2) and (35) incorporate bulky and hydrophobic groups via the ester group carrying $R_1$, $R_2$ and $R_3$ and one or more UV curable carbon-carbon double bonds via $R_4$ which can be addressed in the (UV) curing process. The reactive diluent provides specific properties and lowers the viscosity of the formulation while entering in the curing process and limiting the use of solvents. The structural similarities enable a good compatibility between the components, particularly between the oligomer and the diluent. The benefits of the reactive diluent of the invention for a respective resin formulation are expected to be similar if compared to the oligomers of the invention. In addition to that, main advantages are seen in pigment wetting, adhesion to difficult apolar substrates, control of shrinkage, and a better

Health, Safety and Environement (HSE) profile related to low volatility and lower Draize value (skin irritation). For the ACE™ hydroxyl acrylate monomer (the adduct of acrylic acid and the glycidyl ester of neodecanoic acid) a Draize value of 0.9 has been found which is lower than for other acrylate monomers. It is believed that the lower irritation number is due to the bulkiness of the ACE™ hydroxyl acrylate monomer. Further, the reactive diluent monomer can contribute specific properties related to its structure and function.

In one embodiment of the present invention the oligomer comprises/has OH groups. In one embodiment of the present invention the reactive diluent comprises/has OH groups. In one embodiment of the present invention the oligomer and the reactive diluent comprise/have OH groups.

Oligomers or reactive diluents that comprise/have OH groups can further be incorporated into the network (the cured resin) by reacting with isocyanate or melamine, for example. This makes it possible to provide "dual-curing/curable" resin formulations, which is an extension of the application field.

In one embodiment of the present invention the resin formulation is free of a catalyst selected from the group consisting of triphenylphosphine, FASCAT® 4101, sulfonic acids, Lewis acids, such as tin-based Lewis acids, titanium-based Lewis acids, or $AlCl_3$, $SnCl_2$, $FeCl_3$, preferably wherein the oligomer and/or the reactive diluent is free of any catalyst.

In one embodiment of the present invention the oligomer is present in the resin formulation in an amount from 40 wt.-% to 70 wt.-%, more preferably in an amount from 50 wt.-% to 60 wt.-%, based on the total weight of the resin formulation.

In one embodiment of the present invention the reactive diluent is present in the resin formulation in an amount from 30 wt.-% to 70 wt.-%, more preferably in an amount from 40 wt.-% to 60 wt.-%, based on the total weight of the resin formulation.

In one embodiment of the present invention the photoinitiator is present in the resin formulation in an amount from 0.5 wt.-% to 10 wt.-%, more preferably in an amount from 0.5 wt.-% to 5 wt.-%, particularly preferably in an amount of 0.5 wt.-% to 3 wt.-%, based on the total weight of the resin formulation.

In one embodiment of the present invention the photoinitiator is selected from the group consisting of: 2-benzyl-2-(dimethylamino)-4'-morpholinobutyrophenone, 2-hydroxy-2-methylpropiophenone, tri methyl benzophenone, methyl benzophenone, 1-hydroxycyclohexylphenyl ketone, isopropyl thioxanthone, 2,2-dimethyl-2-hydroxy-acetophenone, 2,2-dimethoxy-2-phenylacetophenone, 2-methyl-1-[4-(methylthio)phenyl]-2-morpholino-propan-1-one, 2,4,6-trimethylbenzyl-diphenyl-phosphine oxide, 1-chloro-4- propoxythioxanthone, benzophenone, bis(2,6-dimethoxybenzoyl)-2,4,4-trimethyl pentyl phosphine oxide, 5,7-diiodo-3-butoxy-6-fluorone, ethyl 2,4,6-trimethylbenzoylphenylhosphinate, oxy-phenyl-acetic acid 2-[2-oxo-2-phenyl-acetoxy-ethoxy]-ethyl ester, oxy-phenyl acetic acid 2-[2-hydroxy-ethoxy]-ethyl ester, 1-phenyl-2-hydroxy-2-methyl propanone, bis(2,4,6-trimethylbenzoyl)phenylphosphine oxide, camphorquinone, polymeric-photoinitiators such as polymeric benzophenone, and combinations thereof;

preferably the photoinitiator comprises, more preferably consists (essentially) of, a blend of 2,4,6-trimethylbenzyl-diphenyl-phosphine oxide and 2-hydroxy-2-methylpropiophenone.

In one embodiment of the present invention the resin formulation is further comprising:

d) an inhibitor, preferably in an amount from 0.1 wt.-% to 1 wt.-%, more preferably in an amount from 0.1 wt.-% to 0.7 wt.-%, particularly preferably in an amount of 0.1 wt.-% to 0.5 wt.-%, based on the total weight of the resin formulation;

e) further additives.

In one embodiment of the present invention the resin formulation comprising, preferably consisting essentially of, more preferably consisting of,

a) an oligomer comprising, preferably consisting essentially of, more preferably consisting of, 3 to 20, preferably 3 to 10, more preferably 3 to 5, most preferably 3, structural units independently selected from formula (1), formula (2) and formula (35):

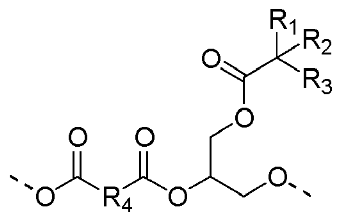
(1)
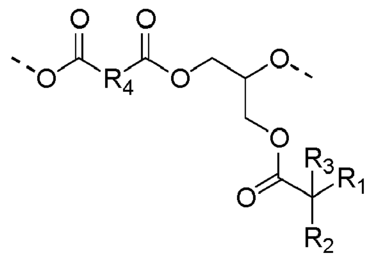
(2)

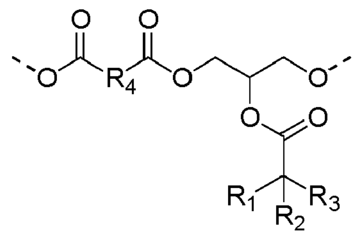
(35)

, wherein the structural units of the oligomer can be the same or can be different,
wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12, particularly preferred from 6 to 10, most preferably is $R_1 + R_2 = 7$,
wherein $R_3$ is methyl,
wherein $R_4$ is a divalent organic group comprising one or more, preferably one, (UV curable) carbon-carbon double bond(s),
preferably in an amount from 40 wt.-% to 70 wt.-%, more preferably in an amount from 50 wt.-% to 60 wt.-%, based on the total weight of the resin formulation;

b) a reactive diluent (comprising one or more, preferably one, (UV curable) carbon-carbon double bond(s)), wherein the reactive diluent comprises exactly one structural unit according to formulae (1), (2) or (35),
preferably in an amount from 30 wt.-% to 70 wt.-%, more preferably in an amount from 40 wt.-% to 60 wt.-%, based on the total weight of the resin formulation;
c) a photoinitiator, preferably in an amount from 0.5 wt.-% to 10 wt.-%, more preferably in an amount from 0.5 wt.-% to 5 wt.-%, particularly preferably in an amount of 0.5 wt.-% to 3 wt.-%, based on the total weight of the resin formulation;
d) an inhibitor, preferably in an amount from 0.1 wt.-% to 1 wt.-%, more preferably in an amount from 0.1 wt.-% to 0.7 wt.-%, particularly preferably in an amount of 0.1 wt.-% to 0.5 wt.-%, based on the total weight of the resin formulation;
e) further additives.

The proportions of each of these components are chosen according to the desired viscosity and the different properties to be given to the resin.

In one embodiment of the present invention the oligomer has a linear structure or a star-shaped structure.

In one embodiment of the present invention the oligomer has a linear structure.

In one embodiment of the present invention the oligomer has a star-shaped structure, preferably with 3 arms.

In one embodiment of the present invention the oligomer further comprises one or more moieties selected from formula (6), formula (7) and formula (36):

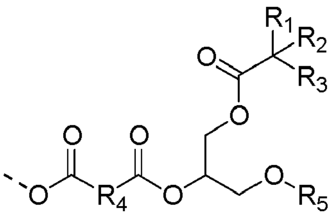 (6) 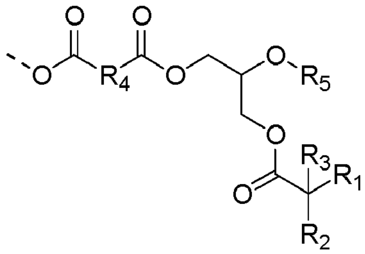 (7)

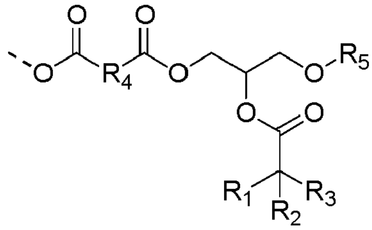 (36)

, wherein $R_1$, $R_2$, $R_3$ are as defined herein at any occurrence,

wherein $R_4$ is a divalent organic group comprising one or more, preferably one, (UV curable) carbon-carbon double bonds,

wherein $R_5$ is H or represented by formula (8):

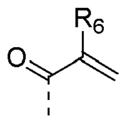 (8)

, wherein $R_6$ is H or methyl.

Formulae (6), (7) and (36) represent end groups that are preferably comprised by the oligomer. $R_5$ represents H or a (meth) acrylic group. These groups can be addressed in the curing process via respective curing mechanisms. That is, the end groups represented by formulae (6), (7) and (36) allow an improved curing by additional curing functionalities.

In one embodiment of the present invention the oligomer further comprises one or more moieties selected from formula (6), formula (7) and formula (36):

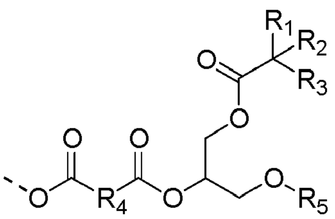 (6) 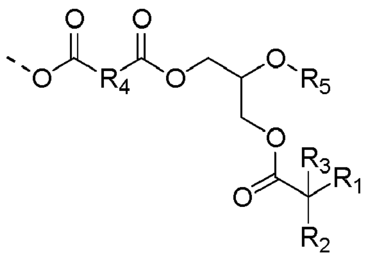 (7)

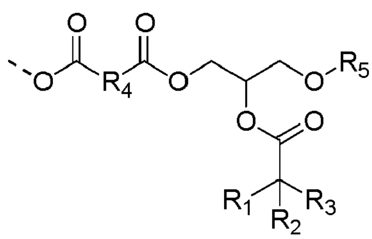 (36)

, wherein $R_1$, $R_2$, $R_3$ are as defined herein at any occurrence,

wherein $R_4$ is a divalent organic group comprising one or more, preferably one, UV curable carbon-carbon double bonds,

wherein $R_5$ is H.

In one embodiment of the present invention the oligomer further comprises one or more moieties selected from formula (6), formula (7) and formula (36):

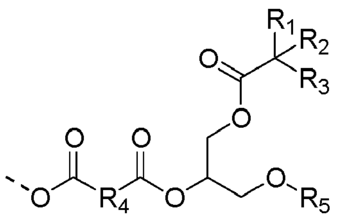 (6) 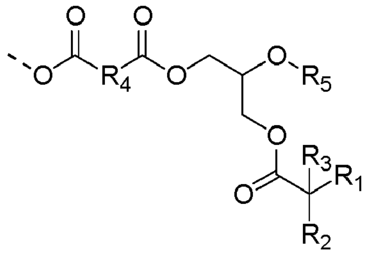 (7)

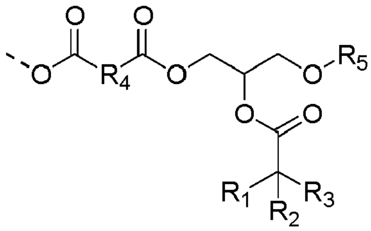 (36)

, wherein $R_1$, $R_2$, $R_3$ are as defined herein at any occurrence,
wherein $R_4$ is selected from formulae (3), (4) and (5) and the $R_4$-formulae can be the same or can be different:

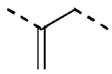 (3) 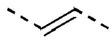 (4) 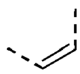 (5)

, wherein $R_5$ is H or represented by formula (8):

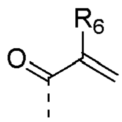 (8)

, wherein $R_6$ is H or methyl.

In one embodiment of the present invention the oligomer further comprises one or more moieties selected from formula (6), formula (7) and formula (36):

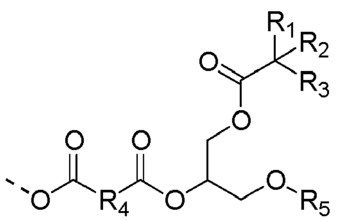 (6) 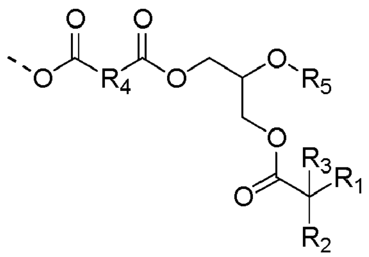 (7)

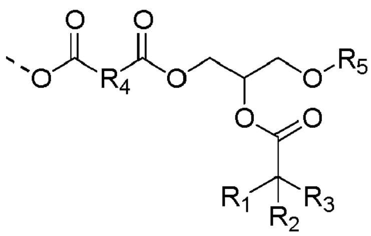 (36)

, wherein $R_1$, $R_2$, $R_3$ are as defined herein at any occurrence,
wherein $R_4$ is selected from formulae (3), (4) and (5) and the $R_4$-formulae can be the same or can be different:

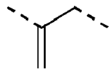 (3) 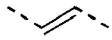 (4) 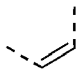 (5)

, wherein $R_5$ is H.

In one embodiment of the present invention the oligomer further comprises one or more moieties selected from formula (6), formula (7) and formula (36):

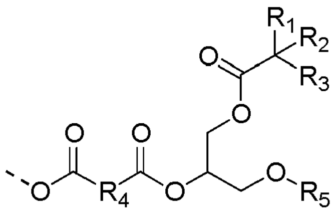 (6) 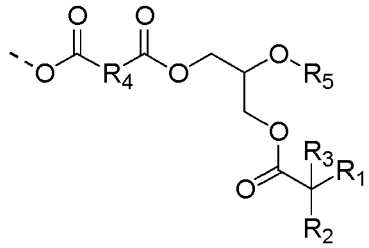 (7)

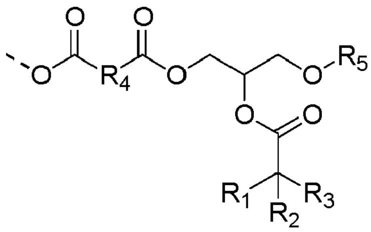 (36)

, wherein $R_1$, $R_2$, $R_3$ are as defined herein at any occurrence,
wherein $R_4$ is represented by formula (3):

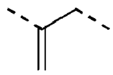 (3)

, wherein $R_5$ is H.

In one embodiment of the present invention the oligomer further comprises one or more moieties selected from formula (6), formula (7) and formula (36):

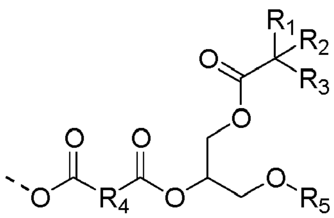 (6) 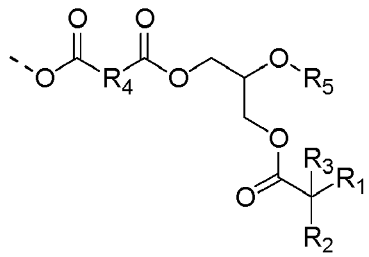 (7)

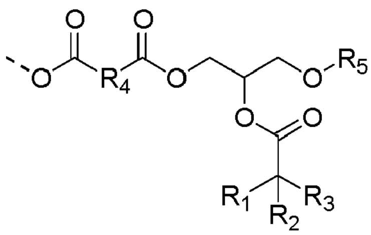 (36)

, wherein $R_1$, $R_2$, $R_3$ are as defined herein at any occurrence,
wherein $R_4$ is represented by formula (3):

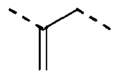 (3)

, wherein $R_5$ is H or represented by formula (8):

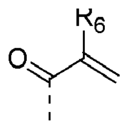 (8)

, wherein $R_6$ is H or methyl.

In one embodiment of the present invention the oligomer further comprises one or more moieties selected from formula (6), formula (7) and formula (36):

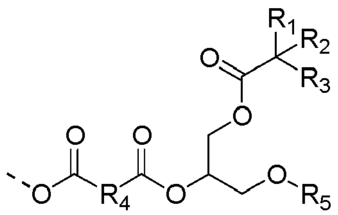 (6) 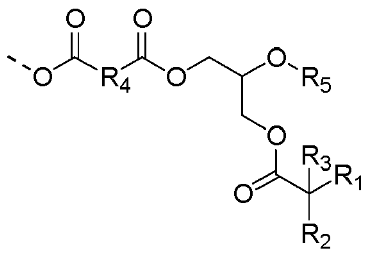 (7)

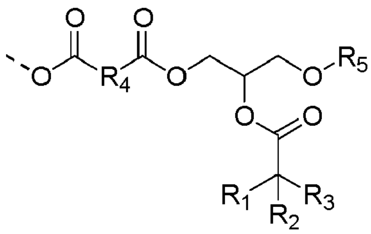 (36)

, wherein $R_1$, $R_2$, $R_3$ are as defined herein at any occurrence,
wherein $R_4$ is a divalent organic group comprising one or more, preferably one, UV curable carbon-carbon double bonds,
wherein $R_5$ is H or represented by formula (8):

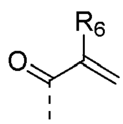 (8)

, wherein $R_6$ is H or methyl;
optionally the oligomer further comprises one or more structural units selected from formulae (9) to (12), formula (37) and formula (38):

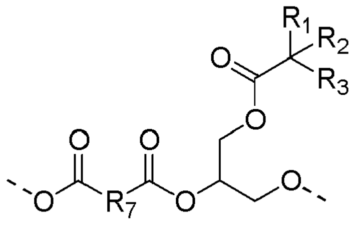 (9)

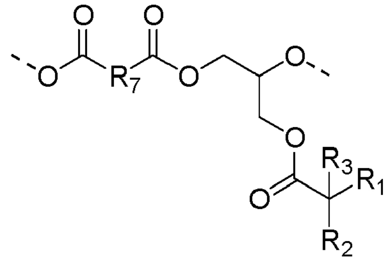 (10)

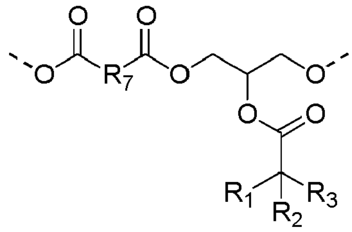 (37)

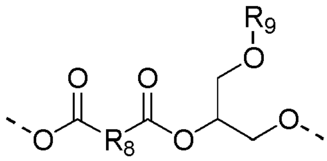 (11)

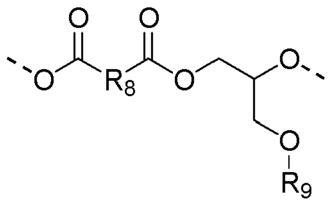 (12)

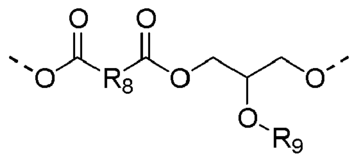 (38)

, wherein the structural units can be the same or different,

wherein $R_7$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,

wherein $R_8$ is a divalent organic group comprising one or more UV curable carbon-carbon double bonds, or

$R_8$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,

wherein $R_9$ is selected from formula (13) and formula (14),

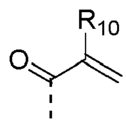 (13) 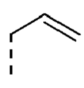 (14)

, wherein $R_{10}$ is H or methyl,

preferably optionally

the oligomer further comprises one or more moieties selected from formulae (15) to (18), formula (39) and formula (40):

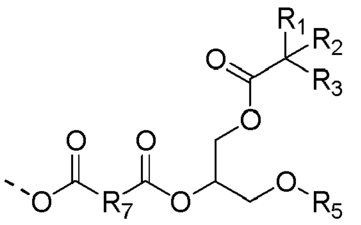
(15)

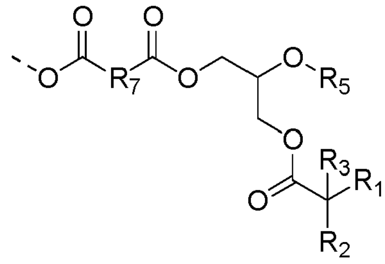
(16)

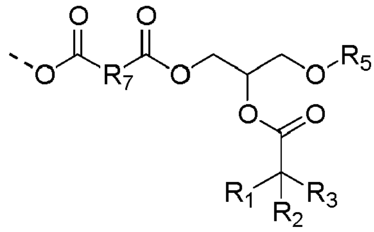
(39)

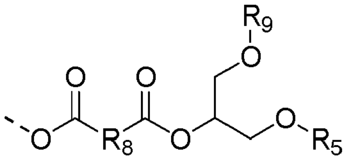
(17)

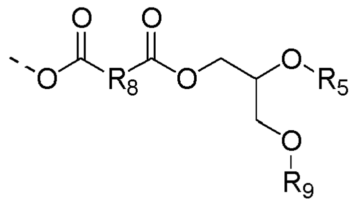
(18)

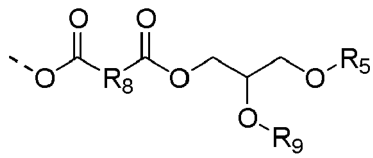
(40)

, wherein the moieties can be the same or different.

Formulae (9), (10) and (37) represent additional structural units that are preferably comprised by the oligomer. $R_7$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups. These groups allow a tuning of the properties of the resulting oligomer, and thus, allow for a tuning of the properties of the resin formulation as well as the cured composition.

Formulae (11), (12) and (38) represent additional structural units that are preferably comprised by the oligomer. $R_9$ represents (meth) acrylic or vinyl groups. These groups can be addressed in the curing process via respective curing mechanisms. That is, the groups represented by formulae (11), (12) and (38) allow an improved curing by additional curing functionalities.

Formulae (15) to (18) and (39) to (40) represent end groups that are preferably comprised by the oligomer. These groups allow both a tuning of the properties of the resulting oligomer/resin formulation/cured composition, and an improved curing by additional curing functionalities.

In one embodiment of the present invention the oligomer further comprises one or more moieties selected from formula (6), formula (7) and formula (36):

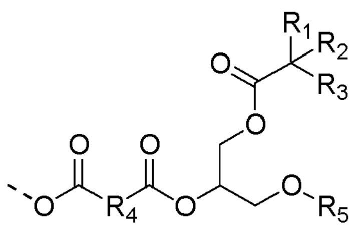
(6)

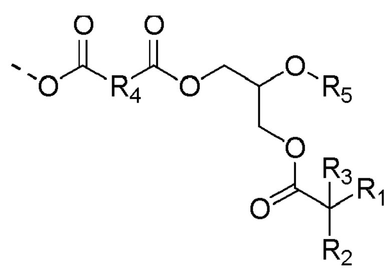
(7)

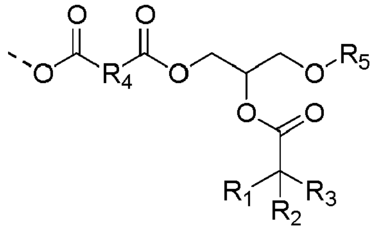

(36)

, wherein $R_1$, $R_2$, $R_3$ are as defined herein at any occurrence, wherein $R_4$ is selected from formulae (3), (4) and (5) and the $R_4$-formulae can be the same or can be different:

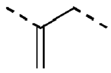 (3) 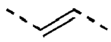 (4) 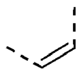 (5)

, wherein $R_5$ is H or represented by formula (8):

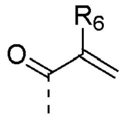 (8)

, wherein $R_6$ is H or methyl;
optionally the oligomer further comprises one or more structural units selected from formulae (9) to (12), formula (37) and formula (38):

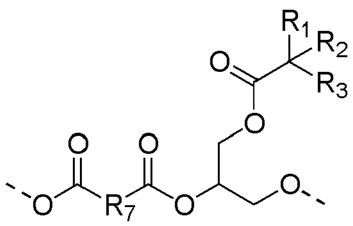 (9)

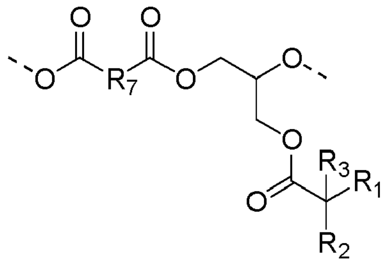 (10)

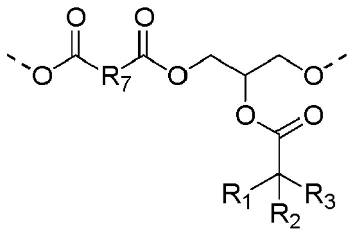

(37)

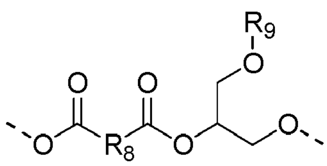 (11)

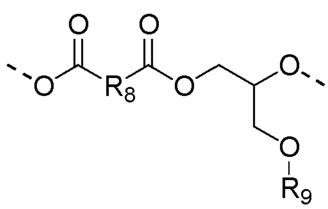 (12)

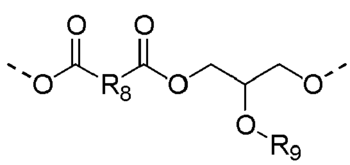

(38)

, wherein the structural units can be the same or different,
wherein $R_7$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,
wherein $R_8$ is selected from formulae (3), (4) and (5):

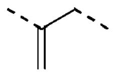 (3) 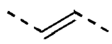 (4) 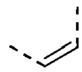 (5)

, or $R_8$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,
wherein $R_9$ is selected from formula (13) and formula (14),

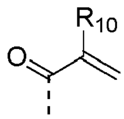 (13) 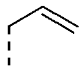 (14)

, wherein $R_{10}$ is H or methyl,
preferably optionally
the oligomer further comprises one or more moieties selected from formulae (15) to (18), formula (39) and formula (40):

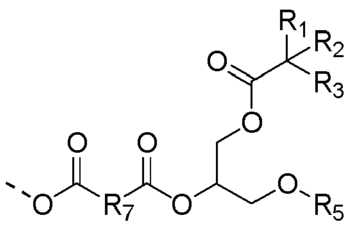 (15) 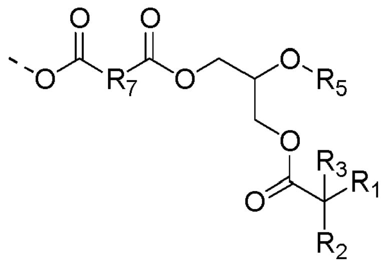 (16)

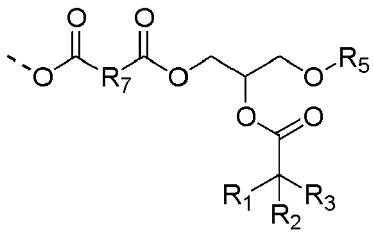 (39)

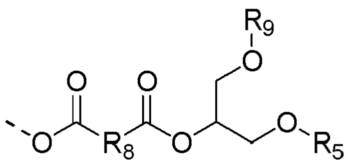 (17) 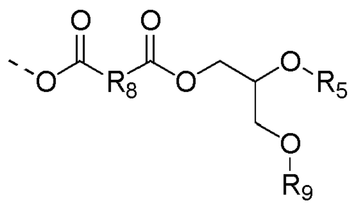 (18)

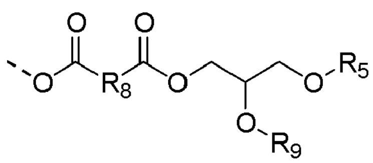 (40)

, wherein the moieties can be the same or different.

In one embodiment of the present invention the oligomer is selected from formulae (33), (34), (47), (21), (22) and (42):

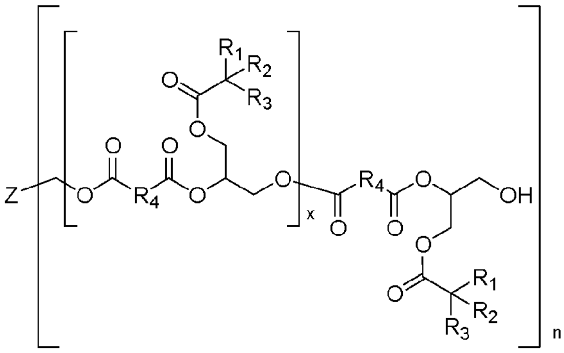

(33)

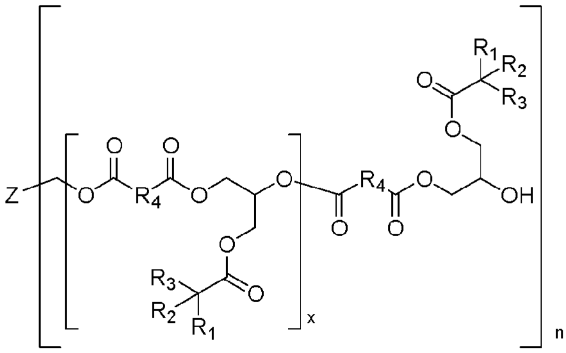

(34)

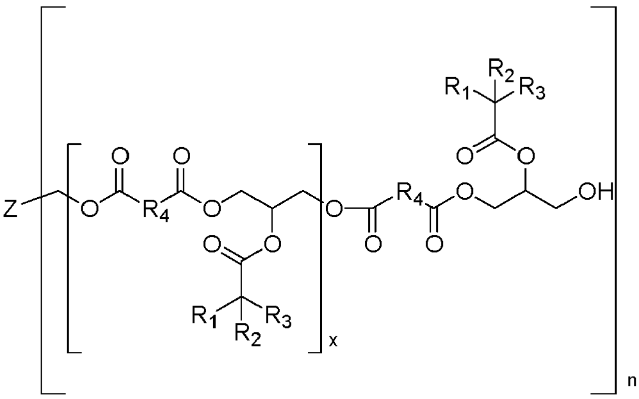

(47)

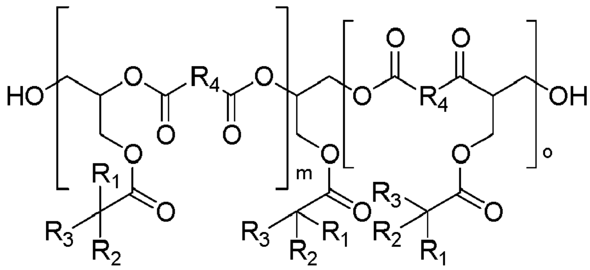

(21)

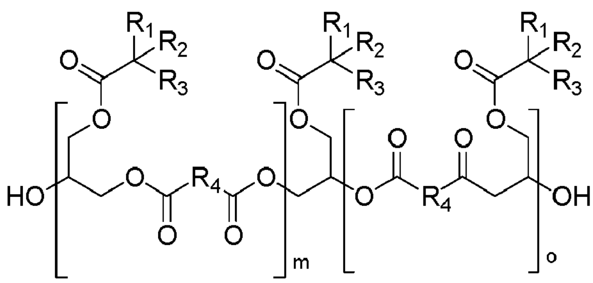

(22)

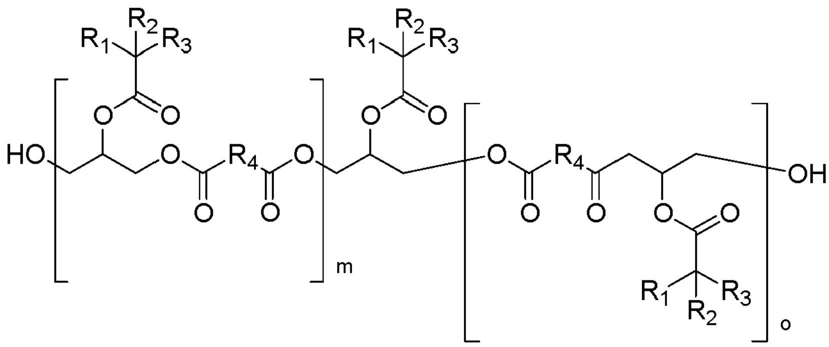

(42)

, wherein $R_1$, $R_2$, $R_3$ are as herein,

wherein $R_4$ is a divalent organic group comprising one or more, preferably one, UV curable carbon-carbon double bonds,

wherein Z represents an n-valent organic group,

wherein n is in the range of 3 to 6,

wherein x is in the range of 0 to 4,

wherein m + o is in the range of 3 to 11.

In one embodiment of the present invention m = 0 and o is in the range of 3 to 11 or m is in the range of 3 to 11 o = 0.

In one embodiment of the present invention Z is an n-valent organic group derived from a polyol comprising at least 3 hydroxyl groups;

wherein at least 3 of the at least 3 hydroxyl groups form the linking of Z to a structural unit of the oligomer;

wherein the polyol is selected from the group consisting of glycerol, trimethylolpropane, pentaerythritol and sugar alcohols with 4-6 hydroxyl groups, preferably the polyol is trimethylolpropane.

In one embodiment of the present invention n is in the range of 3 to 5, preferably n is in the range of 3 to 4, more preferably n = 3. In one embodiment of the present invention x is in the range of 0 to 2, preferably x is in the range of 0 to 1, more preferably x = 0.

In one embodiment of the present invention

m is in the range of 2 to 6 and o is in the range of 1 to 5,

more preferably m is in the range of 2 to 4 and o is in the range of 1 to 3,

most preferably m = 2 and o = 1.

In one embodiment of the present invention the oligomer is selected from formulae (19) to (22), formula (41) and formula (42):

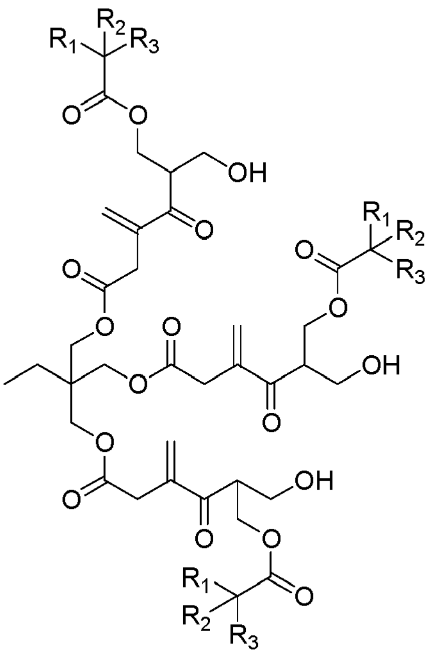

(19)

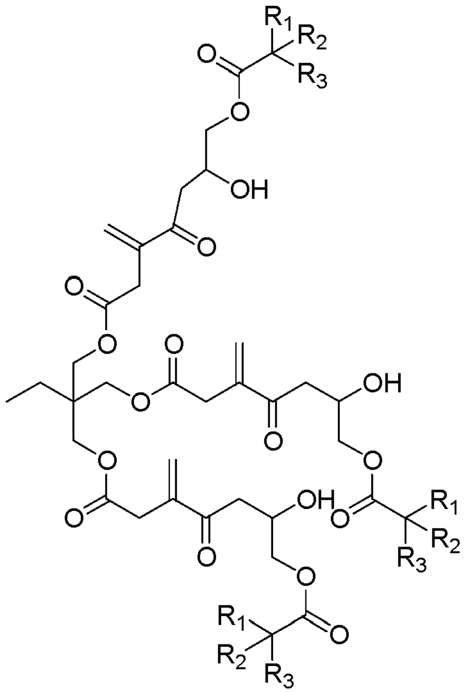

(20)

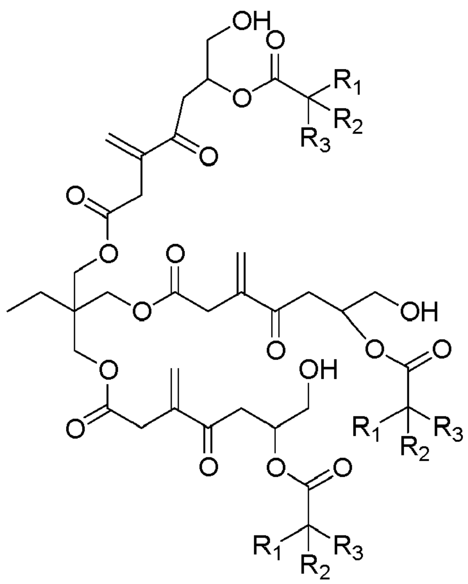

(41)

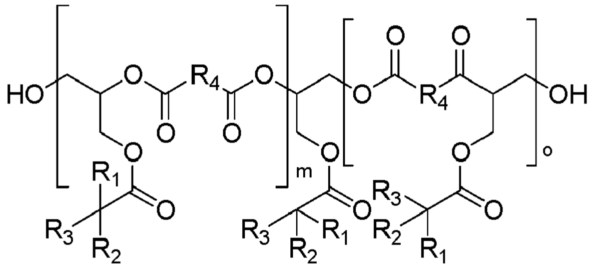

(21)

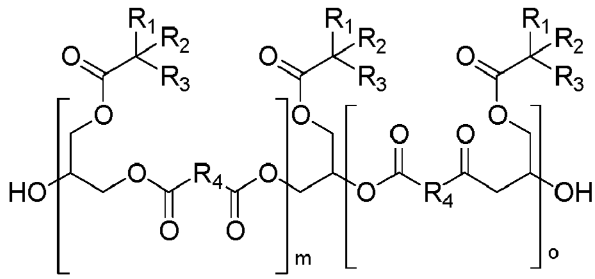

(22)

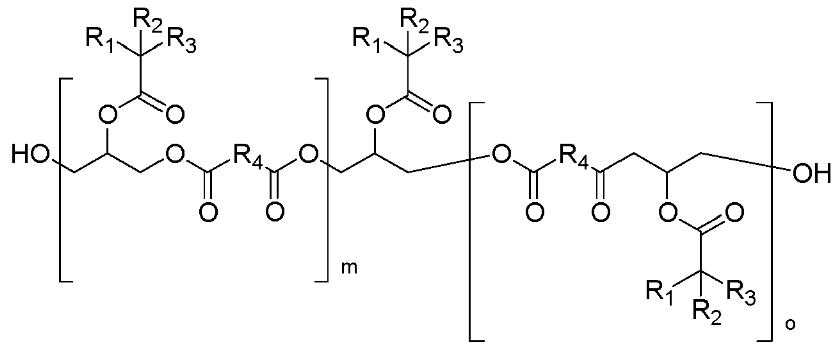

(42)

, wherein $R_1$, $R_2$, $R_3$ are as defined herein at any occurrence,
wherein $R_4$ is a divalent organic group comprising one or more (UV curable) carbon-carbon double bonds,
wherein n is 3,
wherein m + o is in the range of 3 to 11.

Formulae (19) to (22), (33), (34), (41), (42), and (47) represent the preferred chemical structures of the oligomers, i.e. linear polyesters ((21), (22), (42)) or star-shaped polyesters ((19), (20), (41), (33), (34), (47)). These chemical structures/architectures lead to favourable viscosities for the resulting resin formulation and allow for improved processing of the same.

The structures of formulae (19) to (22), (33), (34), (41), (42), and (47) correspond to oligomers formed either via direct opening of an epoxy ring in the synthesis of a glycidyl ester with a carboxylic acid group or via ortho-ester mediation, respectively, i.e. these represent the situation in which only one of these 3 reaction mechanisms have taken place and the others have been suppressed. Under certain reaction conditions, it can be assumed that only one of these 3 reaction mechanisms will actually take place and that the formulae (19) to (22), (33), (34), (41), (42), (47) are created accordingly. Under different reaction conditions, however, it can also be assumed that all three reaction mechanisms take place simultaneously (with certain preferences) and that combinations of the structural units of formulae (19) to (22), (33), (34), (41), (42), and (47) in one co-oligomer and/or mixtures of oligomers of formulae (19) to (22), (33), (34), (41), (42), or (47) are present accordingly. The following scheme shows a structural section of a (co-)oligomer that has a combination of all 3 possible structural units/(co-)monomers, wherein p, q and r represent the molar fraction of said structural units/(co-) monomers in the (co-)oligomer:

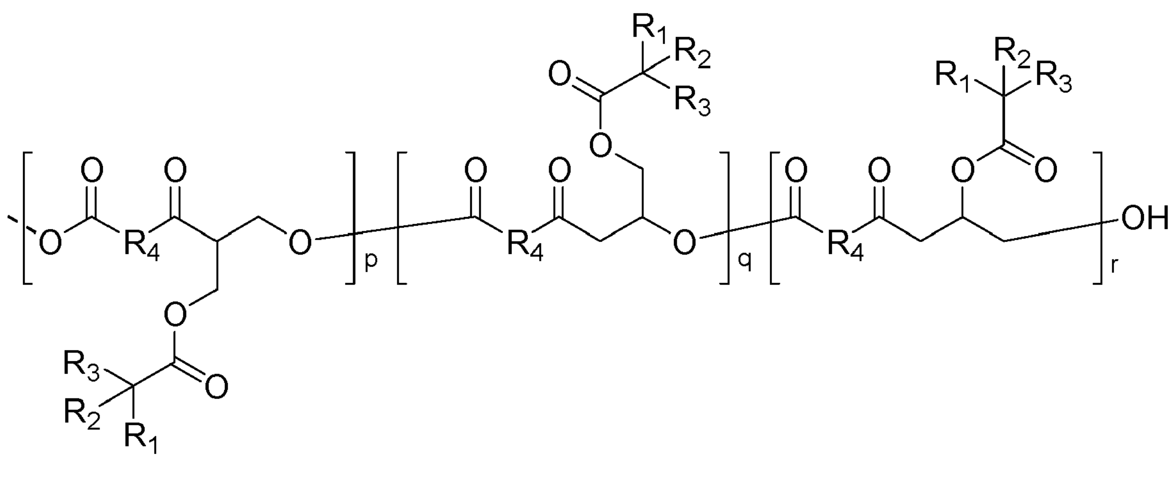

In one embodiment of the present invention $R_4$ is selected from formulae (3), (4) and (5) and can be same or different:

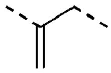 (3) 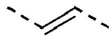 (4) 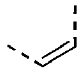 (5) .

In one embodiment of the present invention the oligomer is selected from formulae (19), (20), (41),

wherein $R_1$, $R_2$, $R_3$ are as defined herein at any occurrence,
wherein $R_4$ is selected from formulae (3), (4) and (5) and can be same or different:

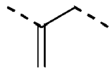 (3) 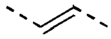 (4) 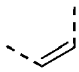 (5)

, preferably $R_4$ is represented by formula (3).

In one embodiment of the present invention the oligomer is selected from formulae (21) to (22) and (42),

wherein $R_1$, $R_2$, $R_3$ are as defined herein at any occurrence,
wherein $R_4$ is selected from formulae (3), (4) and (5) and can be same or different:

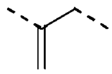 (3) 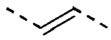 (4) 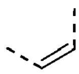 (5)

, preferably $R_4$ is represented by formula (3),
m is in the range of 2 to 6 and o is in the range of 1 to 5,
more preferably m is in the range of 2 to 4 and o is in the range of 1 to 3,
most preferably m = 2 and o = 1.

In one embodiment of the present invention the reactive diluent is independently selected from formula (23), formula (24) and formula (43):

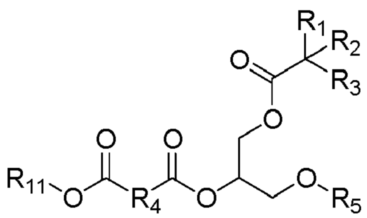 (23) 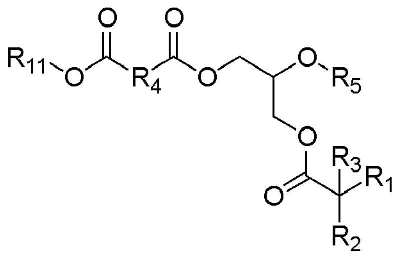 (24)

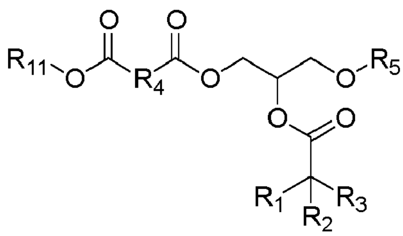 (43)

, wherein $R_1$, $R_2$, $R_3$ are as defined herein at any occurrence,
wherein $R_4$ is a divalent organic group comprising one or more, preferably one, (UV curable) carbon-carbon double bonds,
wherein $R_5$ is H or represented by formula (8):

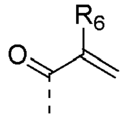 (8)

, preferably $R_5$ is H,

wherein $R_6$ is H or methyl,

wherein $R_{11}$ is independently selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or

$R_{11}$ is independently selected from formulae (25) to (30) and formulae (44) to (46):

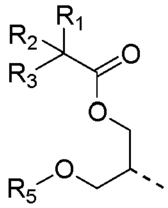 (25)

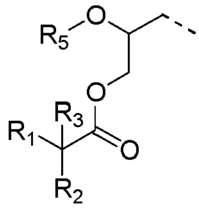 (26)

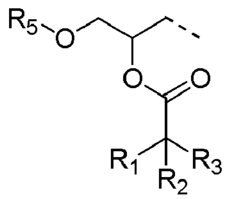 (44)

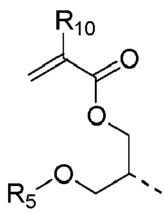 (27)

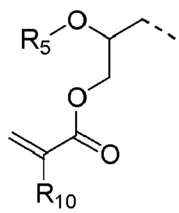 (28)

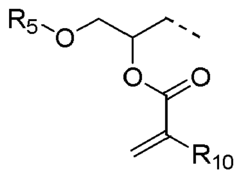 (45)

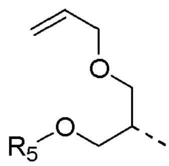 (29)

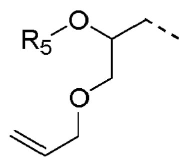 (30)

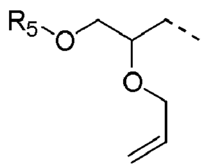 (46)

, wherein $R_{10}$ is H or methyl.

In one embodiment of the present invention the reactive diluent is independently selected from formula (23),

formula (24) and formula (43):

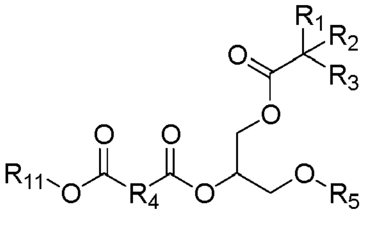 (23) 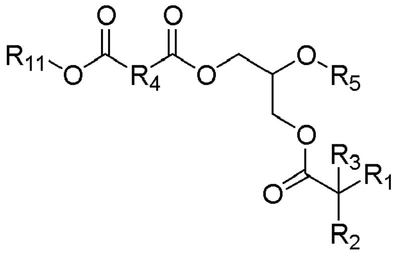 (24)

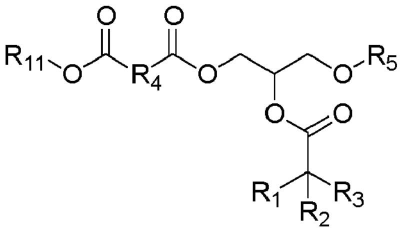 (43)

, wherein $R_1$, $R_2$, $R_3$ are as defined herein at any occurrence,
wherein $R_4$ is represented by formulae (3), (4) or (5):

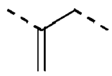 (3) 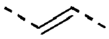 (4) 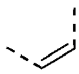 (5)

, wherein $R_5$ is H or represented by formula (8):

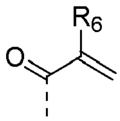 (8)

, wherein $R_6$ is H or methyl,
wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is independently selected from formulae (25) to (30) and formulae (44) to (46):

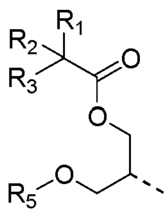 (25) 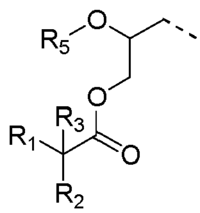 (26)

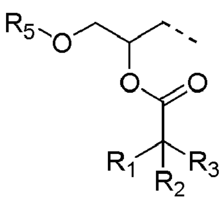 (44)

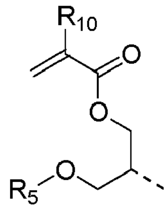
(27)

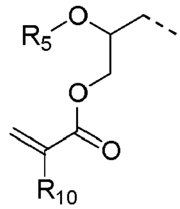
(28)

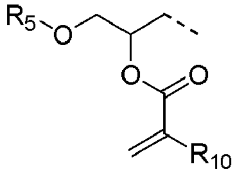
(45)

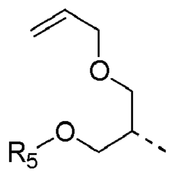
(29)

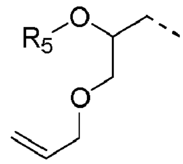
(30)

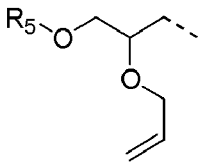
(46)

, wherein $R_{10}$ is H or methyl.

In one embodiment of the present invention the reactive diluent is independently selected from formulae (23), (24) and (43):

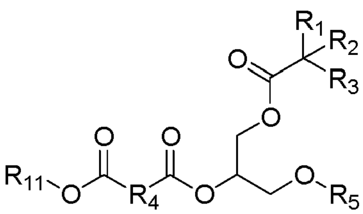
(23)

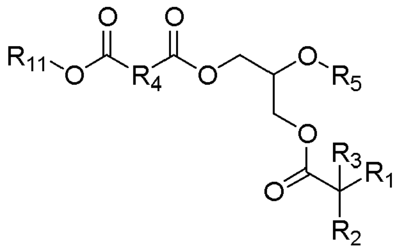
(24)

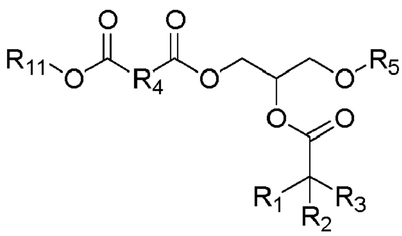
(43)

, wherein $R_1$, $R_2$, $R_3$ are as defined herein at any occurrence,
wherein $R_4$ is represented by formulae (3), (4) or (5):

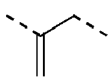
(3)

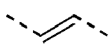
(4)

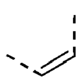
(5)

, wherein $R_5$ is H,

wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is independently selected from formulae (25), (26) and (44):

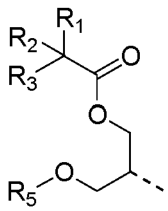 (25) 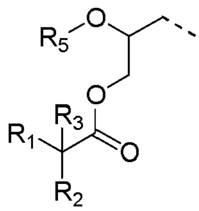 (26)

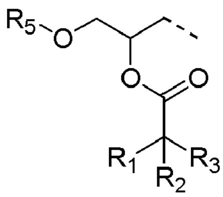 (44)

, wherein $R_1$, $R_2$, $R_3$ are as defined herein at any occurrence,
wherein $R_5$ is H.

In one embodiment of the present invention the reactive diluent is independently selected from formula (23), formula (24) and formula (43):

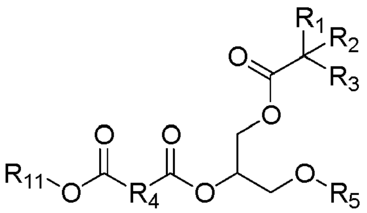 (23) 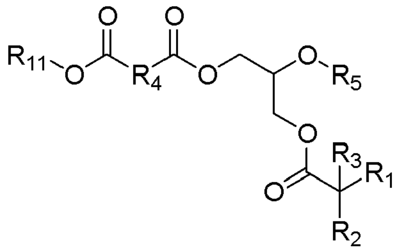 (24)

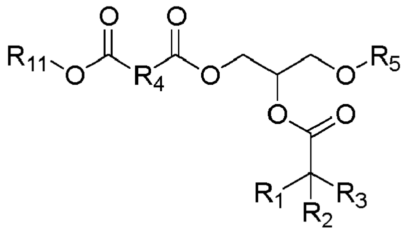 (43)

, wherein $R_1$, $R_2$, $R_3$ are as defined herein at any occurrence,
wherein $R_4$ is represented by formula (3):

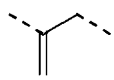 (3)

, wherein $R_5$ is H,
wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is independently selected from formulae (25), (26) and (44):

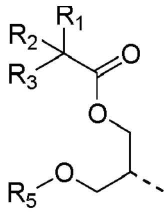 (25) 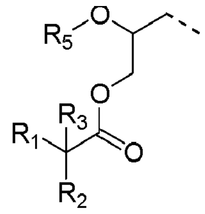 (26)

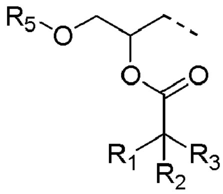 (44)

, wherein $R_1$, $R_2$, $R_3$ are as defined herein at any occurrence,
wherein $R_5$ is H.

In one embodiment of the present invention the reactive diluent is independently selected from formula (23), formula (24) and formula (43):

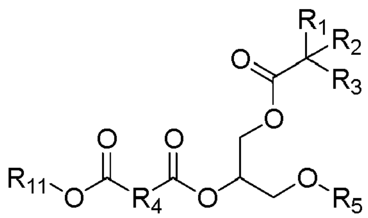 (23) 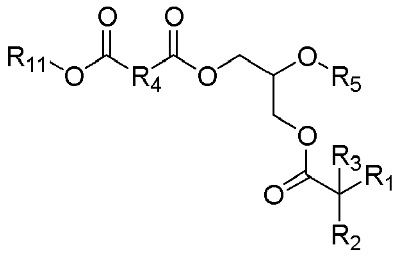 (24)

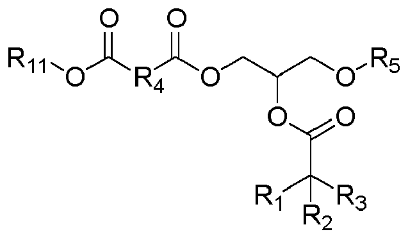 (43)

, wherein $R_1$, $R_2$, $R_3$ are as defined herein at any occurrence,
wherein $R_4$ is represented by formula (3):

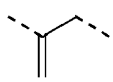 (3)

, wherein $R_5$ is H,
wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl.

In one embodiment of the present invention the reactive diluent is independently selected from formula (23), formula (24) and formula (43):

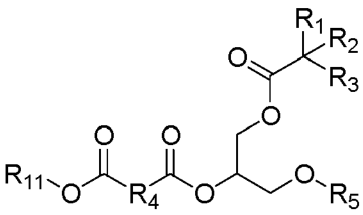
(23)

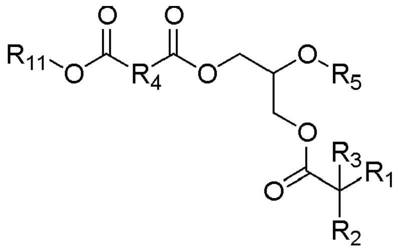
(24)

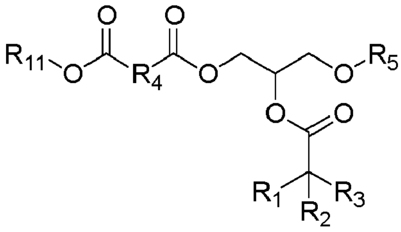
(43)

, wherein $R_1$, $R_2$, $R_3$ are as defined herein at any occurrence,
wherein $R_4$ is represented by formula (3):

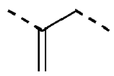
(3)

, wherein $R_5$ is H,
wherein $R_{11}$ is independently selected from formulae (25), (26) and (44):

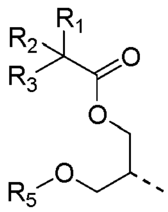
(25)

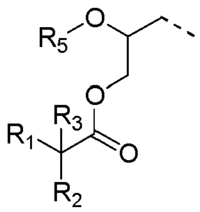
(26)

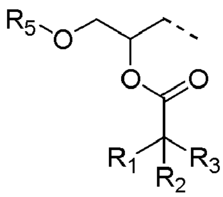
(44)

, wherein $R_1$, $R_2$, $R_3$ are as defined herein at any occurrence,
wherein $R_5$ is H.

In one embodiment of the present invention the resin formulation comprises further reactive diluents, wherein the further reactive diluents are selected from the group consisting of trimethylolpropane triacrylate (TMPTA), 1,6-hexanediol diacrylate (HDDA), and the ACE™ hydroxyl acrylate monomer (the adduct of acrylic acid and the glycidyl ester of neodecanoic acid); preferably the further reactive diluents comprise the ACE™ hydroxyl acrylate monomer (the adduct of acrylic acid and the glycidyl ester of neodecanoic acid).

It is assumed that the combination of the ACE™ hydroxyl acrylate monomer (the adduct of acrylic acid and the glycidyl ester of neodecanoic acid) with the oligomers of the invention, e.g. an itaconate-based oligomer, leads to favourable properties due to the structural similarity (i.e. the bulky and hydrophobic group in each component) and the resulting high compatibility of these components in mixtures.

The ACE™ hydroxyl acrylate monomer is the adduct of acrylic acid and the glycidyl ester of neodecanoic acid, i.e. the acrylate ester of the glycidyl ester of neodecanoic acid with a hydroxyl group on one of the side chains of the molecule, and has the following chemical structure:

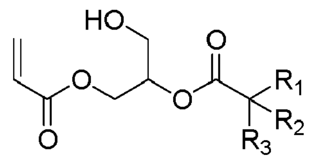

, wherein $R_1 = CH_3$ and $R_2$ and $R_3$ are H or linear or branched unsubstituted alkyl groups, wherein $R_2$ and $R_3$ comprise 7 carbon atoms.

In one embodiment of the present invention the resin formulation comprises a further reactive diluent, wherein the further reactive diluent is the ACE™ hydroxyl acrylate monomer (the adduct of acrylic acid and the glycidyl ester of neodecanoic acid).

In one embodiment of the present invention the reactive diluent is selected from formula (23), formula (24) and formula (43):

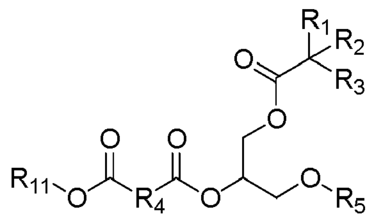
(23)
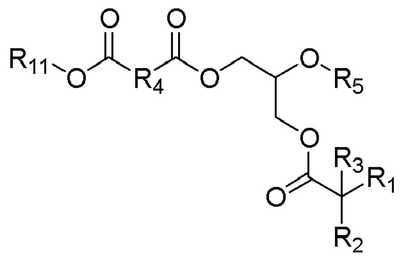
(24)

, wherein $R_4$ is represented by formula (3):

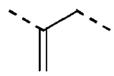
(3)

, $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is selected from formulae (25), (26) and (44):

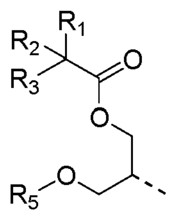
(25)
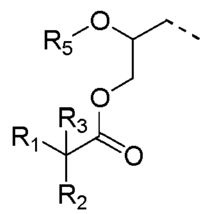
(26)

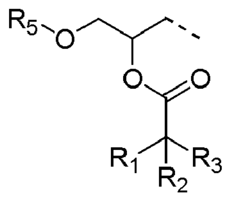
(44)

, wherein $R_1$, $R_2$, $R_3$ are as defined herein at any occurrence,
wherein $R_5$ is H.

In one embodiment of the present invention the reactive diluent is represented by formula (23):

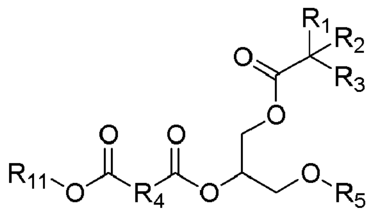

(23)

wherein $R_4$ is represented by formula (3):

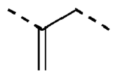

(3)

, $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ represented by formula (25):

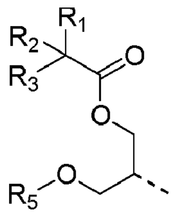

(25)

, wherein $R_1$, $R_2$, $R_3$ are as defined herein at any occurrence,
wherein $R_5$ is H.

In one embodiment of the present invention the reactive diluent is represented by formula (23):

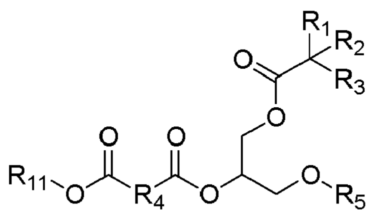

(23)

wherein $R_4$ is represented by formula (3):

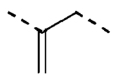

(3)

, $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is independently selected from formulae (25), (26) and (44):

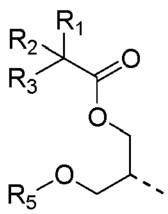

(25)

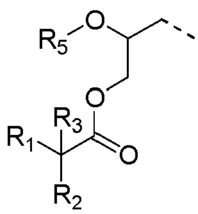

(26)

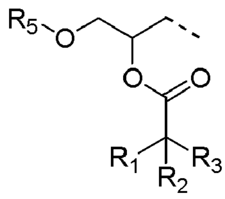

(44)

, wherein $R_1$, $R_2$, $R_3$ are as defined herein at any occurrence,
wherein $R_5$ is H,
preferably, $R_{11}$ is represented by formulae (44).

In one embodiment of the present invention the reactive diluent is selected from formula (23), formula (24) and formula (43):

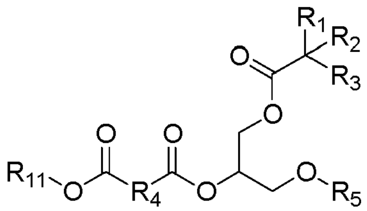

(23)

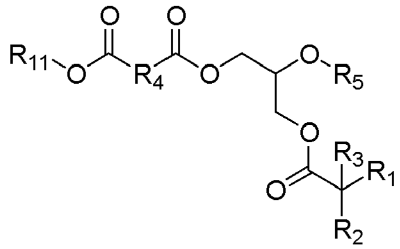

(24)

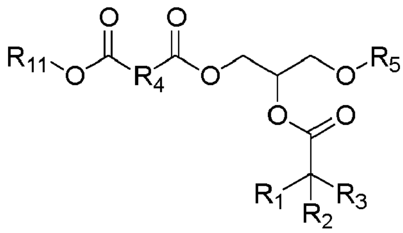

(43)

, preferably the reactive diluent is represented by formulae (43).
wherein $R_4$ is represented by formula (3):

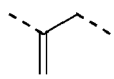

(3)

, $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is independently selected from formulae (25), (26) and (44):

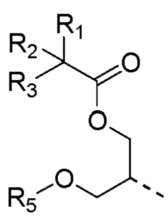

(25)

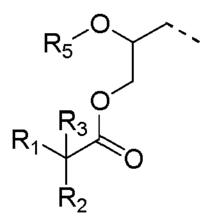

(26)

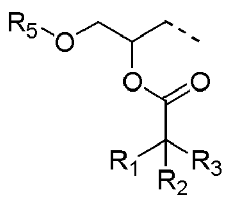

(44)

, wherein $R_1$, $R_2$, $R_3$ are as defined herein at any occurrence, wherein $R_5$ is H, preferably $R_{11}$ is represented by formulae (44).

In one embodiment of the present invention the reactive diluent is represented by formula (23):

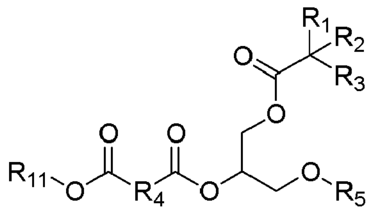

(23)

wherein $R_4$ is represented by formula (3):

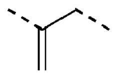

(3)

, $R_{11}$ is substituted or unsubstituted $C_{5-12}$ cycloalkyl, preferably cyclohexyl, or $R_{11}$ represented by formula (25):

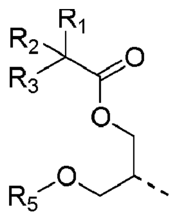

(25)

, wherein $R_1$, $R_2$, $R_3$ are as defined herein at any occurrence, wherein $R_5$ is H.

In one embodiment of the present invention the reactive diluent is represented by formula (23):

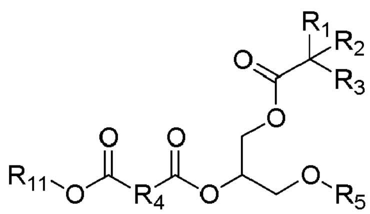

(23)

wherein $R_4$ is represented by formula (3):

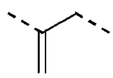

(3)

, $R_{11}$ is substituted or unsubstituted $C_{5-12}$ cycloalkyl, preferably cyclohexyl, wherein $R_1$, $R_2$, $R_3$ are as defined herein at any occurrence, wherein $R_5$ is H.

In one embodiment of the present invention the reactive diluent is selected from formula (23), formula (24) and formula (43):

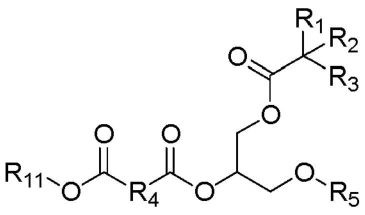 (23) 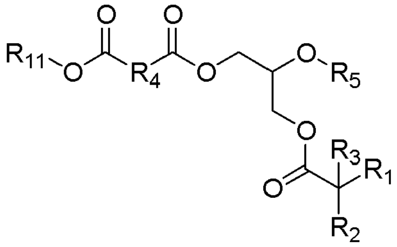 (24)

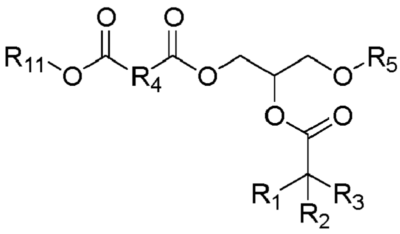 (43)

, preferably the reactive diluent is represented by formulae (43).
wherein $R_4$ is represented by formula (3):

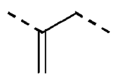 (3)

, $R_{11}$ is substituted or unsubstituted $C_{5-12}$ cycloalkyl, preferably cyclohexyl,
wherein $R_1$, $R_2$, $R_3$ are as defined herein at any occurrence,
wherein $R_5$ is H.

In one embodiment of the present invention the reactive diluent is represented by formula (23):

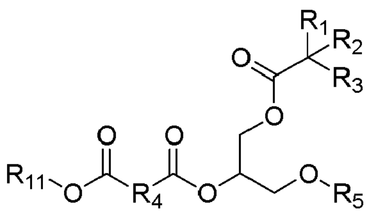 (23)

wherein $R_4$ is represented by formula (3):

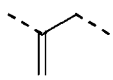 (3)

, $R_{11}$ represented by formula (25):

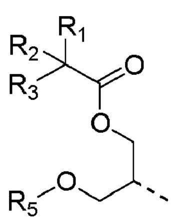 (25)

, wherein $R_1$, $R_2$, $R_3$ are as defined herein at any occurrence,
wherein $R_5$ is H.

The present invention also refers to a use of the resin formulations as defined herein in UV-curing processes.

UV (Ultra-Violet) curing involves the polymerization and crosslinking of functional monomers and oligomers (usually liquid) into a crosslinked polymer network (usually solid film) induced by UV lights (photons). The curing is fast and may occur in a fraction of a second. Curing proceeds by either free radical or cationic mechanism. Reactive monomers, oligomers and photoinitiators are the three major chemical components in a typical UV curing formulation. Those systems can also be combined with thermal/heat cure approaches to leverage oxygen inhibition; stress development and heterogenous network in a dual-cure approach.

The present invention also refers to a preparation of a resin formulation by combining at least (a) and (b), and optionally (c), as defined herein.

An oligomer (a) as defined herein is selected based on final application requirements, and mixed with one or more low viscous reactive diluents/monomers (b) to reach application viscosity and fine-tune the properties of the oligomer like flexibility, adhesion, flow, cure speed, etc. A photoinitiator package (c) is added to the mixture based on the light source and the interference that might be generated by the fully formulated system. In addition, optional additives (d) may be added to the mixture to modify gloss, wetting properties, strength, rheological properties, etc.

The present invention also refers to a coated article comprising a coating obtained by applying a resin formulation onto an article and drying (and applying UV radiation) said resin formulation, wherein said resin formulation is as defined herein.

The present invention also refers to an oligomer comprising, preferably consisting essentially of, more preferably consisting of, 3 to 20, preferably 3 to 10, more preferably 3 to 5, most preferably 3, structural units independently selected from formula (1), formula (2) and formula (35):

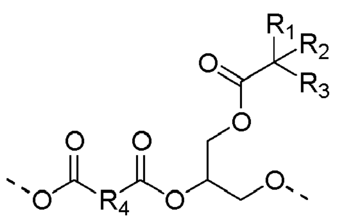
(1)

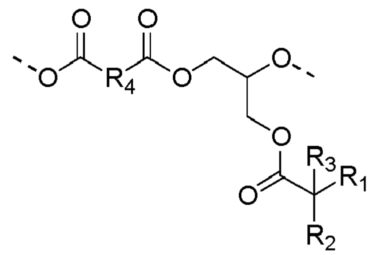
(2)

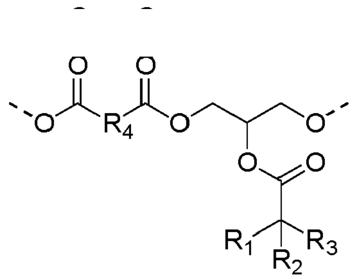
(35)

, wherein the structural units of the oligomer can be the same or can be different,

, wherein the dashed lines represent the linking in the oligomer,

wherein $R_1$, $R_2$, $R_3$ are as defined herein at any occurrence;

wherein $R_4$ is a divalent organic group comprising one or more, preferably one, (UV curable) carbon-carbon double bonds.

In one embodiment the molecular weight of the oligomer is more than 1000 g/mol and below 5000 g/mol, or the molecular weight of the oligomer is more than 1000 g/mol and below 3000 g/mol.

In one embodiment all except one structural unit comprise a (UV curable) carbon-carbon double-bond.

In one embodiment of the present invention the oligomer comprises, preferably consists essentially of, more preferably consists of repetition units or (co-)monomers based on itaconic acid/anhydride and groups one or two groups according to formula (1').

Preferably, the oligomer represents the (uncured) resin of the resin formulation.

In one embodiment of the present invention the oligomer comprises/has OH groups.

Oligomers that comprise/have OH groups can further be incorporated into the network (the cured resin) by reacting with isocyanate or melamine, for example. This makes it possible to provide "dual-curing/curable" resins, which is an extension of the application field.

In one embodiment of the present invention the oligomer comprises, preferably consists essentially of, more preferably consists of, 3 to 20, structural units independently selected from formula (1), formula (2) and formula (35):

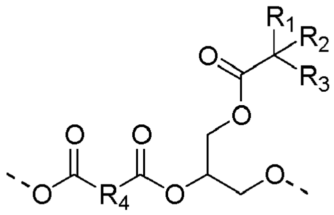
(1)
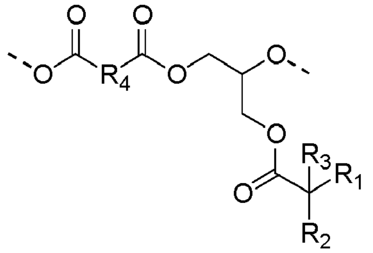
(2)

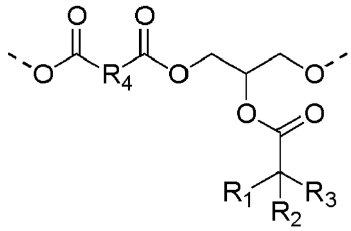
(35)

, preferably the structural units are represented by formula (1),
wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12,
wherein $R_3$ is methyl,
wherein $R_4$ is a divalent organic group comprising one or more, preferably one, (UV curable) carbon-carbon double bonds.

In one embodiment of the present invention the oligomer comprises, preferably consists essentially of, more preferably consists of, 3 to 20, structural units independently selected from formula (1), formula (2) and formula (35):

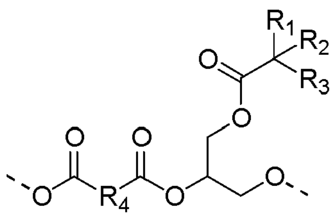
(1)
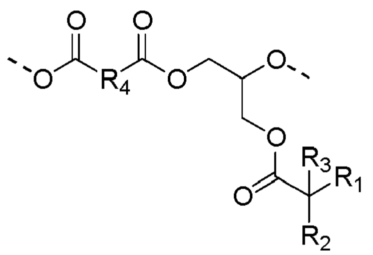
(2)

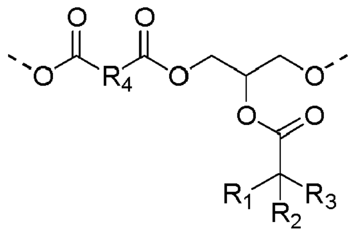
(35)

, preferably the structural units are represented by formula (2),
wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12,
wherein $R_3$ is methyl,
wherein $R_4$ is a divalent organic group comprising one or more, preferably one, (UV curable) carbon-carbon double bonds.

In one embodiment of the present invention the oligomer comprises, preferably consists essentially of, more

preferably consists of, 3 to 20, structural units independently selected from formula (1), formula (2) and formula (35):

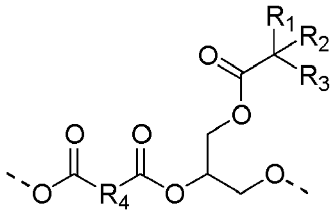
(1)
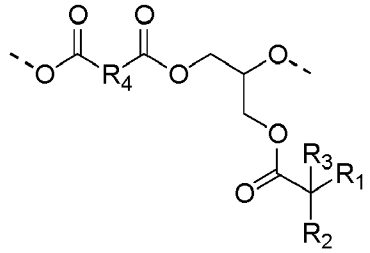
(2)

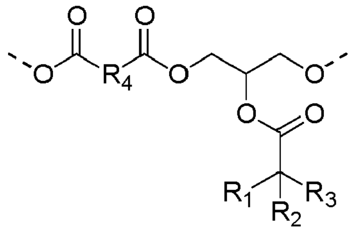
(35)

, preferably the structural units are represented by formula (35),
wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12, preferably from 3 to 12, further preferred from 4 to 12,
wherein $R_3$ is methyl,
wherein $R_4$ is a divalent organic group comprising one or more, preferably one, (UV curable) carbon-carbon double bonds.

In one embodiment of the present invention the total number of carbon atoms of $R_4$ is below 10, preferably below 7, more preferably below 5.

In one embodiment of the present invention $R_4$ can be selected from the group of formulae (3), (4) and (5):

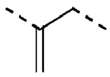
(3)
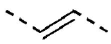
(4)
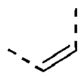
(5)

wherein the $R_4$-formulae of the structural units of the oligomer can be the same or can be different.

In one embodiment of the present invention $R_4$ is represented by formula (3):

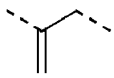
(3)
.

In one embodiment of the present invention the oligomer further comprises one or more moieties selected from formula (6), formula (7) and formula (36):

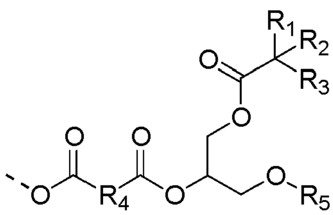
(6)
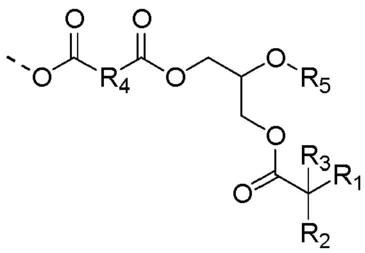
(7)

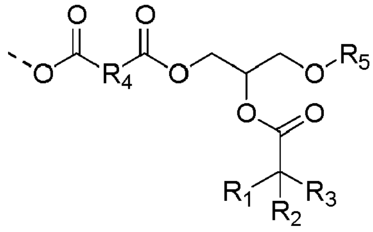 (36)

, wherein $R_1$, $R_2$, $R_3$ are as defined herein at any occurrence,

wherein $R_4$ is a divalent organic group comprising one or more, preferably one, (UV curable) carbon-carbon double bonds,

wherein $R_5$ is H or represented by formula (8):

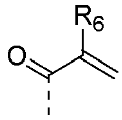 (8)

, wherein $R_6$ is H or methyl.

In one embodiment of the present invention the oligomer further comprises one or more moieties selected from formula (6), formula (7) and formula (36):

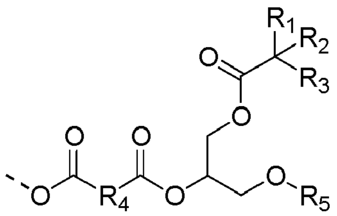 (6) 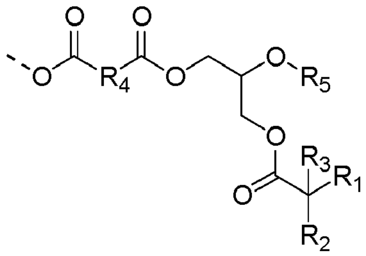 (7)

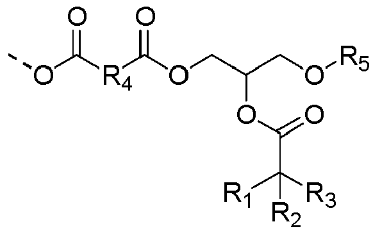 (36)

, wherein $R_1$, $R_2$, $R_3$ are as defined herein at any occurrence,

wherein $R_4$ is selected from formulae (3), (4) and (5) and the $R_4$-formulae can be the same or can be different:

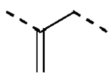 (3) 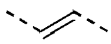 (4) 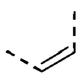 (5)

, wherein $R_5$ is H or represented by formula (8):

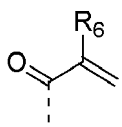 (8)

, wherein $R_6$ is H or methyl.

In one embodiment of the present invention the oligomer further comprises one or more moieties selected from

formula (6), formula (7) and formula (36):

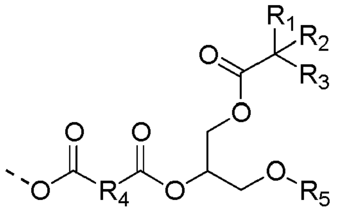 (6) 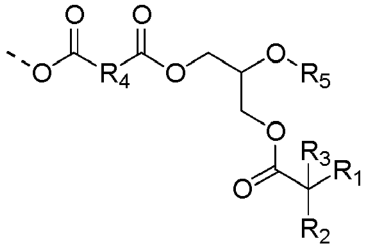 (7)

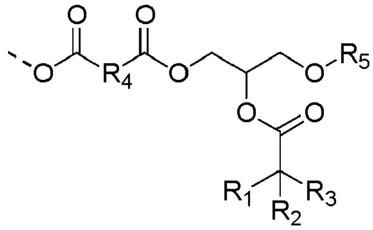 (36)

, wherein $R_1$, $R_2$, $R_3$ are as defined herein at any occurrence,
wherein $R_4$ is selected from formulae (3), (4) and (5) and the $R_4$-formulae can be the same or can be different:

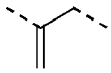 (3) 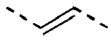 (4) 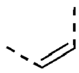 (5)

, wherein $R_5$ is H.

In one embodiment of the present invention the oligomer has a linear structure or a star-shaped structure.

In one embodiment of the present invention the oligomer has a linear structure.

In one embodiment of the present invention the oligomer has a star-shaped structure, preferably with 3 arms.

In one embodiment of the present invention the oligomer further comprises one or more moieties selected from formula (6), formula (7) and formula (36):

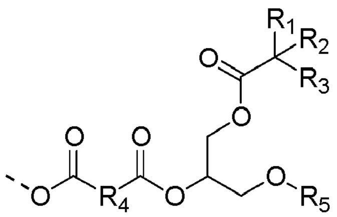 (6) 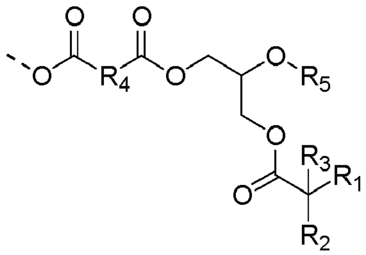 (7)

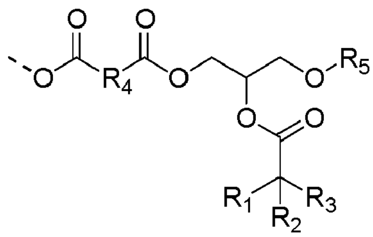 (36)

, wherein $R_1$, $R_2$, $R_3$ are as defined herein at any occurrence,
wherein $R_4$ is represented by formula (3):

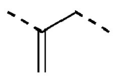 (3)

, wherein $R_5$ is H.

In one embodiment of the present invention the oligomer further comprises one or more moieties selected from formula (6), formula (7) and formula (36):

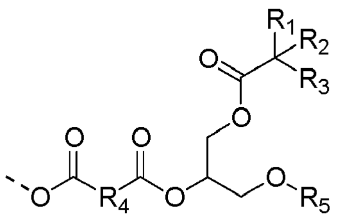
(6)

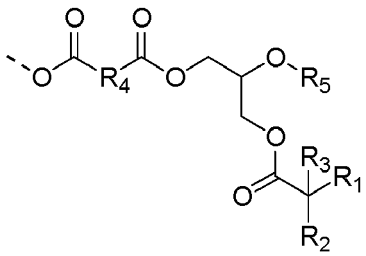
(7)

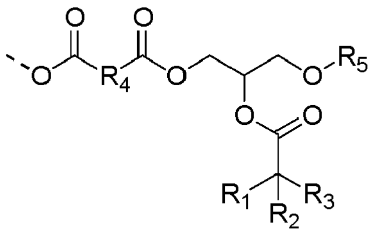
(36)

, wherein $R_1$, $R_2$, $R_3$ are as defined herein at any occurrence,
wherein $R_4$ is represented by formula (3):

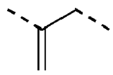
(3)

, wherein $R_5$ is H or represented by formula (8):

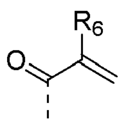
(8)

, wherein $R_6$ is H or methyl.

In one embodiment of the present invention the oligomer further comprises one or more moieties selected from formula (6), formula (7) and formula (36):

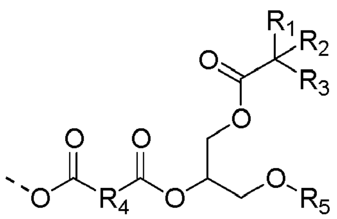
(6)

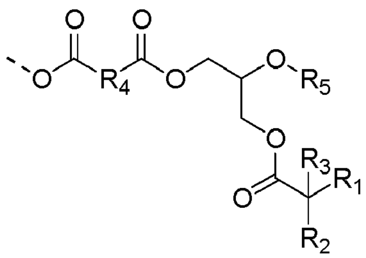
(7)

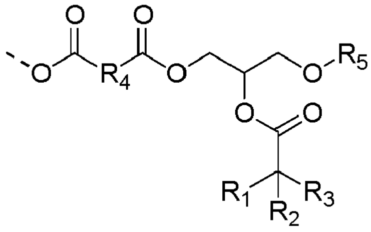 (36)

, wherein $R_1$, $R_2$, $R_3$ are as defined herein at any occurrence,

wherein $R_4$ is a divalent organic group comprising one or more, preferably one, (UV curable) carbon-carbon double bonds,

wherein $R_5$ is H.

In one embodiment of the present invention the oligomer further comprises one or more moieties selected from formula (6), formula (7) and formula (36):

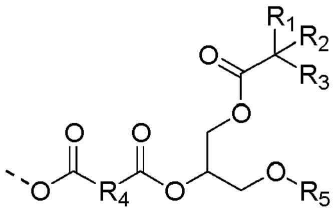 (6) 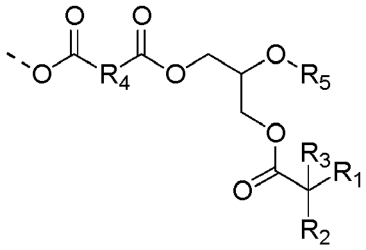 (7)

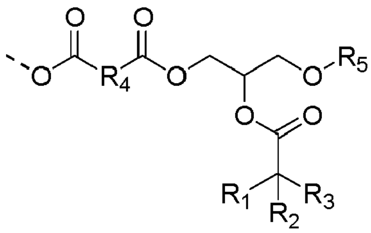 (36)

, wherein $R_1$, $R_2$, $R_3$ are as defined herein at any occurrence,

wherein $R_4$ is a divalent organic group comprising one or more, preferably one, (UV curable) carbon-carbon double bonds,

wherein $R_5$ is H or represented by formula (8):

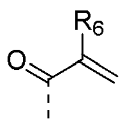 (8)

, wherein $R_6$ is H or methyl;

optionally the oligomer further comprises one or more structural units independently selected from formulae (9) to (12), formula (37) and formula (38):

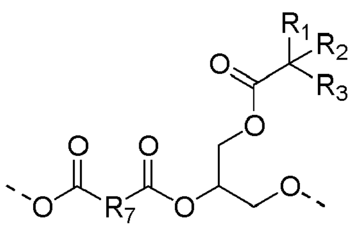 (9) 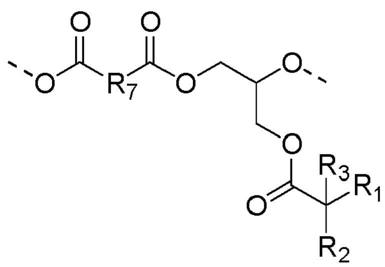 (10)

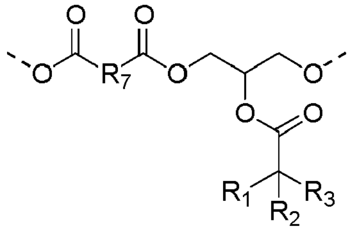 (37)

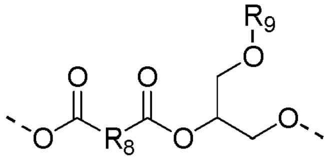 (11) 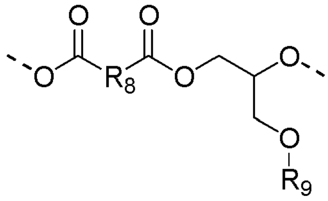 (12)

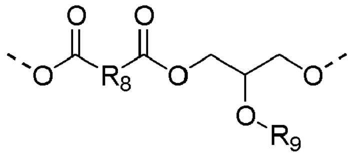 (38)

, wherein the structural units can be the same or different,
wherein $R_1$, $R_2$, $R_3$ are as defined herein at any occurrence,
wherein $R_7$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,
wherein $R_8$ is a divalent organic group comprising one or more UV curable carbon-carbon double bonds, or
$R_8$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,
wherein $R_9$ is selected from formula (13) and formula (14),

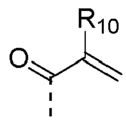 (13) 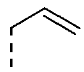 (14)

, wherein $R_{10}$ is H or methyl;
preferably optionally
the oligomer further comprises one or more moieties independently selected from formulae (15) to (18), formula (39) and formula (40):

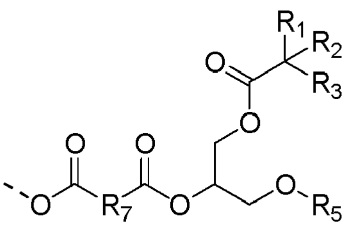 (15) 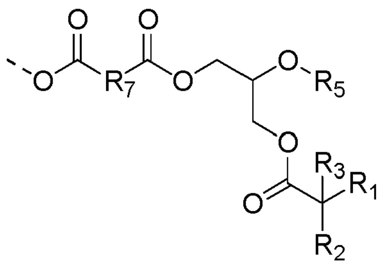 (16)

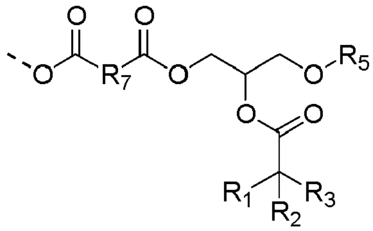 (39)

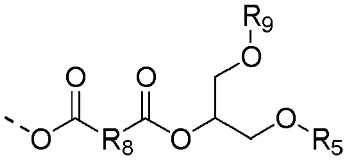 (17) 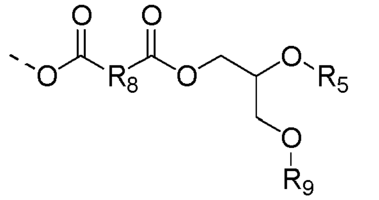 (18)

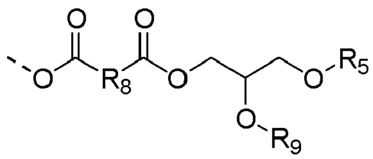 (40)

, wherein the moieties can be the same or different,
wherein $R_1$, $R_2$, $R_3$ are as defined herein at any occurrence,
wherein $R_5$ is as defined herein at any occurrence,
wherein $R_7$, $R_8$, $R_9$ are as defined herein at any occurrence.

In one embodiment of the present invention the oligomer further comprises one or more moieties selected from formula (6), formula (7) and formula (36):

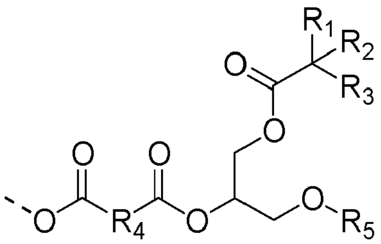 (6) 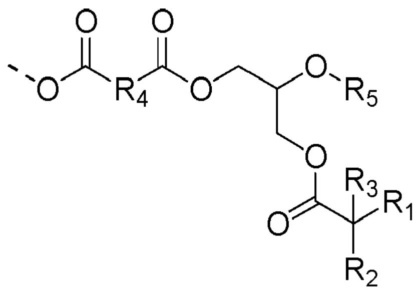 (7)

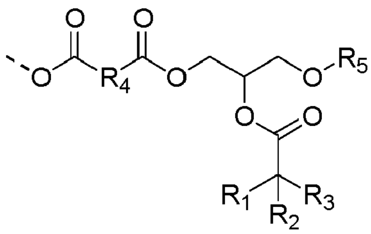 (36)

, wherein $R_1$, $R_2$, $R_3$ are as defined herein at any occurrence,
wherein $R_4$ is selected from formulae (3), (4) or (5) and can be the same or can be different:

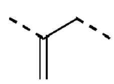 (3) 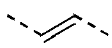 (4) 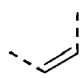 (5)

, wherein $R_5$ is H or represented by formula (8):

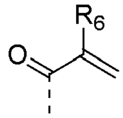 (8)

, wherein $R_6$ is H or methyl;
optionally the oligomer further comprises one or more structural units independently selected from formulae (9) to (12), formula (37) and formula (38):

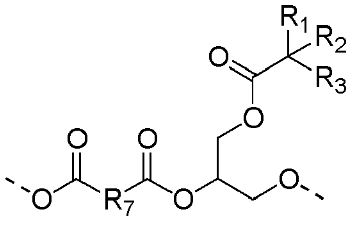 (9) 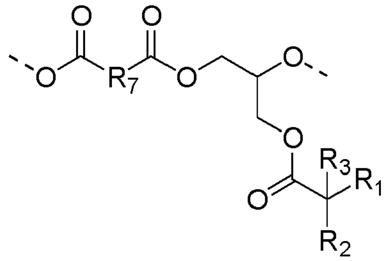 (10)

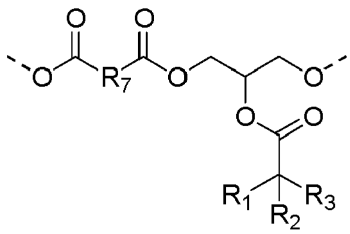 (37)

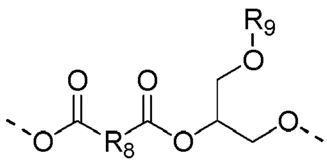 (11) 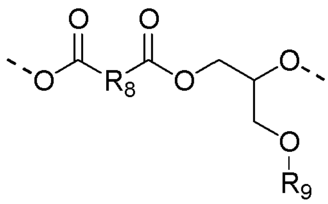 (12)

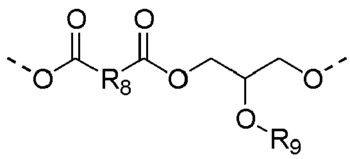 (38)

, wherein the structural units can be the same or different,
wherein $R_1$, $R_2$, $R_3$ are as defined herein at any occurrence,
wherein $R_7$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,
wherein $R_8$ is selected from formulae (3), (4) and (5):

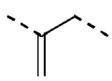 (3) 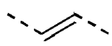 (4) 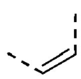 (5)

, or $R_8$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,
wherein $R_9$ is selected from formula (13) and formula (14),

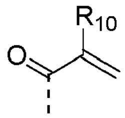 (13) 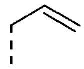 (14)

, wherein $R_{10}$ is H or methyl;
preferably optionally
the oligomer further comprises one or more moieties independently selected from formulae (15) to (18), formula (39) and formula (40):

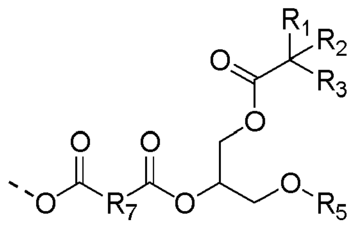 (15)

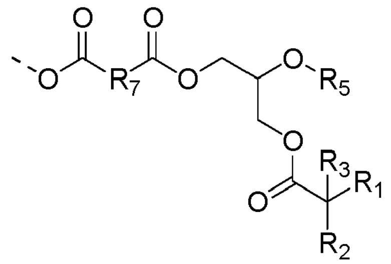 (16)

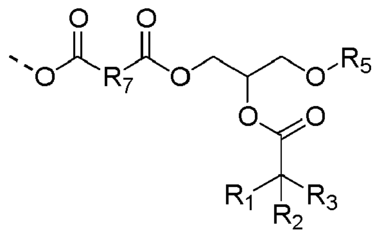 (39)

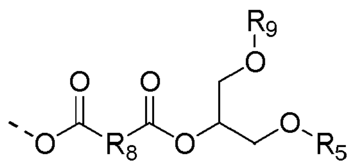 (17)

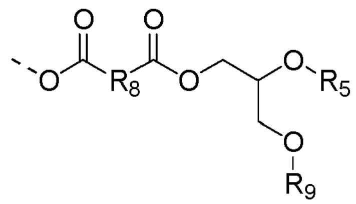 (18)

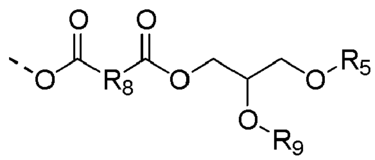 (40)

, wherein the moieties can be the same or different,
wherein $R_1$, $R_2$, $R_3$ are as defined herein at any occurrence,
wherein $R_5$ is as defined herein at any occurrence,
wherein $R_7$, $R_8$, $R_9$ are as defined herein at any occurrence.

In one embodiment of the present invention the oligomer is selected from formulae (33), (34), (47), (21), (22) and (42):

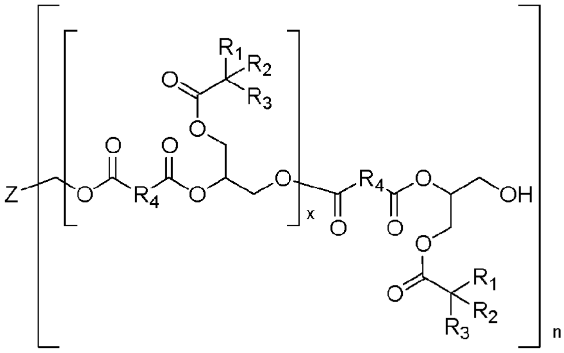

(33)

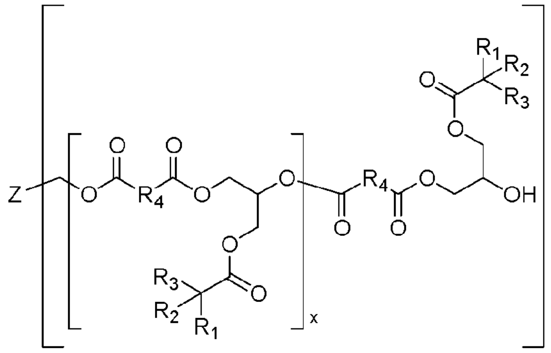

(34)

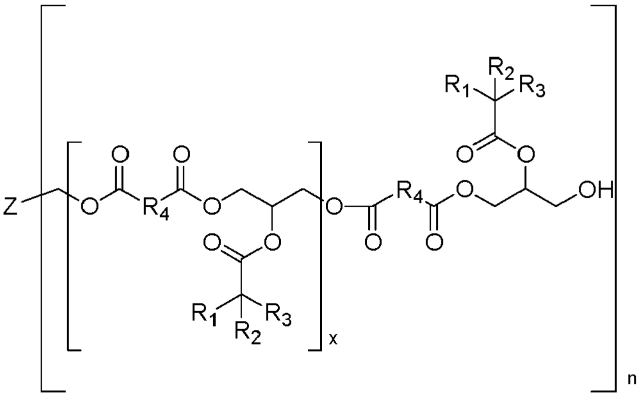

(47)

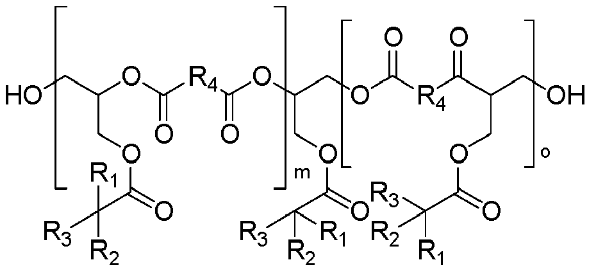

(21)

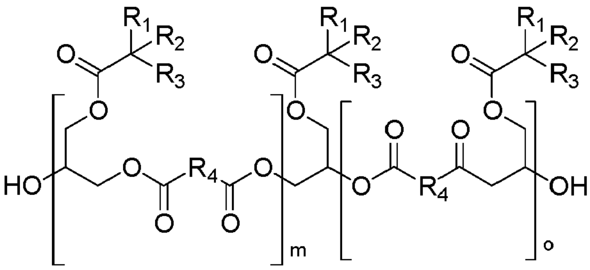

(22)

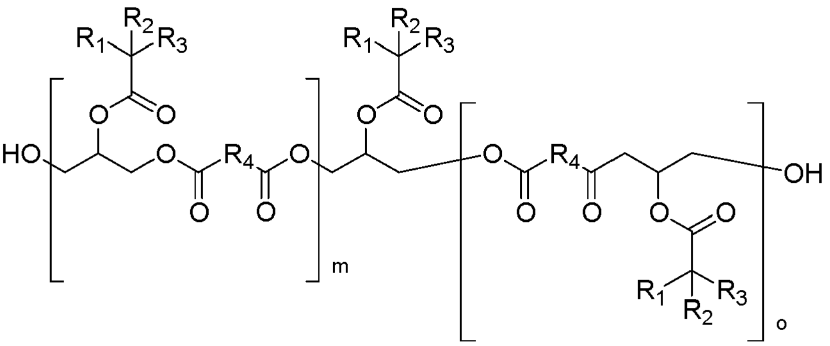

(42)

, wherein $R_1$, $R_2$, $R_3$ are as herein,
wherein $R_4$ is a divalent organic group comprising one or more UV curable carbon-carbon double bonds,
wherein Z represents an n-valent organic group,
wherein n is in the range of 3 to 6,
wherein x is in the range of 0 to 4,
wherein m + o is in the range of 3 to 11.

In one embodiment of the present invention m = 0 and o is in the range of 3 to 11 or m is in the range of 3 to 11 o = 0.

In one embodiment of the present invention Z is an n-valent organic group derived from a polyol comprising at least 3 hydroxyl groups;

wherein at least 3 of the at least 3 hydroxyl groups form the linking of Z to a structural unit of the oligomer;
wherein the polyol is selected from the group consisting of glycerol, trimethylolpropane, pentaerythritol and sugar alcohols with 4-6 hydroxyl groups, preferably the polyol is trimethylolpropane.

In one embodiment of the present invention n is in the range of 3 to 5, preferably n is in the range of 3 to 4, more preferably n = 3. In one embodiment of the present invention x is in the range of 0 to 2, preferably x is in the range of 0 to 1, more preferably x = 0.

In one embodiment of the present invention

m is in the range of 2 to 6 and o is in the range of 1 to 5,
more preferably m is in the range of 2 to 4 and o is in the range of 1 to 3,
most preferably m = 2 and o = 1.

In one embodiment of the present invention the oligomer is selected from formulae (19) to (22), formula (41) and formula (42):

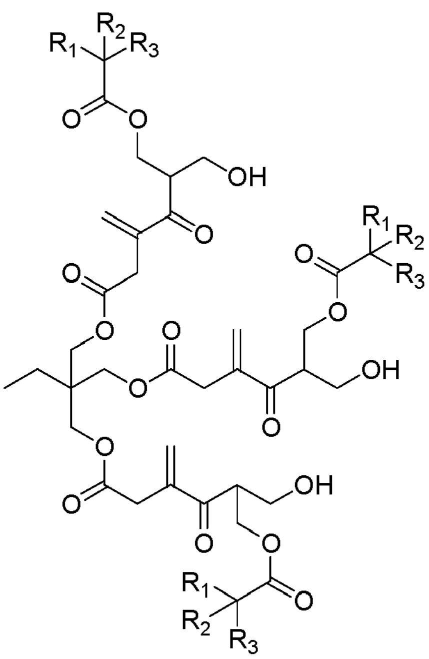

(19)

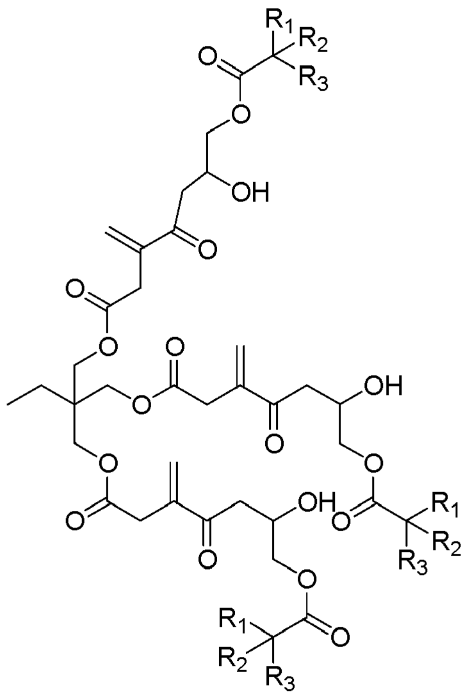

(20)

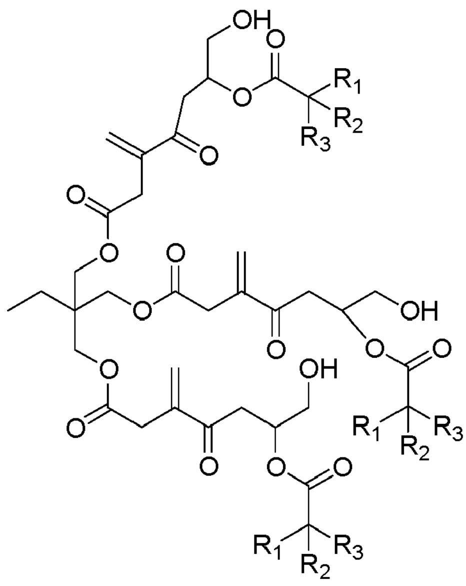

(41)

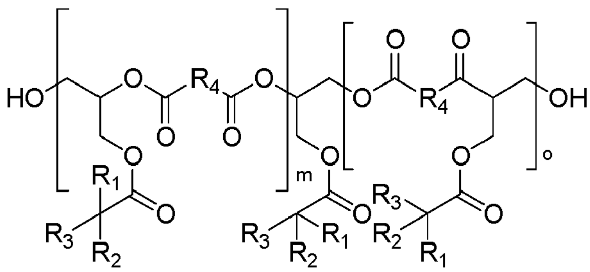

(21)

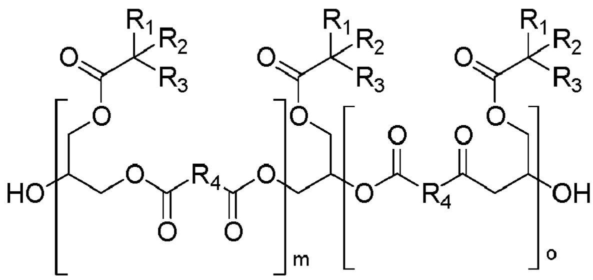

(22)

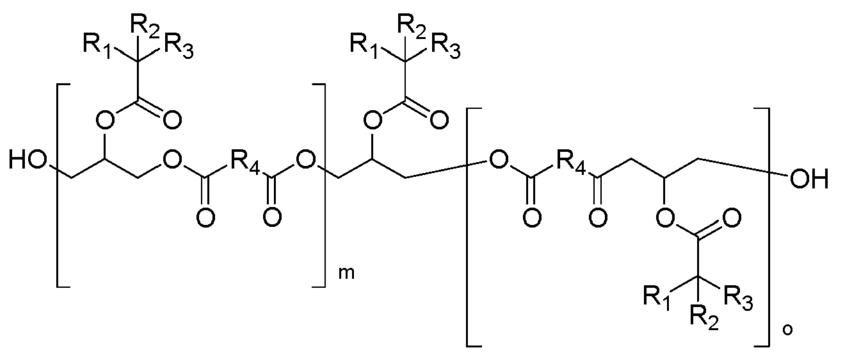

(42)

, wherein $R_1$, $R_2$, $R_3$ are as defined herein at any occurrence,
wherein $R_4$ is a divalent organic group comprising one or more (UV curable) carbon-carbon double bonds,
wherein n is 3,
wherein m + o is in the range of 3 to 11.

Formulae (19) to (22), (33), (34), (41), (42), and (47) represent the preferred chemical structures of the oligomers,

i.e. linear polyesters ((21), (22), (42)) or star-shaped polyesters ((19), (20), (41), (33), (34), (47)). These chemical structures/architectures lead to favourable viscosities for the resulting resin formulation and allow for improved processing of the same.

The structures of formulae (19) to (22), (33), (34), (41), (42), and (47) correspond to oligomers formed either via direct opening of an epoxy ring in the synthesis of a glycidyl ester with a carboxylic acid group or via ortho-ester mediation, respectively, i.e. these represent the situation in which only one of these 3 reaction mechanisms have taken place and the others have been suppressed. Under certain reaction conditions, it can be assumed that only one of these 3 reaction mechanisms will actually take place and that the formulae (19) to (22), (33), (34), (41), (42), (47) are created accordingly. Under different reaction conditions, however, it can also be assumed that all three reaction mechanisms take place simultaneously (with certain preferences) and that combinations of the structural units of formulae (19) to (22), (33), (34), (41), (42), and (47) in one co-oligomer and/or mixtures of oligomers of formulae (19) to (22), (33), (34), (41), (42), or (47) are present accordingly. The following scheme shows a structural section of a (co-)oligomer that has a combination of all 3 possible structural units/(co-)monomers, wherein p, q and r represent the molar fraction of said structural units/(co-) monomers in the (co-)oligomer:

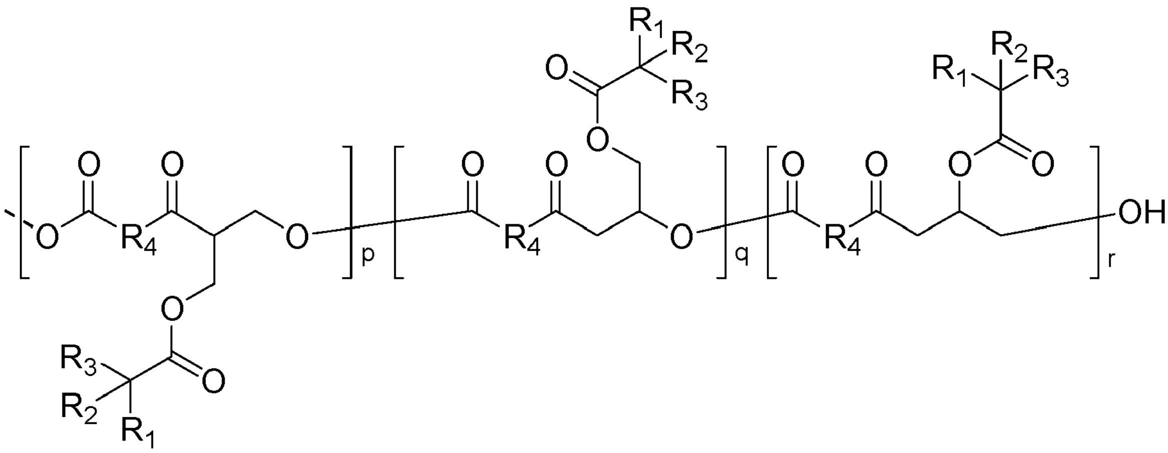

In one embodiment of the present invention $R_4$ is selected from formulae (3), (4) and (5) and can be same or different:

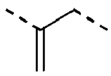 (3) 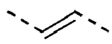 (4) 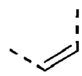 (5) .

In one embodiment of the present invention the oligomer is selected from formulae (19), (20), (41),

wherein $R_1$, $R_2$, $R_3$ are as defined herein at any occurrence,
wherein $R_4$ is selected from formulae (3), (4) and (5) and can be same or different:

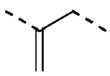 (3) 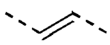 (4) 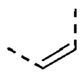 (5)

, preferably $R_4$ is represented by formula (3).

In one embodiment of the present invention the oligomer is selected from formulae (21) to (22) and (42),

wherein $R_1$, $R_2$, $R_3$ are as defined herein at any occurrence,
wherein $R_4$ is selected from formulae (3), (4) and (5) and can be same or different:

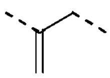 (3) 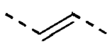 (4) 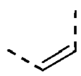 (5)

, preferably $R_4$ is represented by formula (3),
m is in the range of 2 to 6 and o is in the range of 1 to 5,

more preferably m is in the range of 2 to 4 and o is in the range of 1 to 3,
most preferably m = 2 and o = 1.

The present invention also refers to a resin formulation comprising the oligomer as defined herein and optionally a reactive diluent, preferably a reactive diluent as defined herein.

In one embodiment of the present invention the resin formulation comprises further reactive diluents, wherein the further reactive diluents are, for example, selected from the group consisting of trimethylolpropane triacrylate (TMPTA), 1,6-hexanediol diacrylate (HDDA), and the ACE™ hydroxyl acrylate monomer (the adduct of acrylic acid and the glycidyl ester of neodecanoic acid); preferably the further reactive diluents comprise the ACE™ hydroxyl acrylate monomer (the adduct of acrylic acid and the glycidyl ester of neodecanoic acid).

It is assumed that the combination of the ACE™ hydroxyl acrylate monomer (the adduct of acrylic acid and the glycidyl ester of neodecanoic acid) with the oligomers of the invention, e.g. an itaconate-based oligomer, leads to favourable properties due to the structural similarity (i.e. the bulky and hydrophobic group in each component) and the resulting high compatibility of these components in mixtures.

In one embodiment of the present invention the resin formulation comprises a reactive diluent, wherein the reactive diluent is the ACE™ hydroxyl acrylate monomer (the adduct of acrylic acid and the glycidyl ester of neodecanoic acid).

The present invention also refers to a reactive independently selected from formula (23), formula (24) and formula (43):

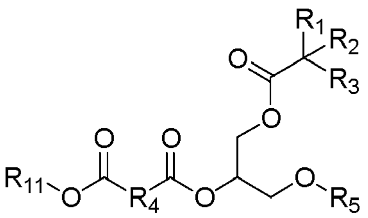
(23)

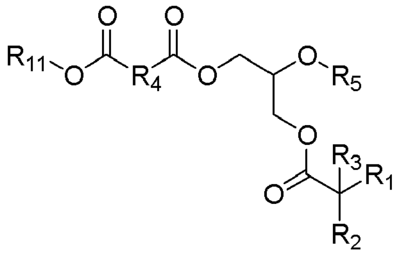
(24)

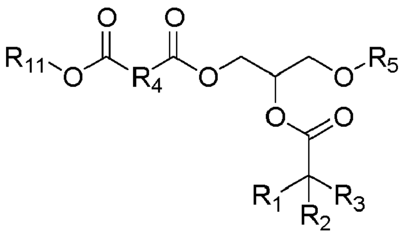
(43)

, $R_1$, $R_2$, $R_3$ are as defined herein at any occurrence,
wherein $R_4$ is a divalent organic group comprising one or more, preferably one, (UV curable) carbon-carbon double bonds,
wherein $R_5$ is H or represented by formula (8):

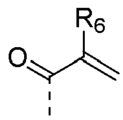
(8)

, preferably $R_5$ is H,
wherein $R_6$ is H or methyl,
wherein $R_{11}$ is independently selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is independently selected formulae (25) to (30) and formulae (44) to (46):

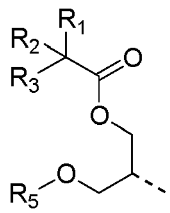
(25)
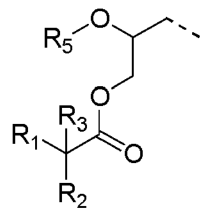
(26)

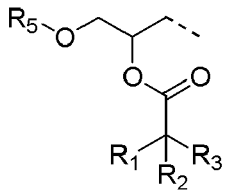
(44)

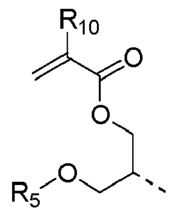
(27)
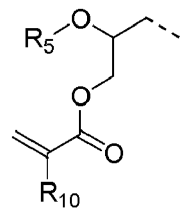
(28)

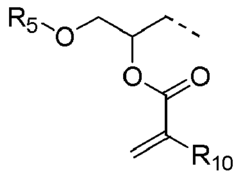
(45)

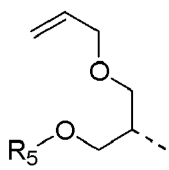
(29)
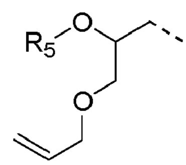
(30)

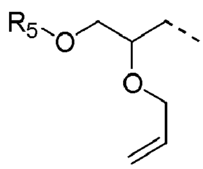
(46)

, wherein $R_{10}$ is H or methyl.

In one embodiment of the present invention the reactive diluent comprises/has OH groups, preferably due to the $R_5$ being H.

Reactive diluent that comprise/have OH groups can further be incorporated into the network (the cured resin) by reacting with isocyanate or melamine, for example. This makes it possible to provide "dual-curing/curable" reactive diluents, which is an extension of the application field.

In one embodiment of the present invention the reactive diluent independently selected from formula (23), formula (24) and formula (43):

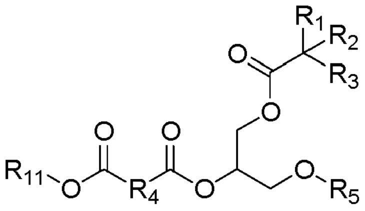 (23) 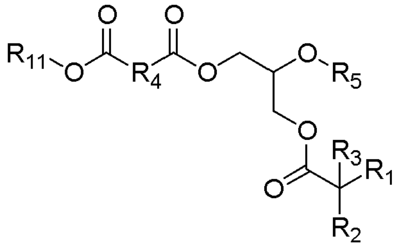 (24)

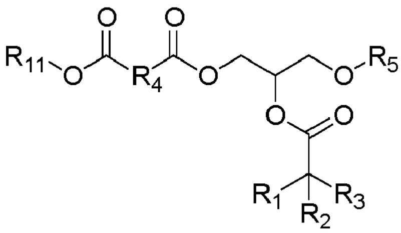 (43)

, $R_1$, $R_2$, $R_3$ are as defined herein at any occurrence,
wherein $R_4$ is selected from formulae (3), (4) and (5):

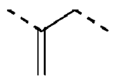 (3) 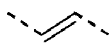 (4) 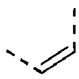 (5)

, preferably $R_4$ is represented by formula (3),
wherein $R_5$ is H or represented by formula (8):

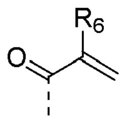 (8)

, preferably $R_5$ is H,
wherein $R_6$ is H or methyl,
wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is independently selected from formulae (25) to (30) and formulae (44) to (46):

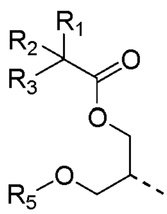 (25) 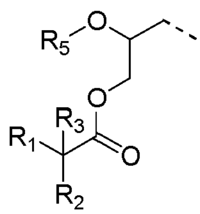 (26)

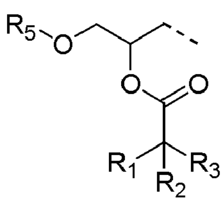 (44)

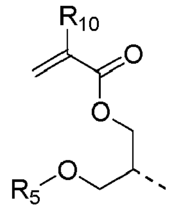 (27) 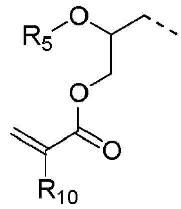 (28)

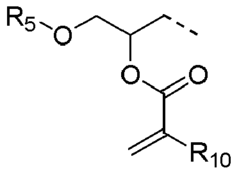 (45)

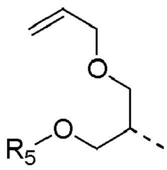 (29) 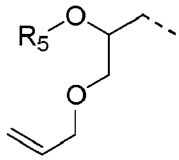 (30)

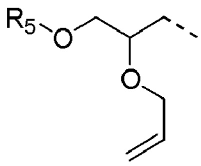 (46)

, wherein $R_{10}$ is H or methyl.

In one embodiment of the present invention the reactive diluent is independently selected from formula (23), formula (24) and formula (43):

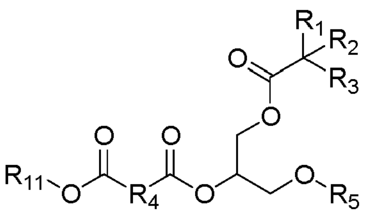 (23) 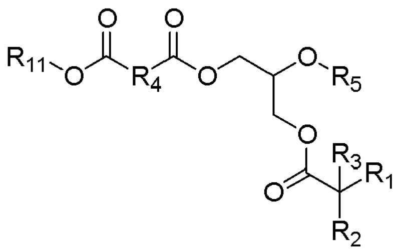 (24)

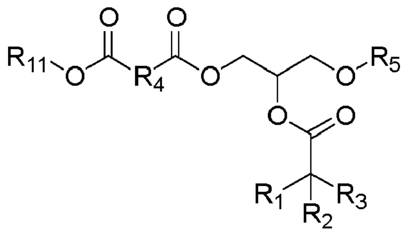 (43)

, wherein $R_1$, $R_2$, $R_3$ are as defined herein at any occurrence,
wherein $R_4$ is represented by formula (3):

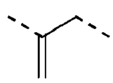 (3)

, wherein $R_5$ is H,

wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl, or
$R_{11}$ is selected from formulae (25), (26) and (44):

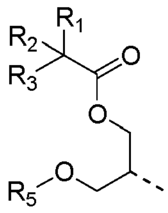

(25)

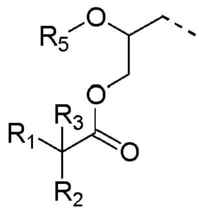

(26)

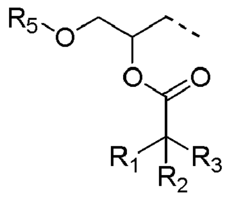

(44)

, wherein $R_1$, $R_2$, $R_3$ are as defined herein at any occurrence,
wherein $R_5$ is H.

In one embodiment of the present invention the reactive diluent is independently selected from formula (23), formula (24) and formula (43):

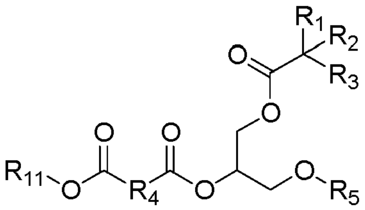

(23)

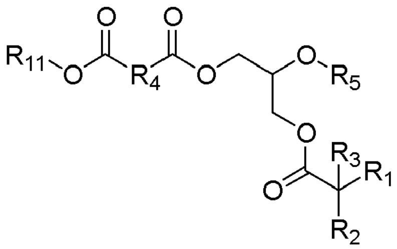

(24)

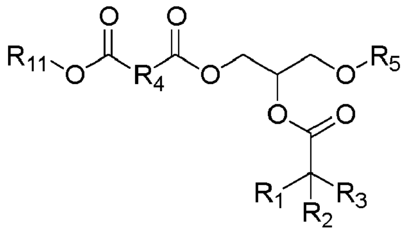

(43)

, wherein $R_1$, $R_2$, $R_3$ are as defined herein at any occurrence,
wherein $R_4$ is represented by formula (3):

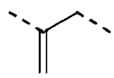

(3)

, wherein $R_5$ is H,
wherein $R_{11}$ is independently selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, preferably cyclohexyl.

In one embodiment of the present invention the reactive diluent is independently selected from formula (23), formula (24) and formula (43):

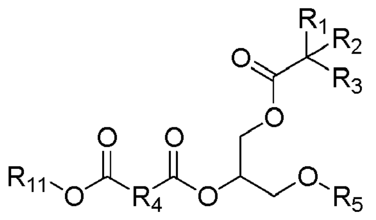 (23) 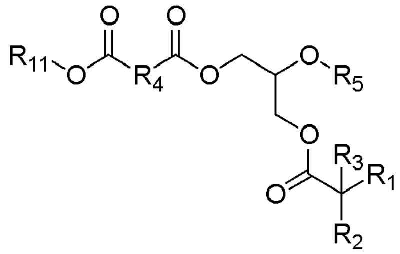 (24)

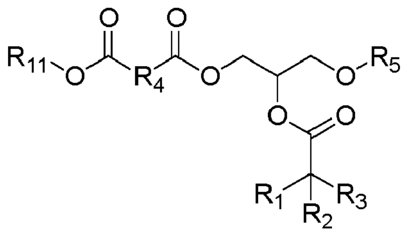 (43)

, wherein $R_1$, $R_2$, $R_3$ are as defined herein at any occurrence,
wherein $R_4$ is represented by formula (3):

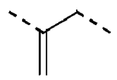 (3)

, wherein $R_5$ is H,
wherein $R_{11}$ is independently selected from formulae (25), (26) and (44):

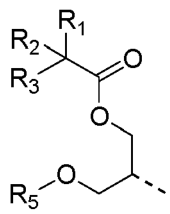 (25) 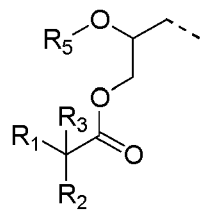 (26)

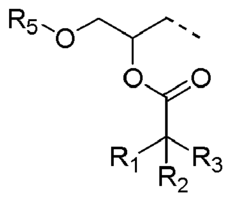 (44)

, wherein $R_1$, $R_2$, $R_3$ are as defined herein at any occurrence,
wherein $R_5$ is H.

The present invention also refers to a resin formulation comprising the reactive diluent as defined herein.

In one embodiment of the present invention the resin formulation further comprises a resin, preferably an oligomer, more preferably an oligomer as defined herein.

In one embodiment of the present invention the resin formulation comprises further reactive diluents, wherein the further reactive diluents are selected from the group consisting of trimethylolpropane triacrylate (TMPTA), 1,6-hexanediol diacrylate (HDDA), and the ACE™ hydroxyl acrylate monomer (the adduct of acrylic acid and the glycidyl ester of neodecanoic acid); preferably the further reactive diluents comprise the ACE™ hydroxyl acrylate monomer (the adduct of acrylic acid and the glycidyl ester of neodecanoic acid).

The term "resin" as used herein refers to organic components that can be oligomers or polymers of lower molecular weight (e.g. with degrees of polymerization below 50) comprising functional groups (such as OH groups or double bonds) that are accessible for crosslinking or curing reactions leading to oligomers/polymers of higher molecular weight and/or crosslinked oligomer/polymer networks. The oligomers or polymers of lower molecular weight can also be

called prepolymers in the art. The crosslinking or curing usually leads to toughening or hardening of the material and can be carried out, e.g., by UV light irradiation or by using curing agents or hardeners such as isocyanates, silanes or alkoxy melamines.

The term "resin formulation" as used herein refers to a composition comprising at least a resin and a diluent, preferably a reactive diluent, in which the resin is dispersed, suspended or at least partially dissolved in an aqueous or organic medium, wherein preferably which the resin is dispersed, suspended or at least partially dissolved in the diluent. Typically, the "resin formulation" refers to a pre-cured composition. The "resin formulation" may further comprise a curing agent or hardener such as isocyanates, silanes or alkoxymelamines, a photoinitiator, an inhibitor, and further additives such as pigments, stabilizers (to prevent gel storage), dyes, defoamers, adhesion promoters, flatting agents, wetting agents, slip aids, tackifiers, anti-oxydants, plasticizers, oxygen scavengers, flow agents, levelling agents, solvents, and catalysts.

The term "oligomer" as used herein will be understood to mean a molecule that encompasses a backbone (also referred to as "main chain") of three or more, such as 3 to 20, distinct types of structural/repeat/monomer units (the smallest constitutional unit of the molecule). A larger number of structural/repeat/monomer units (such as more than 20 structural/repeat/monomer) is referred to as a "polymer". The distinguishing criterion according to IUPAC is whether a small change in the number of structural/repeat/monomer units already causes a significant change in the properties. A(n) oligomer/polymer can be a "homo-oligomer/polymer" when having only one type of structural/repeat/monomer units or a "co-oligomer/polymer" when having two or more types of structural/repeat/monomer units. The term "oligomer/polymer" can refer to oligomers/polymers of diverse architectures such as linear, branched, grafted, cyclic, or crosslinked. Further, it will be understood that oligomers/polymers may include residues from initiators or other elements attendant to the synthesis of such an oligomer/polymer, where such residues are understood as not being covalently incorporated thereto but inseparably mixed. Further, such residues and other elements, while normally removed during post polymerization purification processes, are typically mixed or co-mingled with the oligomer/polymer such that they generally remain with the oligomer/polymer when it is transferred between vessels or between solvents or dispersion media. The oligomer determines the fundamental properties of the (cured) resin formulation, such as adhesion, chemical resistance, UV resistance or colour, depending on its functionality groups and their degree of functionality.

The term "structural units", "monomer unit", "monomeric unit", "repeat unit", "repetition unit", or "repeating unit" as used herein refers to the moieties of an oligomer/polymer that correspond to the monomers after they have been polymerized.

"Linking in the oligomer" means a covalent bonding of the structural units of the oligomer to an adjacent structural unit or to an end group or initiating group of the oligomer. The initiating group can be an end group of the oligomer, or in case of star-shaped oligomers or oligomers synthesized in a divergent oligomer/polymer synthesis strategy a core or central group of the oligomer/polymer that may be starting point in the oligomerization/polymerization process

It is noted that not all hydrogen atoms are shown in the formulas throughout the application. Furthermore, any atom in the general formulas can be substituted by any of its isotopes. That is, any hydrogen atom can be substituted by deuterium D.

The term "substituted" with respect to groups such alkyl groups as used herein refers to groups, wherein one or more hydrogen atoms are replaced by different atoms or groups such as halogens (e.g. Cl, Br, I, F), amines (primary, secondary, tertiary), sulphates, phosphates, ethers, esters, OH, SH, $CF_3$, CN, $C_{1-4}$ heteroalkyl, $C_{6-14}$ aryl and $C_{4-12}$ heteroaryl groups etc.

The term "unsubstituted" with respect to groups such alkyl groups as used herein refers to groups, wherein no hydrogen is replaced by a different atoms or groups such as halogens (e.g. Cl, Br, I, F), amines (primary, secondary, tertiary), sulphates, phosphates, ethers, esters, OH, SH, $CF_3$, CN, $C_{1-4}$ heteroalkyl, $C_{6-14}$ aryl and $C_{4-12}$ heteroaryl groups etc.

The term "linear $C_{1-11}$ alkyl" refers to a straight-chained saturated hydrocarbon group having 1 to 11 carbon atoms. Linear $C_{1-11}$ alkyl include, for example, methyl, ethyl, propyl, butyl, pentyl, hexyl, heptyl, octyl and nonyl.

The term "branched $C_{1-11}$ alkyl" refers to a branched-chained saturated hydrocarbon group having 1 to 11 carbon atoms. Branched $C_{1-11}$ alkyl include, for example, 1-methyl ethyl, 1-methyl propyl, 2-methyl propyl, 1-methyl butyl, 2-methyl butyl, 3-methyl butyl, 1 -methyl pentyl, 2-methyl pentyl, 3-methyl pentyl, 4-methyl pentyl, 1-methyl hexyl, 2-methyl hexyl, 3- methyl hexyl, 4-methyl hexyl, 5-methyl hexyl, 1-methyl heptyl, 2-methyl heptyl, 3-methyl heptyl, 4-methyl heptyl, 5-methyl heptyl, 6-methyl heptyl, 1-methyl octyl, 2-methyl octyl, 3- methyl octyl, 4-methyl octyl, 5-methyl octyl, 6-methyl octyl and 7-methyl octyl, 1-ethyl propyl, 1-ethyl butyl, 2-ethyl butyl, 1-ethyl pentyl, 2-ethyl pentyl, 3-ethyl pentyl, 1-ethyl hexyl, 2-ethyl hexyl, 3-ethyl hexyl, 4-ethyl hexyl, 1-ethyl heptyl, 2-ethyl heptyl, 3-ethyl heptyl, 4-ethyl heptyl and 5-ethyl heptyl, 1 -propyl butyl, 1-propyl pentyl, 2-propyl pentyl, 1-propyl hexyl, or 2-propyl hexyl and 3-propyl hexyl.

The term "aryl" refers to and includes both single-ring aromatic hydrocarbyl groups and polycyclic aromatic ring systems. The polycyclic rings may have two or more rings in which two carbons are common to two adjoining rings (the rings are "fused") wherein at least one of the rings is an aromatic hydrocarbyl group, e.g., the other rings can be cycloalkyls, cycloalkenyls, aryl, heterocycles, and/or heteroaryls. Preferred aryl groups are those containing six to thirty carbon atoms,

preferably six to twenty carbon atoms, more preferably six to twelve carbon atoms. Especially preferred is an aryl group having six carbons, ten carbons or twelve carbons. Suitable aryl groups include phenyl, biphenyl, triphenyl, triphenylene, tetraphenylene, naphthalene, anthracene, phenalene, phenanthrene, fluorene, pyrene, chrysene, perylene, and azulene, preferably phenyl, biphenyl, triphenyl, triphenylene, fluorene, and naphthalene. Additionally, the aryl group is optionally substituted.

The term "aromatic group" as used herein refers to groups having aromaticity, namely having a conjugated ring of unsaturated bonds or ion pairs of electrons and satisfying the Hückel's rule which states that an aromatic system should have 4n + 2 n-electrons, where n is an integer ≥ 0. Said aromatic group includes aryl and heteroaryl groups. The aromatic group may be substituted or unsubstituted. The aromatic group includes both single-ring aromatic hydrocarbyl groups and polycyclic aromatic ring systems. The polycyclic rings may have two or more rings in which two carbons are common to two adjoining rings (the rings are "fused") wherein at least one of the rings is an aromatic hydrocarbyl group, e.g., the other rings can be cycloalkyls, cycloalkenyls, aryl, heterocycles, and/or heteroaryls.

As used herein, the term "heteroalkyl" refers to an alkyl group as described herein in which one or more carbon atoms is replaced by a heteroatom. Suitable heteroatoms include oxygen, sulfur, nitrogen, phosphorus, and the like. Examples of heteroalkyl groups include, but are not limited to, alkoxy, amino, thioester, polyethylene glycol), and alkyl-substituted amino.

The term "heteroaryl" refers to and includes both single-ring aromatic groups and polycyclic aromatic ring systems that include at least one heteroatom. The heteroatoms include, but are not limited to O, S, N, P, B, Si, and Se. In many instances, O, S, or N are the preferred heteroatoms. Hetero-single ring aromatic systems are preferably single rings with 5 or 6 ring atoms, and the ring can have from one to six heteroatoms. The hetero-polycyclic ring systems can have two or more rings in which two atoms are common to two adjoining rings (the rings are "fused") wherein at least one of the rings is a heteroaryl, e.g., the other rings can be cycloalkyls, cycloalkenyls, aryl, heterocycles, and/or heteroaryls. The hetero-polycyclic aromatic ring systems can have from one to six heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably dibenzothiophene, dibenzofuran, dibenzoselenophene, carbazole, indolocarbazole, imidazole, pyridine, triazine, benzimidazole, 1,2-azaborine, 1,3-azaborine, 1,4-azaborine, borazine, and aza-analogs thereof. Additionally, the heteroaryl group is optionally substituted.

The term "UV curable carbon-carbon double bond" refers to a carbon-carbon double bond that is accessible in a curing process initiated by ultraviolet (UV) light irradiation to generate the crosslinked or cured resin (or oligomer/polymer network) by forming covalent bonds with chain extenders, crosslinkers and other carbon-carbon double bonds of the oligomer/polymer or the reactive diluent comprising a UV curable carbon-carbon double bond. Typically, the UV curing process involves a photoinitiator which creates radicals when exposed to radiation (UV or visible), which in turn start the curing, oligomerization/polymerization or crosslinking reactions.

The term "reactive diluent" refers to a compound which disperses, suspends or at least partially dissolves the resin, and further, lowers the viscosity and chemically reacts during curing.

The term "coated article" refers to an article comprising a substrate coated with one or more layers comprising a coating formed by the resin formulation as defined herein. Preferred articles may be structures or materials such as wood, plastics, vinyl flooring, metal, flexible packaging films, and electronic assemblies, wherein the coating formed by the resin formulation does not modify their intrinsic properties.

## Examples

1. Synthesis of the reactive diluents: maleate and itaconate (di)hydroxyesters

Examples 1 and 2 (using a solvent):

In a glass reactor equipped with an anchor stirrer, a reflux condenser and nitrogen inlet, the selected anhydride was reacted with cyclohexanol in an equimolar ratio in 5wt% of Butyl Acetate at a temperature of 80°C. The reaction was continued until conversion of the anhydride had reached at least 90 mol% by acid-base titration. The corresponding amount of ethanol was added to the reactor to push finalize the conversion of anhydride. Cardura™ E10P (CE10P) was then fed to the reactor in one step in an equimolar ratio to the initial anhydride whilst keeping the temperature at 80°C. The

system was then allowed to react further for 2 hours at 80°C. Epoxy Group Content (EGC) and Acid Value (AV) measurements were used to measure remaining unconverted acid, and the corresponding amount of Cardura™ E10P was added to obtain full conversion. The reactor was allowed to cool to room temperature, and the product was collected and characterized. See Table 1 below.

Structure of Example 1 ((possibly) a mixture of):

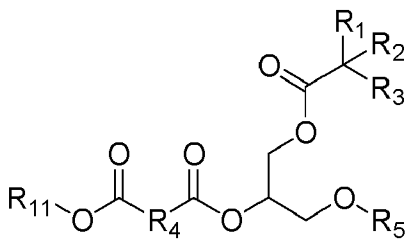

and

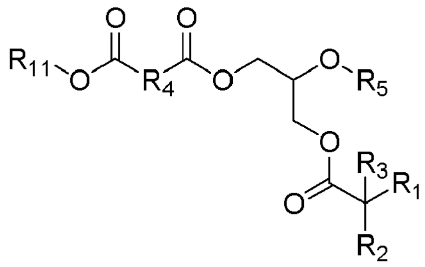

and

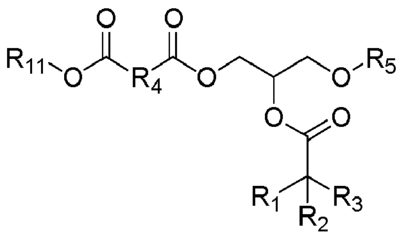

with $R_1 = CH_3$;
$R_2$ and $R_3$ are H or linear or branched unsubstituted alkyl groups, wherein $R_2$ and $R_3$ comprise 7 carbon atoms;
$R_4$ is

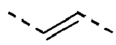

or

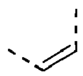

; $R_5$ is H;
$R_{11}$ is

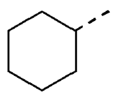

.

Structure of Example 2 ((possibly) a mixture of):

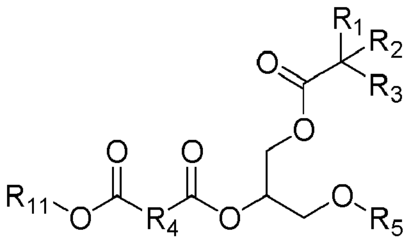 and 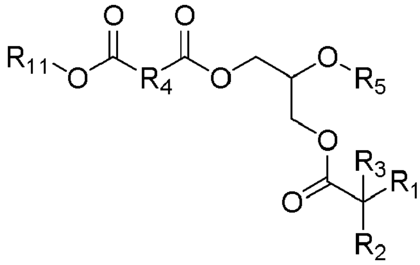

and

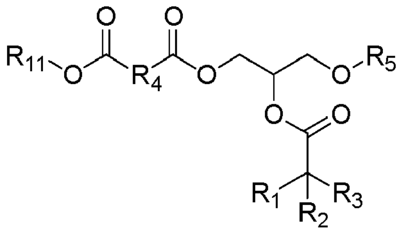

with $R_1 = CH_3$;
$R_2$ and $R_3$ are H or linear or branched unsubstituted alkyl groups, wherein $R_2$ and $R_3$ comprise 7 carbon atoms;
$R_4$ is

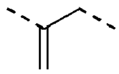

; $R_5$ is H;
$R_{11}$ is

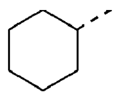

.

Example 3 (solvent-free):

In a glass reactor equipped with an anchor stirrer, a reflux condenser and nitrogen inlet, itaconic Anhydride was reacted with cyclohexanol in an equimolar ratio at a temperature of 80°C. The reaction was continued until conversion of the anhydride had reached at least 90 mol% monitored by acid-base titration. Cardura™ E10P was then added in an equimolar ratio by portions to control the exotherm. Temperature was raised up to 120°C and the reaction was continued. Epoxy Group Content (EGC) and Acid Value (AV) measurements were used to measure remaining acid, and the corresponding amount of Cardura™ E10P was added to complete the conversion. The reactor was allowed to cool to room temperature, and the product was collected and characterized. See Table 1 below.

Structure of Example 3 ((possibly) a mixture of):

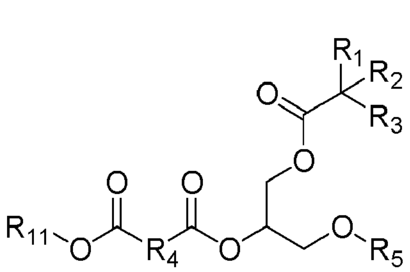 and 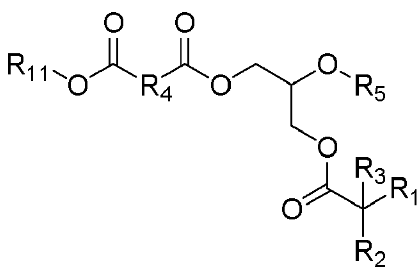

and

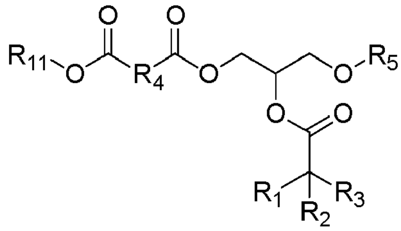

with $R_1$ and $R_2$ are H or linear or branched unsubstituted alkyl groups, wherein $R_1$ + $R_2$ comprise 7 carbon atoms;
$R_3$ = $CH_3$;
$R_4$ is

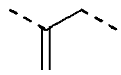

; $R_5$ is H;
$R_{11}$ is

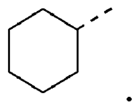

Example 4 (solvent-free):

In a glass reactor equipped with an anchor stirrer, a reflux condenser and nitrogen inlet, itaconic acid (IA) was dissolved in a minimum of Cardura™ E10P at a temperature of 120°C. Additional Cardura™ E10P was added during one hour in a molar ratio 1:2 IA:Cardura™ E10P to form a dihydroxy ester. EGC and AV measurements were used to measure the remaining acid, and the corresponding amount of Cardura™ E10P was added to complete the conversion. The reactor was allowed to cool to room temperature, and the product was collected and characterized. See Table 1 below.

Structure of Example 4 ((possibly) a mixture of):

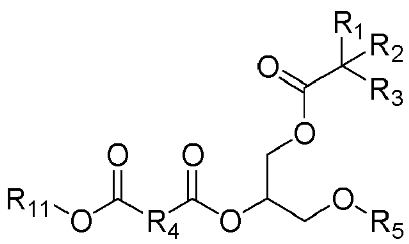

and

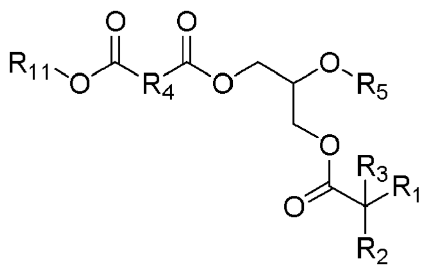

and

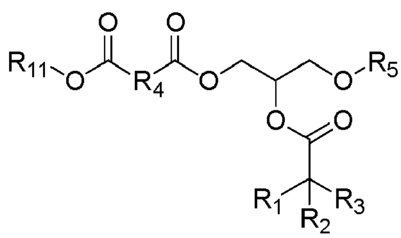

with $R_1$ and $R_2$ are H or linear or branched unsubstituted alkyl groups, wherein $R_1 + R_2$ comprise 7 carbon atoms; $R_3 = CH_3$; $R_4$ is

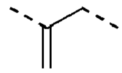

; $R_5$ is H; $R_{11}$ is

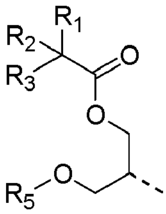
or
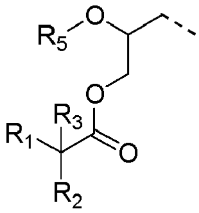
or
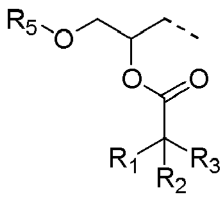
.

Table 1: Synthesis recipes of the diluents.

| Adduct reference | Maleate Hydroxy Ester (Ex.1) | | Itaconate Hydroxy Ester (Ex.2) | | Hydroxy Ester Itaconate (Ex.3) (HEI) | | Dihydroxy Ester Itaconate (Ex.4) (DHEI) | |
|---|---|---|---|---|---|---|---|---|
| Initial reactor charge | *Wt%* | *Intake in 500mL reactor* (g) | *Wt%* | *Intake in 500mL reactor (g)* | *Wt%* | *Intake in 500mL reactor (g)* | *Wt%* | *Intake in 500mL reactor (g)* |
| Cyclohexanol | 22,7 | 90,8 | 22,0 | 88,0 | 22 | 88 | - | - |
| Anhydride | 22,5 | 89,9 | 24,9 | 99,5 | 24,9 | 99,5 | - | - |
| Itaconic Acid | - | - | - | - | - | - | 21,4 | 75,2 |
| n-Butyl acetate | 5,0 | 20,0 | 5,0 | 20,0 | - | - | - | - |
| CE10P | - | - | - | - | - | - | 19,6 | 69,2 |
| CE10P addition | | | | | | | | |
| CE10P | 54,8 | 219,3 | 53,1 | 212,6 | 53,1 | 212,5 | 59,0 | 207,6 |
| Viscosity (spindle #4, 100 rpm - mPa.s) | 630 | | 4 012 | | 5 968 | | 19 290 | |
| Color (Pt/Co) | 60 | | 191 | | 223 | | 43 | |
| Mw (g/mol) by integration GPC data | 461 | | 486 | | 567 | | 765 | |
| HEI: hydroxy ester itaconate of Ex.3, DHEI: dihydroxy ester itaconate of Ex.4, CE10P: Cardura™ E10P Glycidyl Ester. | | | | | | | | |

A difference Color (Pt/Co) was observed between the hydroxyester and dihydroxyester formed. This is attributable to the use of different raw materials. Itaconic anhydride leads to yellowing of the final resin, which is not the case with the itaconic acid used for dihydroxyester.

2. Synthesis of the polyesters

Example 5 (Linear Itaconate Polyester (solvent-free)):

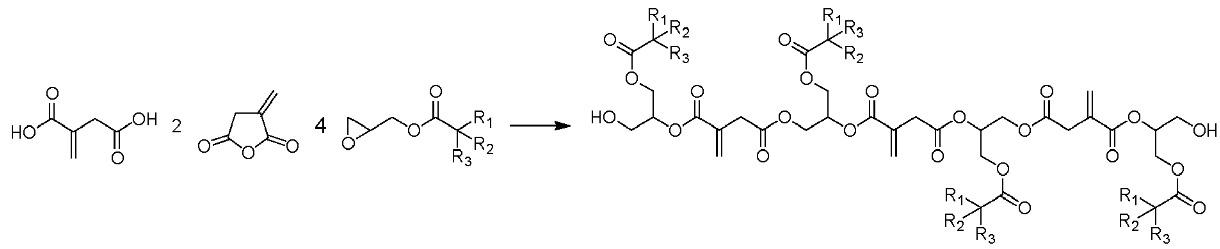

In a glass reactor equipped with an anchor stirrer, a reflux condenser and nitrogen inlet, itaconic acid (IA) was dissolved in a minimum of Cardura™ E10P at a temperature of 140°C. Then Cardura™ E10P was added for one hour to reach a molar ratio 1:2 IA:Cardura™ E10P to form a diester. EGC and AV measurements are used to measure remaining acid, and the corresponding amount of Cardura™ E10P was added to complete the conversion. Itaconic Anhydride (IAnh) was then added by portions in an equimolar ratio to Cardura™ E10P, while keeping the temperature at 140°C. The conversion was monitored by AV and EGC measurements. Additional Cardura™ E10P was then fed to the reactor in an equimolar ratio to Itaconic anhydride to form a Linear Itaconate Polyester. The temperature was kept at 140°C and the conversion followed by AV and EGC. Additional Cardura™ E10P was added to finalize the conversion. The reactor was allowed to cool to room temperature, and the product was collected and characterized. See Table 2 below.

Structure of Example 5:

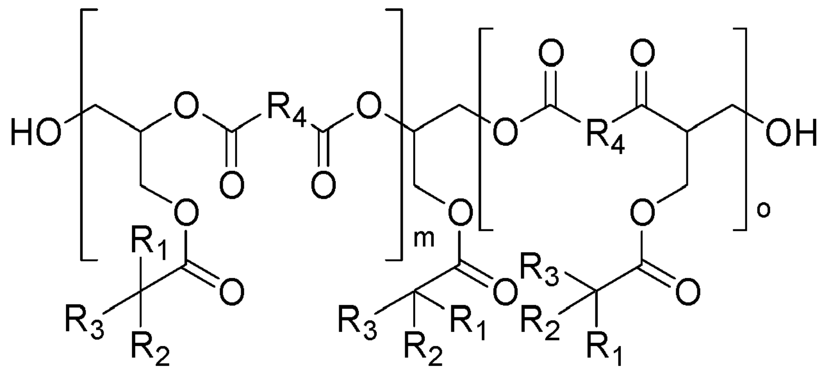

with $R_1$ and $R_2$ are H or linear or branched unsubstituted alkyl groups, wherein $R_1$ + $R_2$ comprise 7 carbon atoms; $R_3$ = $CH_3$;
$R_4$ is

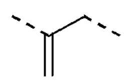

; m = 2; o = 1.

For reasons of clarity, the illustrated structure shows the structure of a linear oligomer obtained after direct opening the epoxide ring of Cardura™ E10P, wherein the OH groups formed after the opening are primary OH groups (corresponding to formula (1)). In addition, it is also conceivable that secondary OH groups are formed after corresponding direct opening of the epoxide ring of Cardura™ E10P after opening (corresponding to formula (2)). The epoxy ring could also be opened in the way of ortho-ester mediation (corresponding to formula (35)). Consequently, a co-oligomer comprising all three possible (co-)monomer structures and/or a mixture of the three oligomers, each of which has one of the structures (1), (2) or (35), would be conceivable as an alternative.

Example 6 (Itaconate Star-shaped polyester (solvent-free)):

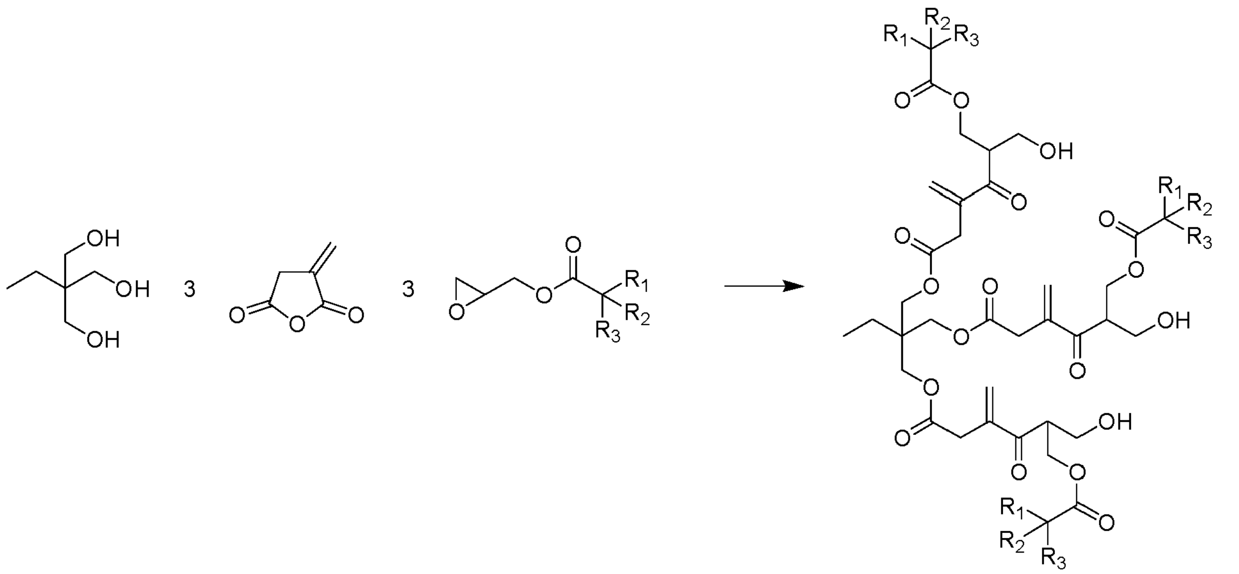

In a glass reactor equipped with an anchor stirrer, a reflux condenser and nitrogen inlet, itaconic Anhydride (IAnh) was reacted with Trimethylolpropane (TMP) in a molar ratio IAnh/TMP 3/1 in a minimum of Cardura™ E10P at a temperature of 140°C. The opening of the anhydride by the alcohol was followed by Acid Value measurement. Cardura™ E10P was added for one hour to reach an equimolar ratio of Itaconic Anhydride and form a Star Polyester. EGC and AV measurements were performed to measure remaining acid, and the corresponding amount of Cardura™ E10P was added to complete the conversion. The reactor was allowed to cool to room temperature, and the product was collected and characterized. See Table 2 below.

Structure of Example 6:

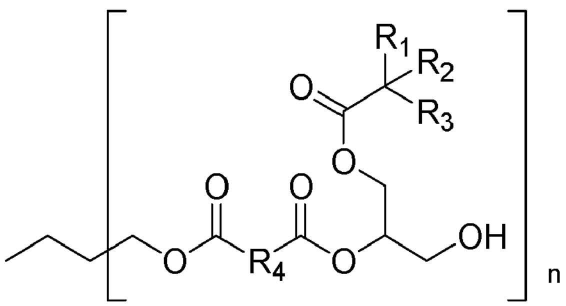

with $R_1$ and $R_2$ are H or linear or branched unsubstituted alkyl groups, wherein $R_1$ + $R_2$ comprise 7 carbon atoms;
$R_3$ = $CH_3$;
$R_4$ is

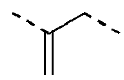

; n = 3.

For reasons of clarity, the illustrated structure shows the structure of a star-shaped oligomer obtained after direct opening the epoxide ring of Cardura™ E10P, wherein the OH groups formed after the opening are primary OH groups

(corresponding to formula (1)). In addition, it is also conceivable that secondary OH groups are formed after corresponding direct opening of the epoxide ring of Cardura™ E10P after opening (corresponding to formula (2)). The epoxy ring could also be opened in the way of ortho-ester mediation (corresponding to formula (35)). Consequently, a co-oligomer comprising all three possible (co-)monomer structures and/or a mixture of the three oligomers, each of which has one of the structures (1), (2) or (35), would be conceivable as an alternative.

Table 2: Synthesis recipes of the oligomers.

| Adduct reference | Linear Itaconate Polyester (Ex.5) (LIPE) | | Star Itaconate Polyester (Ex.6) (SIPE) | |
|---|---|---|---|---|
| Initial reactor charge | *Wt%* | *Intake in 500mL reactor (g)* | *Wt%* | *Intake in 500mL reactor (g)* |
| Itaconic Acid | 9,9 | 35,6 | - | - |
| Itaconic Anhydride | - | - | 28,6 | 114,5 |
| Trimethylolpropane | - | - | 11,4 | 45,7 |
| CE10P | 18,2 | 65,5 | 20,0 | 79,9 |
| CE10P addition | | | | |
| CE10P | 18,2 | 65,5 | 40,0 | 159,9 |
| Itaconic Anhydride addition | | | | |
| Itaconic Anhydride | 17,4 | 62,6 | - | - |
| CE10P addition | | | | |
| CE10P | 36,3 | 130,9 | - | - |
| Viscosity (spindle #4, 10 rpm - mPa.s) | 46 670 | | 365 900 | |
| Color (Pt/Co) | 153 | | - | |
| Mw (g/mol) by integration GPC data | 1301 | | 2227 | |
| LIPE: linear itaconate polyester of Ex.5, SIPE: star itaconate polyester of Ex.6. CE10P is Cardura™ E10P. | | | | |

The solvent-free trifunctional polyesters obtained had even higher viscosities compared to (di)hydroxyesters, with a significant increase by a factor 10 for the star itaconate polyester of Ex.6 (SIPE). The linearity difference and the steric hindrance could explain the important difference in final viscosities. The difference in molar mass between the two products also has a significant impact on their viscosity.

As the synthesis of the diluent and the oligomer shows, glycidyl ester of neodecanoic acid can advantageously be combined with unsaturated building blocks like sustainable itaconate derivatives in a low temperature solvent-free and catalyst-free process without generating water by-product to design a variety of (poly) hydroxyesters suitable for high-performance radiation-curable systems. Among other things, this is due to the fact that a glycidyl neodecanoate (i.e. Cardura™ E10P) was used, which is a mono-epoxy molecule known for its excellent reactivity under low process heat, and for its high boiling point and excellent solvent ability, which makes it a raw material of choice to ease the polyesterification processes. When anhydrides are combined with Cardura™ E10P, the polymerization process can even be further simplified via a polyaddition mechanism at relatively low reaction temperature without the need for additional catalyst or solvent, and without generating water by-product.

## 3. Properties of the diluents and the oligomers

### 3.1 Thermodynamic material properties

A good indicator of the intrinsic properties of the components in formulations is their glass transition temperature. Each component was mixed with 3% in weight of photoinitiator (OMNIRAD 4265), irradiated under Nitrogen flow, and dried in a vacuum furnace during at least 24 hours. A DSC analysis has then been performed and the respective glass transition temperatures (Tg) have been determined.

Table 3: Glass transition temperatures obtained by DSC analysis of the polymerized/cured components.

| Component | Tg (°C) of polymerized/cured component |
|---|---|
| TMPTA (Comparative) | 60* |
| HDDA (Comparative) | 45* |
| ACE (Comparative) | -1 |
| HEI (Ex. 3) | 28 |
| DHEI (Ex. 4) | 14 |
| LIPE (Ex. 5) | -7 |
| SIPE (Ex 6) | 13 |
| TMPTA: trimethylolpropane triacrylate, HDDA: 1,6-hexanediol diacrylate, ACE: ACE™ hydroxyl acrylate monomer (the adduct of acrylic acid and the glycidyl ester of neodecanoic acid), *: Theoretical data issued from Sartomer Product Selector Catalogue. | |

In comparison with TMPTA and HDDA, the products of the invention (HEI, DHEI, LIPE, SIPE) have a lower Tg which induces flexibility and low hardness on the cured films. The theorical Tg of ACE (which is 0°C) was confirmed with these measurements. Moreover, the presence of OH groups of the incorporated Cardura™ E10P moieties in the product seems to lower the Tg (by comparing Tg obtained for HEI having one Cardura™ E10P moiety with DHEI having two Cardura™ E10P moieties). The value measured for the Linear Polyester was significantly lower than for the other itaconate-based compounds. It must be noticed that it has been measured using another temperature scale:
-20°C to 100°C - 10°C/min instead of the -20°C/min scale.

3.2 Reactivity of the diluents

The Gel Content of the reactive diluents and monomers was determined by MEK resistance analysis. Each formulation contains 3% in weight of photoinitiator (Omnirad 4265) and 0.5% of inhibitor (methoxyphenol) in addition to the respective diluents or monomer. The formulations were poured into Teflon molds, covered with a Polyester film, and then irradiated for 1 sec to 5 min, depending on the type of reactive diluent, to obtain a demoldable resin.

Table 4: Gel Content based on MEK resistance analysis.

| Diluent | Curing time | Gel Content |
|---|---|---|
| TMPTA (Comparative) | 1 s | 73% |
| | 5 s | 85% |
| | 20 s | 99% |
| HDDA (Comparative) | 1 s | 70% |
| | 5 s | 100% |
| | 10 s | 100% |
| | 30 s | 100% |
| ACE (Comparative) | 1 s | 79% |
| | 5 s | 91% |
| | 30 s | 98% |
| HEI (Ex. 3) | 2 min | 0% |
| | 5 min | 48% |
| DHEI (Ex. 4) | 1 min | 16% |
| | 5 min | 69% |

Table 4 shows that the reactivity of monomers and oligomers significantly differs according to their functionality in the following order: Acrylate > Itaconate.

Thus, it can be concluded that acrylic diluents such as TMPTA and HDDA will provide fast curing, high relative hardness, and low flexibility to the coating. ACE will provide also fast curing but a low relative hardness and good flexibility. HEI and DHEI will provide slower curing, lower hardness, and higher flexibility.

## 4. Application examples

Unless stated otherwise, UV-curable mixtures were made with ~50 wt% oligomer, ~50 wt% reactive diluent, a photoinitiator OMNIRAD 4265 from IGM (3%) and an inhibitor methoxyphenol (0.5%). After application on a metal plate, resins were irradiated under Nitrogen flow for 20 seconds with an LED lamp (385 nm) at a distance of 20 cm from the substrate.

### 4.1 Hardness analysis

Hardness test: The hardness analysis was performed on a cured film coated on a metal plate to a thickness of 80 μm. The plate is placed on a Koenig pendulum and the hardness was defined by the time needed to damp the amplitude of the pendulum's oscillation between two predefined angles. A reference measurement was made on a glass plate and the hardness value was determined with the number of oscillations of the pendulum of the reference and the coated metal plate.

$$\text{Hardness in sec (Koenig)} = 240/(\text{Reference value}) \times \text{Number of Oscillation measured}$$

Table 5: Koenig hardness of 50:50 mixtures between oligomers (LIPE or SIPE) and reactive diluents.

| Reactive Diluent | Koenig Hardness (in sec) | |
|---|---|---|
| | LIPE (Ex. 5) | SIPE (Ex 6) |
| TMPTA | 81 | 69 |
| HDDA | 77 | 54 |
| ACE | 13 | 10 |
| TMPTA/HDDA 50/50 | 65 | 65 |
| ACE/TMPTA 65/35 | 20 | 16 |
| DHEI/TMPTA 55/45 | 31 | 27 |
| HEI/TMPTA 55/45 | 33 | 38 |
| HEI | 8 | - |
| DHEI | 14 | - |

The hardness results show that all systems containing itaconate unsaturation have cured in a satisfactory manner, and that the final hardness values are significantly influenced by the respective Tg of the reactive diluents used in the mixture.

### 4.2 Crosscut adhesion tests

Crosscut adhesion tests were performed on metal plate following ASTM D3359 with ISO ranking (1 = best/full adhesion, 5 = worst/no adhesion)

Table 6: Cross-cut adhesion test on metal plate (ISO classification: 1=best, 5=worst).

| Ratio 50/50 | LIPE (Ex. 5) | SIPE (Ex 6) | TMPTA |
|---|---|---|---|
| HEI (Ex. 3) | 2 | 1 | 5 |
| DHEI (Ex. 4) | 2 | 1 | 5 |
| TMPTA | 5 | 5 | 5 |
| ACE | 5 | 5 | 5 |

(continued)

| Ratio 50/50 | LIPE (Ex. 5) | SIPE (Ex 6) | TMPTA |
|---|---|---|---|
| HDDA | 5 | 5 | 5 |

The best adhesion results on metal were obtained with the formulations containing HEI and DHEI as reactive diluents. The significant difference of adhesion measured between acrylate-based and itaconate-based diluents, can be explained by the presence of glycidyl neodecanoate and its hydroxyl group in combination with the better flexibility of itaconate adducts. The slower curing rate of itaconate is probably also partly responsible for the lower shrinkage, and hence, improved adhesion.

4.3 Gel content test (Examples 9 to 11)

Gel Content determination: The Gel Content was determined by performing a MEK resistance test. Around 1g of cured resin was poured into a glass container and immerged in Methyl Ethyl Ketone (MEK) for 24 hours. Then the film was released from the MEK solution and allowed to dry. The Gel Content was calculated by comparing the initial ($m_0$) and the final ($m_F$) weight of the cured film:

$$GC\,(\%) = \frac{m_F}{m_0} \times 100$$

Table 7: Gel content analysis after immersion in MEK (10sec irradiation time).

| Formulation ratio | %Itaconate | %Acrylate | Gel Content (%) |
|---|---|---|---|
| TMPTA | 0% | 100% | 98% |
| ACE/TMPTA 50/50 | 0% | 100% | 97% |
| SIPE/ACE/TMPTA 51/37/12 | 51% | 49% | 74% |
| LIPE/ACE/TMPTA 63/23/14 | 63% | 37% | 83% |
| DHEI/SIPE/TMPTA 37/32/31 | 69% | 31% | 79% |
| DHEI/LIPE/TMPTA 44/32/23 | 77% | 23% | 72% |
| HEI/TMPTA 77/23 | 77% | 23% | 79% |
| DHEI/TMPTA 78/22 | 78% | 22% | 76% |
| DHEI/LIPE 55/45 | 100% | 0% | 89% |
| DHEI/LIPE 50/50 | 100% | 0% | 96% |

Satisfactory results were obtained for all cured films, even for those with very high itaconate content, which was not a given considering the higher reactivity of the acrylates compared to itaconates. An indication of the relative percentage of acrylate or itaconate double bonds are given in Table 7 for each mixture. It can be seen that the system based solely on itaconate derivatives (DHEI + LIPE) performs better than formulations based on mixtures of acrylate and itaconate derivatives, probably because of the better reactive compatibility when mixtures are prepared with similar molecular structure. In addition, the presence of glycidyl neodecanoate could be beneficial to the itaconate reactivity. As far as the inventors know, the itaconate double bonds are slower to react than their acrylate counterparts. The crosslink density was further evaluated by adapting the curing process with longer irradiation time (1min instead of 10sec). As a result, it was found that the gel content of 100%-based itaconate derivatives are comparable to all-acrylate systems as suggested by the results in Table 8 below.

Table 8: Gel content analysis after immersion in MEK (1min irradiation time).

| Formulation ratio | %Itaconate | %Acrylate | Gel Content (%) |
|---|---|---|---|
| TMPTA | 0% | 100% | 98% |
| ACE/TMPTA 50/50 | 0% | 100% | 97% |

(continued)

| Formulation ratio | %Itaconate | %Acrylate | Gel Content (%) |
|---|---|---|---|
| HEI/ LIPE 50/50 | 100% | 0% | 91% |
| DHEI/LIPE 50/50 | 100% | 0% | 96% |
| LIPE/HDDA | 50% | 50% | 88% |
| SIPE/HDDA | 50% | 50% | 90% |

Table 8 shows that similar results can be obtained for itaconate-based resins of the invention compared to acrylate-based resins and by adapting the curing time to the slower reactivity of itaconate under UV-light. Moreover, the formulations which contain a mix of acrylates and itaconates appear to be less resistant to MEK which supports the hypothesis of a lower compatibility of components with inhomogeneous functional groups.

These results obtained on formulated systems are consistent with the ones obtained on the reactive diluents with a diminution of curing time for formulations containing HDDA and TMPTA supported by a higher hardness than with Hexion's monomers and Itaconate-based reactive diluents.

<u>List of citations</u>

1. WO 2008/045478 A1
2. CA 2232875 A1
3. DE 1768622 A
4. WO 2013/113938 A1
5. P. Li et al., Itaconic Acid as a Green Alternative to Acrylic Acid for Producing a Soybean Oil-Based Thermoset: Synthesis and Properties. ACS Sustainable Chem. Eng., 2017, 5, 1228-1236.
6. DE 3803141 A1
7. WO 2022/058473 A1
8. S. Pérocheau Arnaud et al., Itaconic Acid-Based Reactive Diluents for Renewable and Acrylate-Free UV-Curing Additive Manufacturing Materials. ACS Sustainable Chem. Eng., 2021, 9, 17142-17151.
9. L. Papadopoulos et al., Bio-based additive manufacturing materials: An indepth structure-property relationship study of UV-curing polyesters from itaconic acid. European Polymer Journal, 2023, 186, 111872.
10. S. Ma et al., Bio-based epoxy resin from itaconic acid and its thermosets cured with anhydride and comonomers. Green Chem., 2013, 15, 245.
11. S. Wang et al., Making organic coatings greener: Renewable resource, solvent-free synthesis, UV curing and repairability. European Polymer Journal, 2020, 123, 109439.
12. J.-T. Miao et al., Three-Dimensional Printing Fully Biobased Heat-Resistant Photoactive Acrylates from Aliphatic Biomass. ACS Sustainable Chem. Eng., 2020, 8, 9415-9424.
13. Sartomer Product Selector Catalogue


## Claims

**1.** Resin formulation comprising

a) an oligomer comprising 3 to 20, structural units independently selected from formula (1), formula (2) and formula (35):

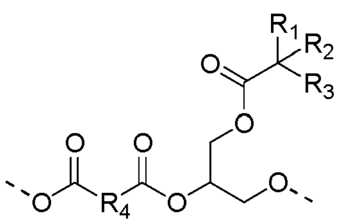
(1)

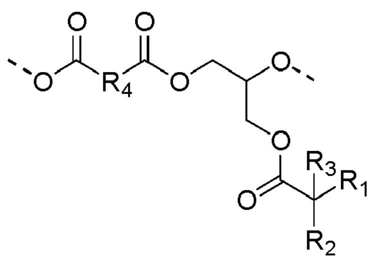
(2)

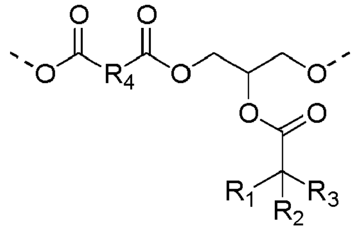

(35)

, wherein the structural units of the oligomer can be the same or can be different,
wherein the dashed lines represent the linking in the oligomer,
wherein $R_1$, $R_2$ are independently selected from the group consisting of hydrogen and linear or branched unsubstituted $C_{1-11}$ alkyl groups, wherein the total number of carbon atoms of $R_1$ and $R_2$ is in the range from 1 to 12,
wherein $R_3$ is methyl,
wherein $R_4$ is a divalent organic group comprising one or more UV curable carbon-carbon double bonds;

b) a reactive diluent comprising exactly one structural unit according to formulae (1), (2) or (35);
c) a photoinitiator.

**2.** The resin formulation according to claim 1, wherein the oligomer further comprises one or more moieties selected from formula (6), formula (7) and formula (36):

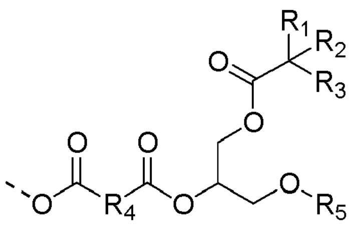

(6)

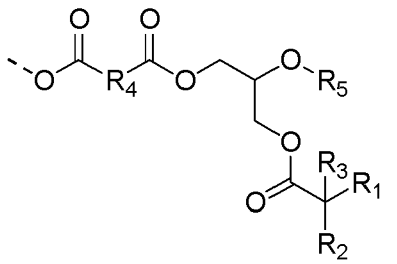

(7)

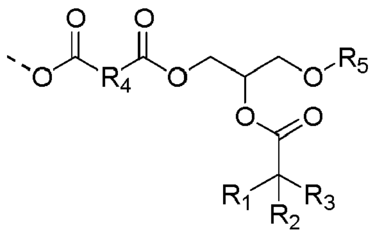

(36)

, wherein $R_1$, $R_2$, $R_3$ are as defined in claim 1,
wherein $R_4$ is a divalent organic group comprising one or more UV curable carbon-carbon double bonds,
wherein $R_5$ is H or represented by formula (8):

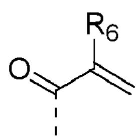

(8)

, wherein $R_6$ is H or methyl;
optionally the oligomer further comprises one or more structural units independently selected from formulae (9) to (12), formula (37) and formula (38):

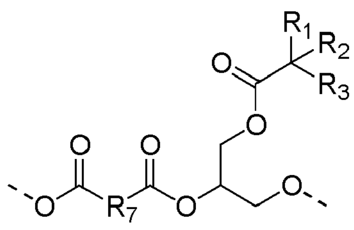

(9)

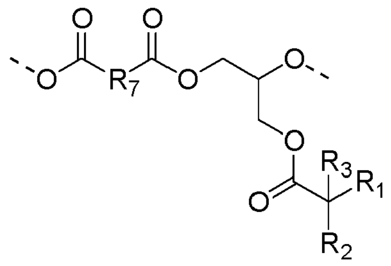

(10)

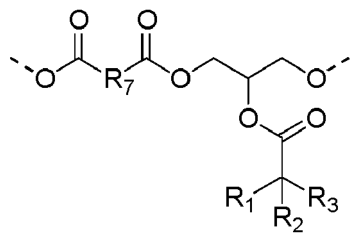

(37)

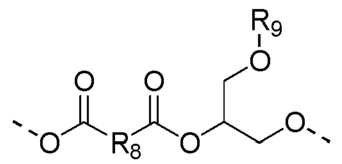

(11)

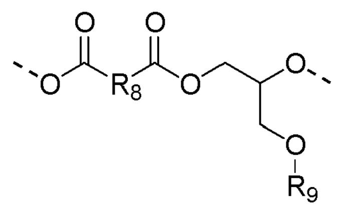

(12)

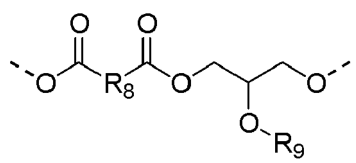

(38)

, wherein the structural units can be the same or different,

wherein $R_7$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,

wherein $R_8$ is a divalent organic group comprising one or more UV curable carbon-carbon double bonds, or $R_8$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,

wherein $R_9$ is selected from formula (13) and formula (14),

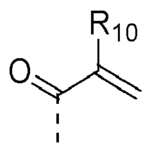

(13)

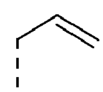

(14)

, wherein $R_{10}$ is H or methyl,

preferably optionally

the oligomer further comprises one or more moieties independently selected from formulae (15) to (18), formula (39) and formula (40):

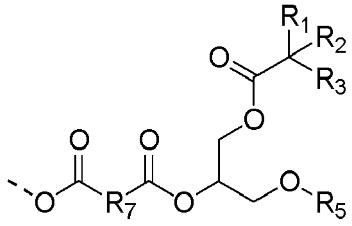 (15) 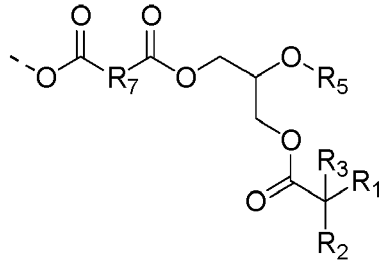 (16)

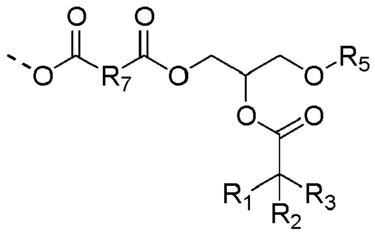 (39)

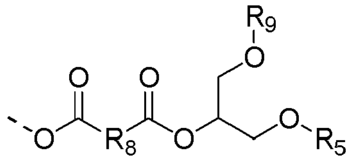 (17) 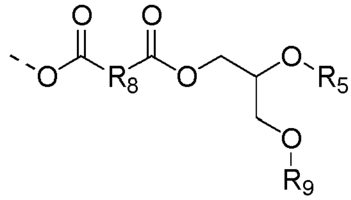 (18)

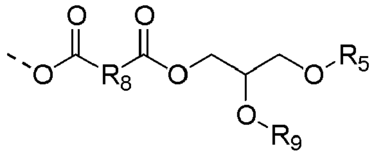 (40)

, wherein the moieties can be the same or different.

**3.** The resin formulation according to claim 1, wherein the oligomer is selected from formulae (33), (34), (47), (21), (22) and (42):

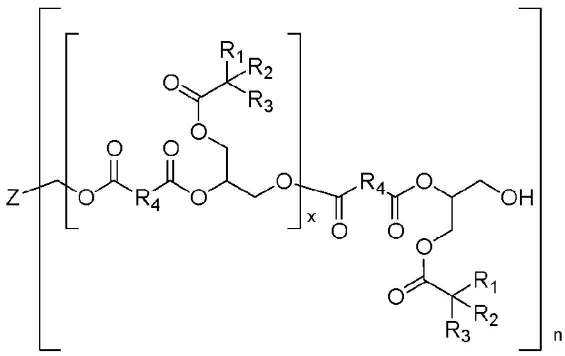 (33) 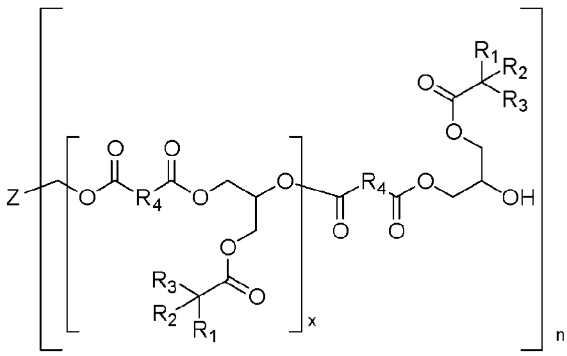 (34)

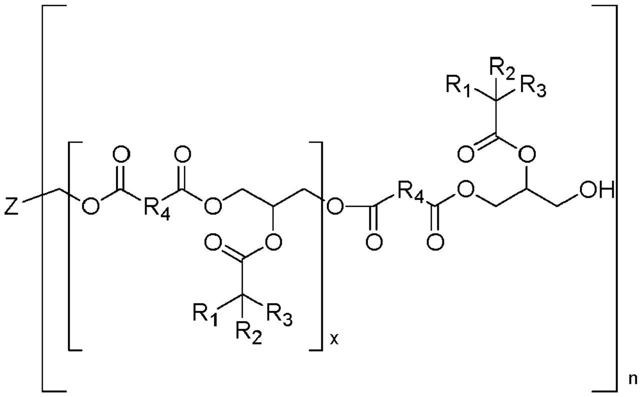

(47)

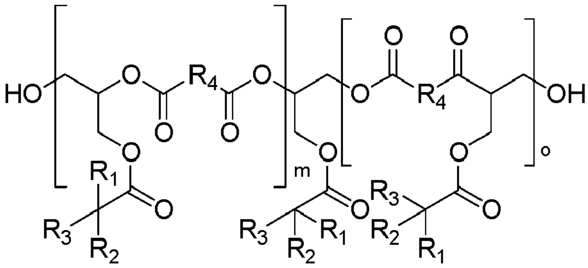

(21)

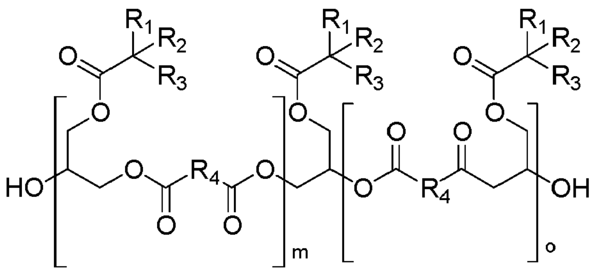

(22)

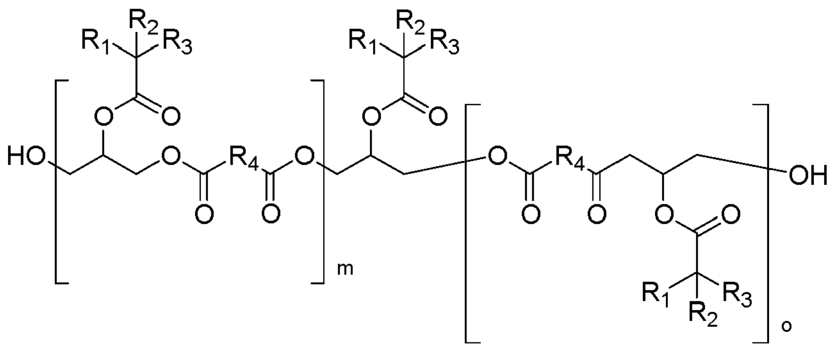

(42)

, wherein $R_1$, $R_2$, $R_3$ are as defined in claim 1,
wherein $R_4$ is a divalent organic group comprising one or more UV curable carbon-carbon double bonds,
wherein Z represents an n-valent organic group,
wherein n is in the range of 3 to 6,
wherein x is in the range of 0 to 4,
wherein m + o is in the range of 3 to 11.

**4.** The resin formulation according to any of the preceding claims, wherein the reactive diluent is selected from formula (23), formula (24) and formula (43):

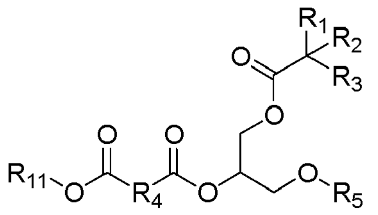 (23) 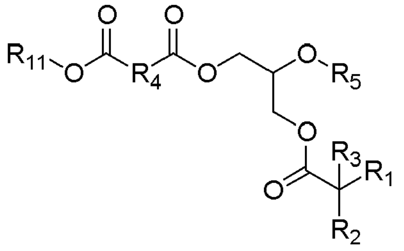 (24)

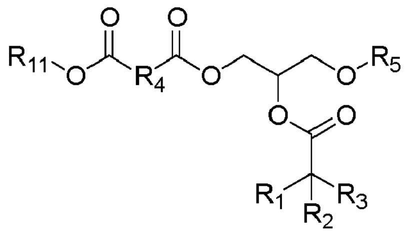 (43)

, wherein $R_1$, $R_2$, $R_3$ are as defined in claim 1,
wherein $R_4$ is a divalent organic group comprising one or more UV curable carbon-carbon double bonds,
wherein $R_5$ is H or represented by formula (8):

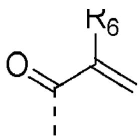 (8)

, wherein $R_6$ is H or methyl,
wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, or
$R_{11}$ is independently selected from formulae (25) to (30) and formulae (44) to (46):

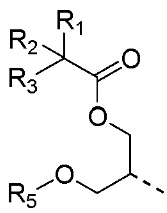 (25) 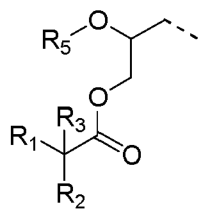 (26)

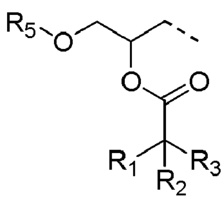 (44)

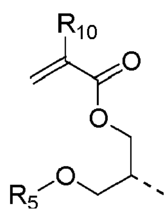 (27) 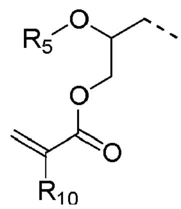 (28)

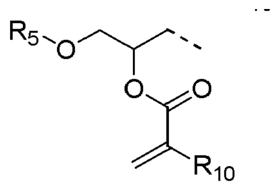

(45)

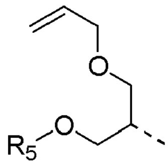

(29)

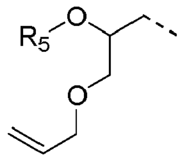

(30)

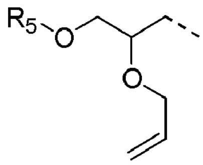

(46)

, wherein $R_{10}$ is H or methyl.

5. The resin formulation according to any of the preceding claims, wherein the resin formulation comprising further reactive diluents selected from the group consisting of trimethylolpropane triacrylate, 1,6-hexanediol diacrylate, and the adduct of acrylic acid and the glycidyl ester of neodecanoic acid.

6. Use of a resin formulation as defined in any of claims 1 to 5 in UV-curing processes.

7. Preparation of a resin formulation by combining at least (a) and (b) as defined in any of claims 1 to 5.

8. Coated article comprising a coating obtained by applying a resin formulation onto an article and drying said resin formulation, wherein said resin formulation is as defined in any of claims 1 to 5.

9. An oligomer comprising 3 to 20 structural units independently selected from formula (1), formula (2) and formula (35):

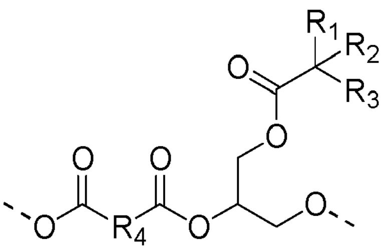

(1)

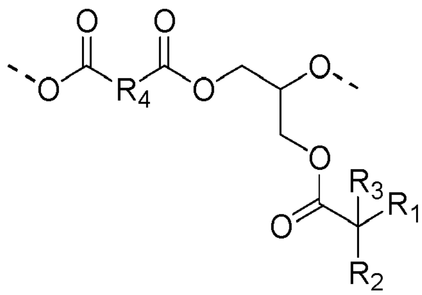

(2)

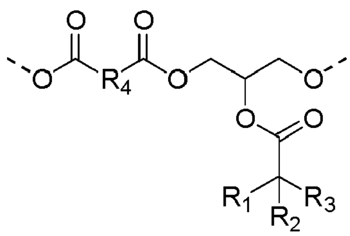

(35)

, wherein the structural units of the oligomer can be the same or can be different,
, wherein the dashed lines represent the linking in the oligomer,
wherein $R_1$, $R_2$, $R_3$ are as defined in claim 1;
wherein $R_4$ is a divalent organic group comprising one or more carbon-carbon double bonds.

**10.** The oligomer according to claim 9, wherein the oligomer further comprises one or more moieties independently selected from formula (6), formula (7) and formula (36):

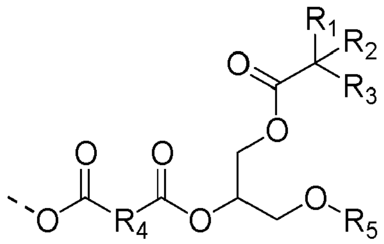
(6)

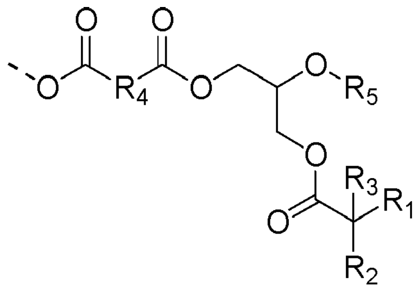
(7)

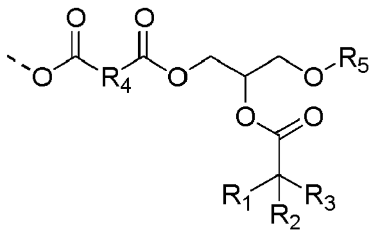
(36)

, wherein $R_1$, $R_2$, $R_3$ are as defined in claim 1,
wherein $R_4$ is a divalent organic group comprising one or more UV curable carbon-carbon double bonds,
wherein $R_5$ is H or represented by formula (8):

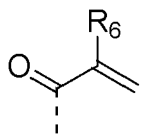
(8)

, wherein $R_6$ is H or methyl;
optionally the oligomer further comprises one or more structural units independently selected from formulae (9) to (12), formula (37) and formula (38):

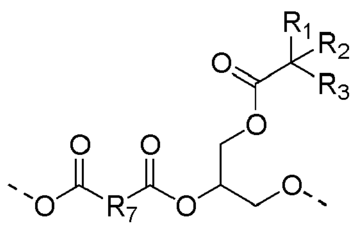
(9)

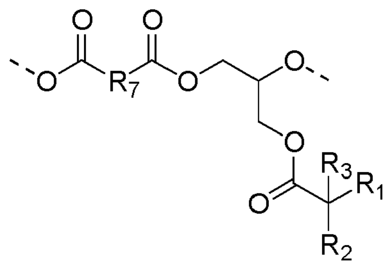
(10)

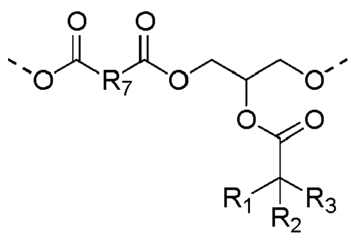
(37)

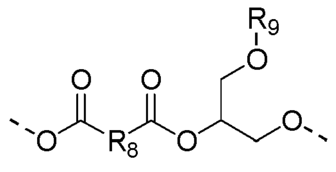 (11) 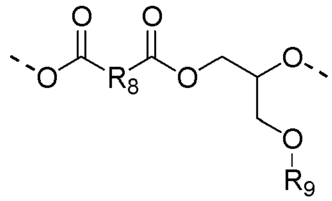 (12)

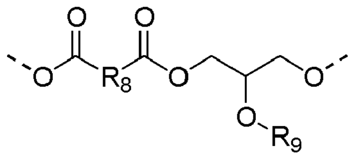 (38)

, wherein the structural units can be the same or different,
wherein $R_1$, $R_2$, $R_3$ are as defined in claim 1,
wherein $R_7$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,
wherein $R_8$ is a divalent organic group comprising one or more UV curable carbon-carbon double bonds, or $R_8$ is selected from the group consisting of: substituted or unsubstituted $C_{1-12}$ alkyl, substituted or unsubstituted $C_{1-10}$ heteroalkyl, substituted or unsubstituted $C_{6-14}$ aryl, and substituted or unsubstituted $C_{4-12}$ heteroaryl groups,
wherein $R_9$ is selected from formula (13) and formula (14),

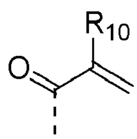 (13) 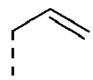 (14)

, wherein $R_{10}$ is H or methyl;
preferably optionally
the oligomer further comprises one or more moieties independently selected from formulae (15) to (18), formula (39) and formula (40):

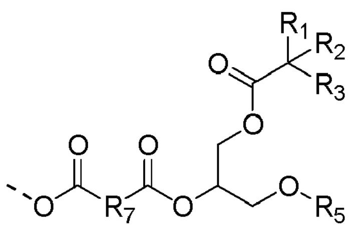 (15) 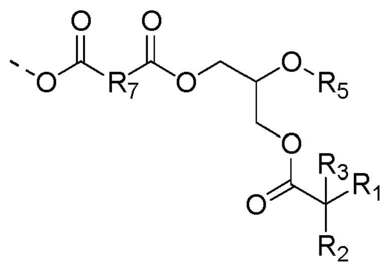 (16)

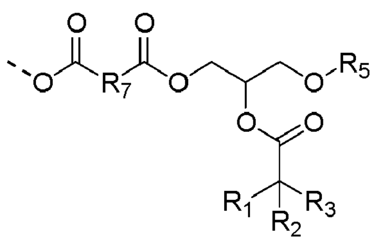 (39)

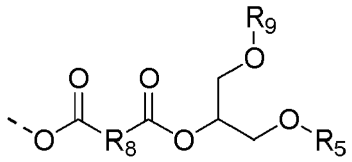 (17)

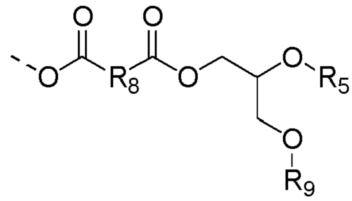 (18)

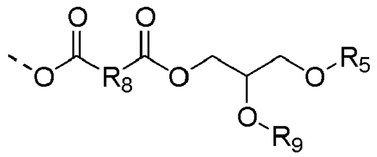 (40)

, wherein the moieties can be the same or different.

**11.** The oligomer according to claim 9, wherein the oligomer is selected from formulae (33), (34), (47), (21), (22) and (42):

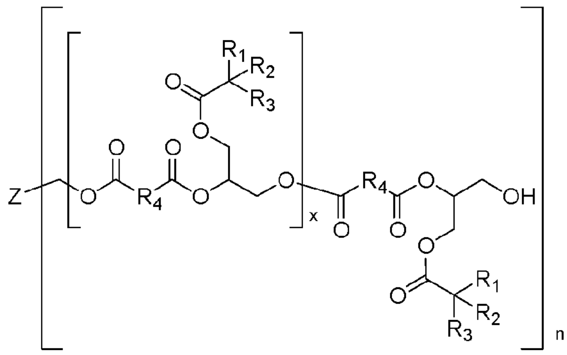 (33)

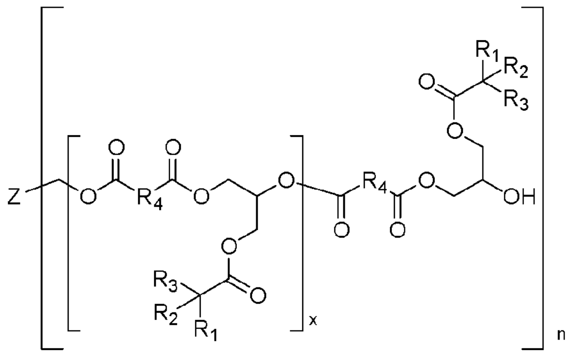 (34)

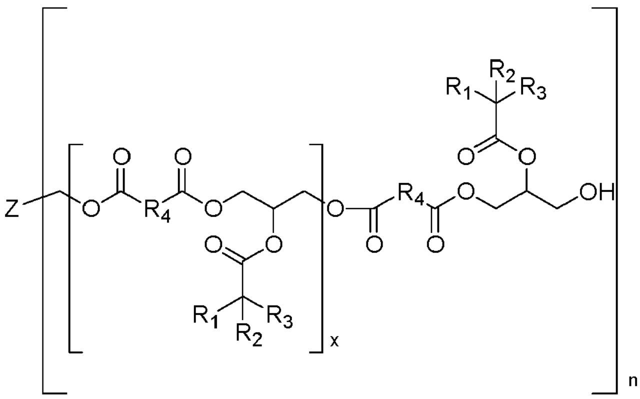 (47)

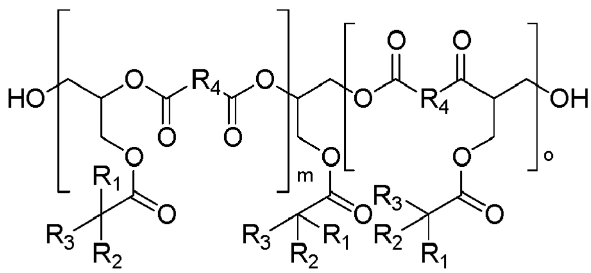 (21)

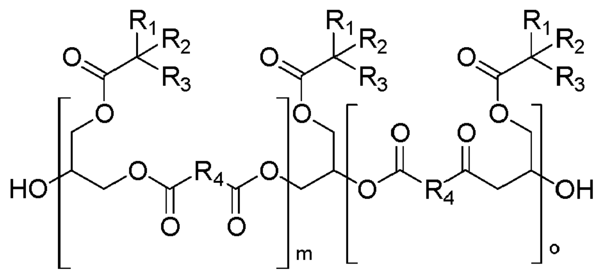

(22)

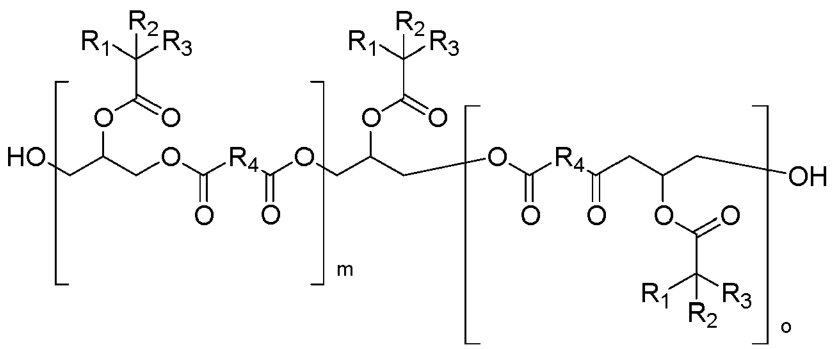

(42)

, wherein $R_1$, $R_2$, $R_3$ are as defined in claim 1,
wherein $R_4$ is a divalent organic group comprising one or more UV curable carbon-carbon double bonds,
wherein Z represents an n-valent organic group,
wherein n is in the range of 3 to 6,
wherein x is in the range of 0 to 4,
wherein m + o is in the range of 3 to 11.

**12.** Resin formulation comprising the oligomer according to any of claims 9 to 11.

**13.** A reactive diluent selected from formula (23), formula (24) and formula (43):

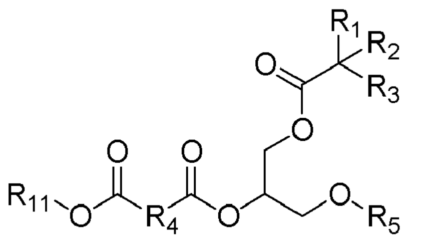

(23)

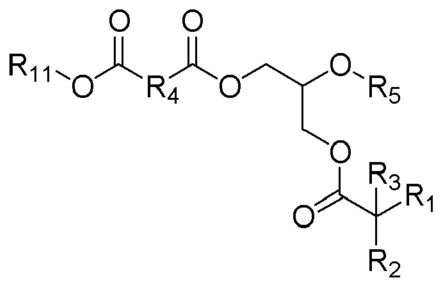

(24)

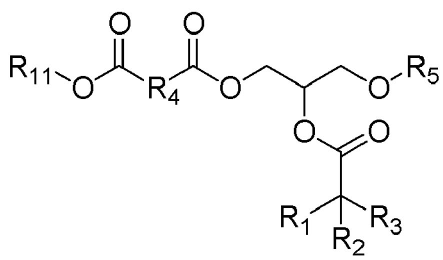

(43)

, $R_1$, $R_2$, $R_3$ are as defined in claim 1,
wherein $R_4$ is a divalent organic group comprising one or more UV curable carbon-carbon double bonds,
wherein $R_5$ is H or represented by formula (8):

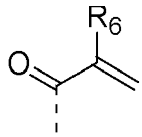
(8)

, wherein $R_6$ is H or methyl,

wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, or

$R_{11}$ is independently selected from formulae (25) to (30) and formulae (44) to (46):

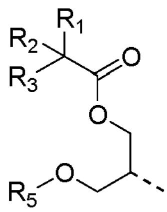
(25)

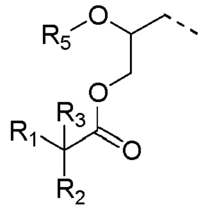
(26)

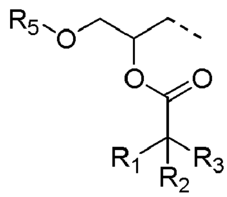
(44)

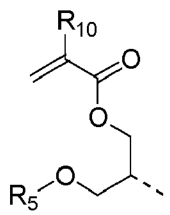
(27)

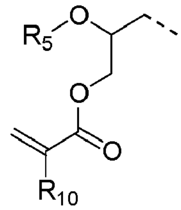
(28)

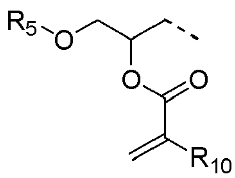
(45)

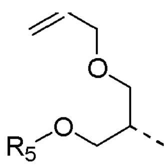
(29)

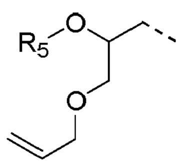
(30)

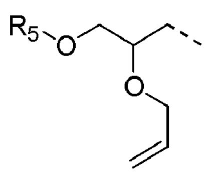
(46)

, wherein $R_{10}$ is H or methyl.

**14.** The reactive diluent according to claim 13, wherein the reactive diluent is selected from formula (23), formula (24) and

formula (43):

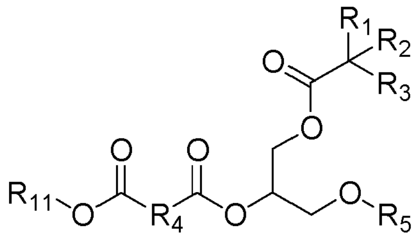
(23)

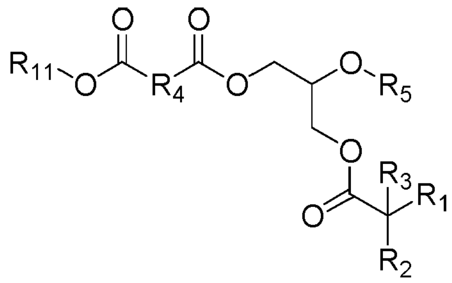
(24)

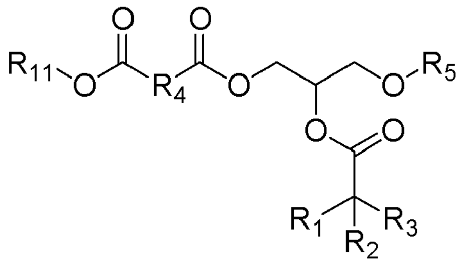
(43)

, wherein $R_1$, $R_2$, $R_3$ are as defined in claim 1,
wherein $R_4$ is represented by formula (3):

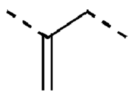
(3)

, wherein $R_5$ is H,
wherein $R_{11}$ is selected from the group consisting of: substituted or unsubstituted $C_{1-12}$ alkyl, substituted or unsubstituted $C_{5-12}$ cycloalkyl, substituted or unsubstituted $C_{1-10}$ heteroalkyl, substituted or unsubstituted $C_{6-14}$ aryl, and substituted or unsubstituted $C_{4-12}$ heteroaryl groups, or
$R_{11}$ is independently selected from formula (25), formula (26) and formula (44):

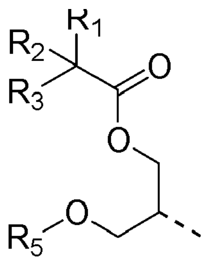
(25)

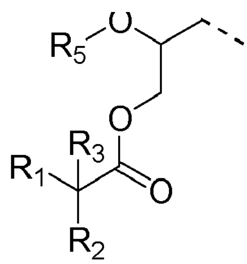
(26)

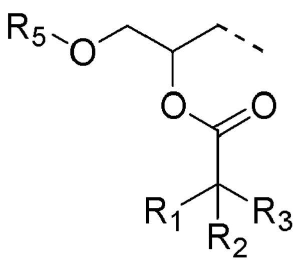
(44)

, wherein $R_1$, $R_2$, $R_3$ are as defined in claim 1,
wherein $R_5$ is H.

**15.** Resin formulation comprising the reactive diluent according to any of claims 13 or 14.

Europäisches Patentamt
European Patent Office
Office européen des brevets

# EUROPEAN SEARCH REPORT

Application Number
EP 24 17 3086

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2011/086975 A1 (MELNYK THOMAS [US] ET AL) 14 April 2011 (2011-04-14)<br>* paragraphs [0080], [0094]; claim 14; example 1 * | 9-12<br>1-8 | INV.<br>C07C69/52<br>C08G63/91<br>C08L67/06<br>C09D7/80<br>C09D167/00<br>C09D167/07 |
| X<br>Y | FR 2 220 509 A1 (DU PONT [US]) 4 October 1974 (1974-10-04)<br>* page 1, lines 23-27; claim 1; example 1 * | 13-15<br>1-8 | |
| X | WO 2005/040241 A1 (RESOLUTION RES BELGIUM S A [BE] ET AL.) 6 May 2005 (2005-05-06)<br>* examples 1-3; table 1 * | 13-15 | |
| X | EP 1 074 572 A1 (DAINIPPON INK & CHEMICALS [JP]) 7 February 2001 (2001-02-07)<br>* example 7 * | 9-12 | |
| | | | TECHNICAL FIELDS SEARCHED (IPC)<br>C09D<br>C07C<br>C08F<br>C08G<br>C09J<br>C09G<br>C08L |

The present search report has been drawn up for all claims

1

| Place of search | Date of completion of the search | Examiner |
|---|---|---|
| The Hague | 4 October 2024 | Jegou, Gwénaëlle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 17 3086

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2024

| Patent document cited in search report | Publication date | Patent family member(s) | Publication date |
|---|---|---|---|
| US 2011086975 A1 | 14-04-2011 | NONE | |
| FR 2220509 A1 | 04-10-1974 | AU 6624974 A | 04-09-1975 |
| | | BE 811817 A | 04-09-1974 |
| | | DE 2410513 A1 | 26-09-1974 |
| | | FR 2220509 A1 | 04-10-1974 |
| | | JP S5012018 A | 07-02-1975 |
| | | JP S49125439 A | 30-11-1974 |
| | | LU 69555 A1 | 21-06-1974 |
| | | NL 7402964 A | 09-09-1974 |
| | | ZA 741390 B | 26-02-1975 |
| WO 2005040241 A1 | 06-05-2005 | EP 1524280 A1 | 20-04-2005 |
| | | TW 200526747 A | 16-08-2005 |
| | | WO 2005040241 A1 | 06-05-2005 |
| EP 1074572 A1 | 07-02-2001 | DE 69921799 T2 | 17-03-2005 |
| | | EP 1074572 A1 | 07-02-2001 |
| | | GB 2352721 A | 07-02-2001 |
| | | JP 2001064376 A | 13-03-2001 |
| | | US 6350849 B1 | 26-02-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008045478 A1
- CA 2232875 A1
- DE 1768622 A
- WO 2013113938 A1
- DE 3803141 A1
- WO 2022058473 A1


### Non-patent literature cited in the description


- **P. LI et al.** Itaconic Acid as a Green Alternative to Acrylic Acid for Producing a Soybean Oil-Based Thermoset: Synthesis and Properties. *ACS Sustainable Chem. Eng.*, 2017, vol. 5, 1228-1236
- **S. PÉROCHEAU ARNAUD et al.** Itaconic Acid-Based Reactive Diluents for Renewable and Acrylate-Free UV-Curing Additive Manufacturing Materials. *ACS Sustainable Chem. Eng.*, 2021, vol. 9, 17142-17151
- **L. PAPADOPOULOS et al.** Bio-based additive manufacturing materials: An in-depth structure-property relationship study of UV-curing polyesters from itaconic acid. *European Polymer Journal*, 2023, vol. 186, 111872
- **S. MA et al.** Bio-based epoxy resin from itaconic acid and its thermosets cured with anhydride and comonomers. *Green Chem.*, 2013, vol. 15, 245
- **S. WANG et al.** Making organic coatings greener: Renewable resource, solvent-free synthesis, UV curing and repairability. *European Polymer Journal*, 2020, vol. 123, 109439
- **J.-T. MIAO et al.** Three-Dimensional Printing Fully Biobased Heat-Resistant Photoactive Acrylates from Aliphatic Biomass. *ACS Sustainable Chem. Eng.*, 2020, vol. 8, 9415-9424
- **L. PAPADOPOULOS et al.** Bio-based additive manufacturing materials: An indepth structure-property relationship study of UV-curing polyesters from itaconic acid. *European Polymer Journal*, 2023, vol. 186, 111872